# EUROPEAN PATENT APPLICATION

(11) **EP 2 348 043 A1**
(43) Date of publication of application: **27.07.2011**
(21) Application number: 10183325.9
(22) Date of filing: 01.10.2002
(51) Int. Cl.: C07K 14/52, A61K 38/19, C07H 21/04, C12N 15/63, C12N 1/21, C12N 1/11, C12N 5/16, C12N 15/12, C07K 14/705

(54) **APO-2 ligand variants and uses thereof**

(30) Priority: 02.10.2001 US 326622 P
(62) Divisional of application: 02766436.6
(71) Applicant: Genentech, Inc., South San Francisco CA 94080-4990 (US)
(72) Inventor: Kelly, Robert F., San Bruno, CA CA 94066 (US); Lindstrom, Stephanie H., Foster City, CA CA 94404 (US)
(74) Representative: Kiddle, Simon John

(57) **Abstract**

The disclosure provides Apo-2 ligand variant peptides. Methods of making and chemically modifying Apo-2 ligand variant polypeptides are also provided. In addition, formulations of Apo-2 ligand variant polypeptides are provided.

## Description

### FIELD OF THE INVENTION

The present invention relates generally to Apo-2 ligand variants, particularly Apo-2 ligand substitution variants, and to chemically modified forms thereof.

### BACKGROUND OF THE INVENTION

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see, e.g., Barr et al., Bio/Technology, 12:487-493 (1994); Steller et al., Science, 267:1445-1449 (1995)]. Apoptotic cell death naturally occurs in many physiological processes, including embryonic development and clonal selection in the immune system [Itoh et al., Cell, 66:233-243 (1991)].

Various molecules, such as tumor necrosis factor-alpha ("TNF-alpha"), tumor necrosis factor-beta ("TNF-beta" or "lymphotoxin-alpha"), lymphotoxin-beta ("LT-beta"), CD30 ligand, CD27 ligand, CD40 ligand, OX-40 ligand, 4-1BB ligand, Apo-1 ligand (also referred to as Fas ligand or CD95 ligand), Apo-2 ligand (also referred to as Apo2L or TRAIL), Apo-3 ligand (also referred to as TWEAK), APRIL, OPG ligand (also referred to as RANK ligand, ODF, or TRANCE), and TALL-1 (also referred to as BlyS, BAFF or THANK) have been identified as members of the tumor necrosis factor ("TNF") family of cytokines [See, e.g., Gruss and Dower, Blood, 85:3378-3404 (1995); Schmid et al., Proc. Natl. Acad. Sci., 83:1881 (1986); Dealtry et al., Eur. J. Immunol., 17:689 (1987); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992), WO 97/01633 published January 16, 1997; WO 97/25428 published July 17, 1997; Marsters et al., Curr. Biol., 8:525-528 (1998); Chicheportiche et al., Biol. Chem., 272:32401-32410 (1997); Hahne et al., J. Exp. Med., 188:1185-1190 (1998); WO98/28426 published July 2, 1998; WO98/46751 published October 22, 1998; WO/98/18921 published May 7, 1998; Moore et al., Science, 285:260-263 (1999); Shu et al., J. Leukocyte Biol., 65:680 (1999); Schneider et al., J. Exp. Med., 189:1747-1756 (1999); Mukhopadhyay et al., J. Biol. Chem., 274:15978-15981 (1999)]. Among these molecules, TNF-alpha, TNF-beta, CD30 ligand, 4-1BB ligand, Apo-1 ligand, Apo-2 ligand (Apo2L/TRAIL) and Apo-3 ligand (TWEAK) have been reported to be involved in apoptotic cell death.

Apo2L/TRAIL was identified several years ago as a member of the TNF family of cytokines. [see, e.g., Wiley et al., Immunity, 3:673-682 (1995); Pitti et al., J. Biol. Chem., 271:12697-12690 (1996); US Patent 6,284,236 issued September 4, 2001] The full-length human Apo2L/TRAIL polypeptide is a 281 amino acid long, Type II transmembrane protein. Some cells can produce a natural soluble form of the polypeptide, through enzymatic cleavage of the polypeptide's extracellular region [Mariani et al., J. Cell. Biol., 137:221-229 (1997)]. Crystallographic studies of soluble forms of Apo2L/TRAIL reveal a homotrimeric structure similar to the structures of TNF and other related proteins [Hymowitz et al., Molec. Cell, 4:563-571 (1999); Hymowitz et al., Biochemistry, 39:633-644 (2000)]. Apo2L/TRAIL, unlike other TNF family members however, was found to have a unique structural feature in that three cysteine residues (at position 230 of each subunit in the homotrimer) together coordinate a zinc atom, and that the zinc binding is important for trimer stability and biological activity. [Hymowitz et al., supra; Bodmer et al., J. Biol. Chem., 275:20632-20637 (2000)]

It has been reported in the literature that Apo2L/TRAIL may play a role in immune system modulation, including autoimmune diseases such as rheumatoid arthritis [see, e.g., Thomas et al., J. Immunol., 161:2195-2200 (1998); Johnsen et al., Cytokine, 11:664-672 (1999); Griffith et al., J. Exp. Med., 189:1343-1353 (1999); Song et al., J. Exp. Med., 191:1095-1103 (2000)].

Soluble forms of Apo2L/TRAIL have also been reported to induce apoptosis in a variety of cancer cells in vitro, including colon, lung, breast, prostate, bladder, kidney, ovarian and brain tumors, as well as melanoma, leukemia, and multiple myeloma [see, e.g., Wiley et al., supra; Pitti et al., supra; Rieger et al., FEBS Letters, 427:124-128 (1998); Ashkenazi et al., J. Clin. Invest., 104:155-162 (1999); Walczak et al., Nature Med., 5:157-163 (1999); Keane et al., Cancer Research, 59:734-741 (1999); Mizutani et al., Clin. Cancer Res., 5:2605-2612 (1999); Gazitt, Leukemia, 13:1817-1824 (1999); Yu et al., Cancer Res., 60:2384-2389 (2000); Chinnaiyan et al., Proc. Natl. Acad. Sci., 97:1754-1759 (2000)]. *In vivo* studies in murine tumor models further suggest that Apo2L/TRAIL, alone or in combination with chemotherapy or radiation therapy, can exert substantial anti-tumor effects [see, e.g., Ashkenazi et al., supra; Walzcak et al., supra; Gliniak et al., Cancer Res., 59:6153-6158 (1999); Chinnaiyan et al., supra; Roth et al., Biochem. Biophys. Res. Comm., 265:1999 (1999)]. In contrast to many types of cancer cells, most normal human cell types appear to be resistant to apoptosis induction by certain recombinant forms of Apo2L/TRAIL [Ashkenazi et al., supra; Walzcak et al., supra]. Jo et al. has reported that a polyhistidine-tagged soluble form of Apo2L/TRAIL induced apoptosis *in vitro* in normal isolated human, but not non-human, hepatocytes [Jo et al., Nature Med., 6:564-567 (2000); see also, Nagata, Nature Med., 6:502-503 (2000)]. It is believed that certain recombinant Apo2L/TRAIL preparations may vary in terms of biochemical properties and biological activities on diseased versus normal cells, depending, for example, on the presence or absence of a tag molecule, zinc content, and % trimer content [See, Lawrence et al., Nature Med., Letter to the Editor, 7:383-385 (2001); Qin et al., Nature Med., Letter to the Editor, 7:385-386 (2001)].

Induction of various cellular responses mediated by such TNF family cytokines is believed to be initiated by their binding to specific cell receptors. Two distinct TNF receptors of approximately 55-kDa (TNFR1) and 75-kDa (TNFR2) have been identified [Hohman et al., J. Biol. Chem., 264:14927-14934 (1989); Brockhaus et al., Proc. Natl. Acad. Sci., 87:3127-3131 (1990); EP 417,563, published March 20, 1991] and human and mouse cDNAs corresponding to both receptor types have been isolated and characterized [Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellular regions. The extracellular portions of both receptors are found naturally also as soluble TNF-binding proteins [Nophar, Y. et al., EMBO J., 9:3269 (1990); and Kohno, T. et al., Proc. Natl. Acad. Sci. U.S.A., 87:8331 (1990)]. The cloning of recombinant soluble TNF receptors was reported by Hale et al. [J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. Each CRD is about 40 amino acids long and contains 4 to 6 cysteine residues at positions which are well conserved [Schall et al., supra; Loetscher et al., supra; Smith et al., supra; Nophar et al., supra; Kohno et al., supra]. In TNFR1, the approximate boundaries of the four CRDs are as follows: CRD1- amino acids 14 to about 53; CRD2- amino acids from about 54 to about 97; CRD3- amino acids from about 98 to about 138; CRD4- amino acids from about 139 to about 167. In TNFR2, CRD1 includes amino acids 17 to about 54; CRD2- amino acids from about 55 to about 97; CRD3- amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., J. Exp. Med., 169:1747-1756 (1989) and Itoh et al., Cell, 66:233-243 (1991)]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD1 [Welcher, A.A. et al., Proc. Natl. Acad. Sci. USA, 88:159-163 (1991)] or a 5-amino acid insertion in this domain [Yan, H. and Chao, M.V., J. Biol. Chem., 266:12099-12104 (1991)] had little or no effect on NGF binding [Yan, H. and Chao, M.V., supra]. p75 NGFR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989); Yan, H. and Chao, M.V., supra]. A similar proline-rich region is found in TNFR2 but not in TNFR1.

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Recently, other members of the TNFR family have been identified. Such newly identified members of the TNFR family include CAR1, HVEM and osteoprotegerin (OPG) [Brojatsch et al., Cell, 87:845-855 (1996); Montgomery et al., Cell, 87:427-436 (1996); Marsters et al., J. Biol. Chem., 272:14029-14032 (1997); Simonet et al., Cell, 89:309-319 (1997)]. Unlike other known TNFR-like molecules, Simonet et al., supra, report that OPG contains no hydrophobic transmembrane-spanning sequence. OPG is believed to act as a decoy receptor, as discussed below.

Pan et al. have disclosed another TNF receptor family member referred to as "DR4" [Pan et al., Science, 276:111-113 (1997)]. The DR4 was reported to contain a cytoplasmic death domain capable of engaging the cell suicide apparatus. Pan et al. disclose that DR4 is believed to be a receptor for the ligand known as Apo-2 ligand or TRAIL.

In Sheridan et al., Science, 277:818-821 (1997) and Pan et al., Science, 277:815-818 (1997), another molecule believed to be a receptor for Apo2L/TRAIL is described [see also, WO98/51793 published November 19, 1998; WO98/41629 published September 24, 1998]. That molecule is referred to as DR5 (it has also been alternatively referred to as Apo-2; TRAIL-R, TR6, Tango-63, hAPO8, TRICK2 or KILLER [Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/0916 published February 25, 1999; WO99/11791 published March 11, 1999]. Like DR4, DR5 is reported to contain a cytoplasmic death domain and be capable of signaling apoptosis. The crystal structure of the complex formed between Apo-2L/TRAIL and DR5 is described in Hymowitz et al., Molecular Cell, 4:563-571 (1999).

A further group of recently identified TNFR family members are referred to as "decoy receptors," which are believed to function as inhibitors, rather than transducers of signaling. This group includes DCR1 (also referred to as TRID, LIT or TRAIL-R3) [Pan et al., Science, 276:111-113 (1997); Sheridan et al., Science, 277:818-821 (1997); McFarlane et al., J. Biol. Chem., 272:25417-25420 (1997); Schneider et al., FEBS Letters, 416:329-334 (1997); Degli-Esposti et al., J. Exp. Med., 186:1165-1170 (1997); and Mongkolsapaya et al., J. Immunol., 160:3-6 (1998)] and DCR2 (also called TRUNDD or TRAIL-R4) [Marsters et al., Curr. Biol., 7:1003-1006 (1997); Pan et al., FEBS Letters, 424:41-45 (1998); Degli-Esposti et al., Immunity, 7:813-820 (1997)], both cell surface molecules, as well as OPG [Simonet et al., supra] and DCR3 [Pitti et al., Nature, 396:699-703 (1998)], both of which are secreted, soluble proteins. Apo2L/TRAIL has been reported to bind those receptors referred to as DcR1, DcR2 and OPG.

Apo2L/TRAIL is believed to act through the cell surface "death receptors" DR4 and DR5 to activate caspases, or enzymes that carry out the cell death program. [See, e.g., Salvesen et al., Cell, 91:443-446 (1997)]. Upon ligand binding, both DR4 and DR5 can trigger apoptosis independently by recruiting and activating the apoptosis initiator, caspase-8, through the death-domain-containing adaptor molecule referred to as FADD/Mortl [Kischkel et al., Immunity, 12:611-620 (2000); Sprick et al., Immunity, 12:599-609 (2000); Bodmer et al., Nature Cell Biol., 2:241-243 (2000)]. In contrast to DR4 and DR5, the DcR1 and DcR2 receptors do not signal apoptosis.

For a review of the TNF family of cytokines and their receptors, see Ashkenazi and Dixit, Science, 281:1305-1308 (1998); Ashkenazi and Dixit, Curr. Opin. Cell Biol., 11:255-260 (2000); Golstein, Curr. Biol., 7:750-753 (1997); Gruss and Dower, supra, and Nagata, Cell, 88;355-365 (1997); Locksley et al., Cell, 104:487-501 (2001).

While zinc binding sites have been shown to play structural roles in protein-protein interactions in certain proteins involving diverse interfaces [Feese et al., Proc. Natl. Acad. Sci., 91:3544-3548 (1994); Somers et al., Nature, 372:478-481 (1994); Raman et al., Cell, 95:939-950 (1998)], none of the previously structurally-characterized members of the TNF family (CD40 ligand, TNF-alpha, or TNF-beta) bind metals. The use of metal ions, such as zinc, in formulations of various hormones, such as human growth hormone (hGH), has been described in the literature. [See, e.g., WO 92/17200 published October 15, 1992). Zinc involvement in hGH binding to receptors was likewise described in WO 92/03478 published March 5, 1992. The roles of zinc binding in interferon-alpha dimers and interferon-beta dimers were reported in Walter et al., Structure, 4:1453-1463 (1996) and Karpusas et al., Proc. Natl. Acad. Sci., 94:11813-11818 (1997), respectively. The structures and biological roles of various metal ions such as zinc have been reviewed in the art, see, e.g., Christianson et al., Advances in Protein Chemistry, 42:281-355 (1991).

### SUMMARY OF THE INVENTION

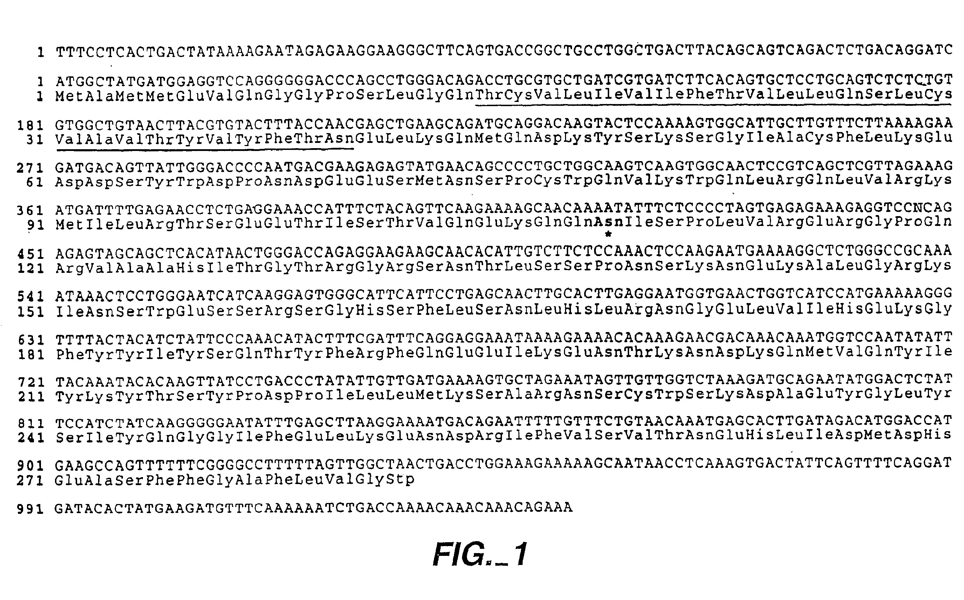

The present invention provides Apo-2 ligand variants. Particularly, the invention provides Apo-2 ligand variants comprising one or more amino acid substitutions in the native sequence of Apo-2 ligand (Figure 1). Optionally, the Apo-2 ligand variants may comprise cysteine, lysine and serine substitutions, such as provided in Table I below. A representative embodiment of the invention includes an isolated Apo-2 ligand variant polypeptide comprising an amino acid sequence which differs from the native sequence Apo-2 ligand polypeptide sequence of Figure 1 (SEQ ID NO:1) and has one or more of the following amino acid substitutions at the residue position(s) in Figure 1 (SEQ ID NO:1) : S96C; S101C; S111C; V114C; R115C; E116C; N134C; N140C; E144C; N152C; S153C; R170C; R170K; R170S; K179C; D234C; E249C; R255C; E263C; H264C. A related embodiment of the invention includes such Apo-2 ligand variant polypeptides that are conjugated or linked to one or more polyol groups such as poly(ethylene glycol). Highly preferred embodiments of the invention include Apo-2 ligand variant polypeptides that have such substitution(s) and further bind to a death receptor selected from the group consisting of DR4 receptor and DR5 receptor and/or induce apoptosis in one or more mammalian cells.

A related embodiment of the invention includes isolated nucleic acids comprising a nucleotide sequence encoding such Apo-2 ligand variants, vectors containing such nucleic acids and host cells containing these vectors (e.g. E. *coli).* A related embodiment includes a method of making Apo-2 ligand variant polypeptides by culturing a host cell containing a vector encoding a Apo-2 ligand variant polypeptide in culture media under conditions sufficient to express the Apo-2 ligand variant polypeptide and then recovering and purifying the Apo-2 ligand variant polypeptide.

Yet another embodiment of the invention is an Apo-2 ligand trimer which includes at least one Apo-2 ligand variant polypeptide comprising an amino acid sequence which differs from the native sequence Apo-2 ligand polypeptide sequence of Figure 1 (SEQ ID NO:1) and has one or more amino acid substitutions at the following residue position(s) in Figure 1 (SEQ ID NO:1) : S96; S101; S111; V114; R115; E116; N134; N140; E144; N152; S153; R170; K179; D234; E249; R255; E263; H264. A related embodiment of the invention includes Apo-2 ligand trimers conjugated or linked to one or more polyol groups such as poly(ethylene glycol). In preferred embodiments of the invention these trimers bind to a death receptor selected from the group consisting of DR4 receptor and DR5 receptor.

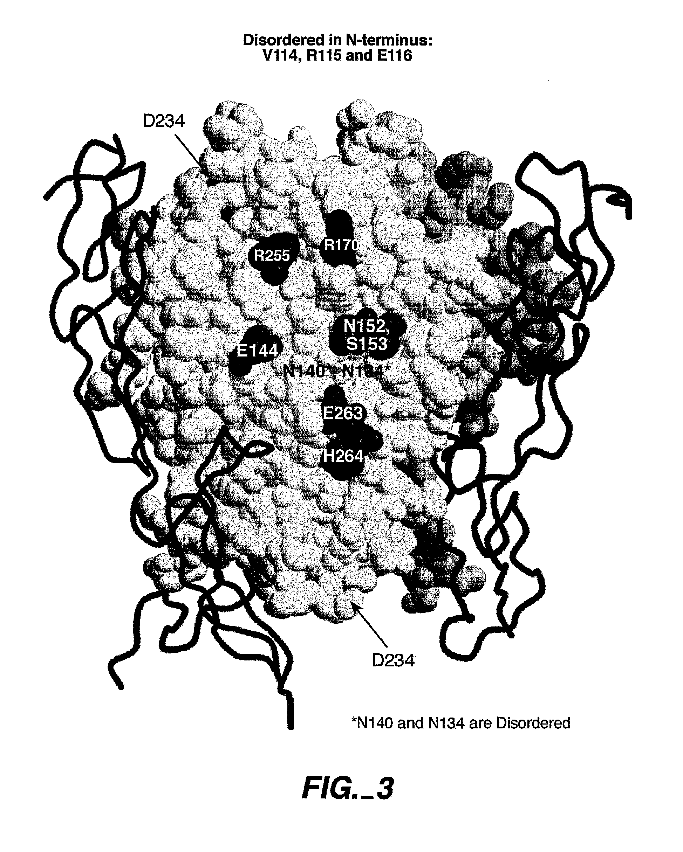

Yet another embodiment of the invention is an isolated Apo-2 ligand variant polypeptide comprising an amino acid sequence which differs from the native sequence Apo-2 ligand polypeptide sequence of Figure 1 (SEQ ID NO:1) and has one or more amino acid substitutions at a residue position identified from an x-ray crystal structure of the DR5•Apo2L complex as shown in Figure 3. In preferred embodiments, the residue position is both outside of the receptor contact region of the DR5•Apo2L complex and displays high solvent accessibility. In highly preferred embodiments, the residue position of such isolated Apo-2 ligand variant polypeptides is located on one face of the Apo2L monomer from top to bottom as shown in the crystal structure of the DR5•Apo2L complex provided in Figure 3. A related embodiment of the invention includes such Apo-2 ligand variant polypeptides conjugated or linked to one or more polyol groups such as poly(ethylene glycol). Highly preferred embodiments of the invention include Apo-2 ligand variant polypeptides that have such substitution(s) and further bind to a death receptor selected from the group consisting of DR4 receptor and DR5 receptor and/or induce apoptosis in one or more mammalian cells.

Yet another embodiment of the invention includes Apo-2 ligand trimer oligomers comprising at least two Apo-2 ligand trimers, wherein at least one Apo-2 ligand monomer in each Apo-2 ligand trimer comprises an Apo-2 ligand variant polypeptide having a cysteine amino acid substitution at amino acid residue position 170 in Figure 1 (SEQ ID NO:1), and wherein the Apo-2 ligand trimers are linked by disulfide bonds between the cysteine amino acid residues at position 170 in the Apo-2 ligand variant polypeptides.

In another embodiment, the invention provides a formulation comprising Apo-2 ligand variant polypeptide. In particular, the invention provides compositions comprising one or more Apo-2 ligand variant polypeptides and a carrier, such as a pharmaceutically-acceptable carrier, and optionally one or more divalent metal ions. In one embodiment, such composition may be included in an article of manufacture or kit. The composition may be a pharmaceutically acceptable formulation useful, for instance, in inducing or stimulating apoptosis in mammalian cancer cells or for treating an immune related disorder, such as arthritis or multiple sclerosis.

In addition, therapeutic methods for using Apo-2 ligand variant polypeptides are provided.

Particular embodiments of the invention include isolated Apo-2 ligand variant polypeptides comprising an amino acid sequence which differs from the native sequence Apo-2 ligand polypeptide sequence of Figure 1 (SEQ ID NO:1) and has one or more of the following amino acid substitutions at the residue position(s) in Figure 1 (SEQ ID NO:1) : S96C; S101C; S111C; V114C; R115C; E116C; N134C; N140C; E144C; N152C; S153C; R170C; R170K; R170S; K179C; D234C; E249C; R255C; E263C; H264C. Isolated nucleic acids, vectors and host cells comprising a nucleotide sequence encoding such Apo-2 ligand variants are further provided. Methods of making Apo-2 ligand variant polypeptide, comprising the steps of: providing such host cell(s), providing culture media; culturing the host cell(s) in the culture media under conditions sufficient to express the Apo-2 ligand variant polypeptide; recovering the Apo-2 ligand variant polypeptide from the host cell or culture media; and purifying the Apo-2 ligand variant polypeptide. Optionally, the Apo-2 ligand variant polypeptides are conjugated or linked to one or more polyol groups that increase the actual molecular weight of the Apo-2 ligand variant polypeptide. Optionally, such Apo-2 ligand variant polypeptide is conjugated or linked to one molecule of poly(ethylene glycol) having a molecular weight of 2000 Daltons or about 2000 Daltons.

Further embodiments of the invention include Apo-2 ligand trimers comprising at least one Apo-2 ligand variant polypeptide comprising an amino acid sequence which differs from the native sequence Apo-2 ligand polypeptide sequence of Figure 1 (SEQ ID NO:1) and has one or more amino acid substitutions at the following residue position(s) in Figure 1 (SEQ ID NO:1): S96; S101; S111; V114; R115; E116; N134; N140; E144; N152; S153; R170; K179; D234; E249; R255; E263; H264.

Further embodiments of the invention include isolated Apo-2 ligand variant polypeptides comprising an amino acid sequence which differs from the native sequence Apo-2 ligand polypeptide sequence of Figure 1 (SEQ ID NO:1) and has one or more amino acid substitutions at a residue position identified from an x-ray crystal structure of the DR5•Apo2L complex as shown in Figure 3 such that the residue position is:
(a) outside of the receptor contact region of the DR5•Apo2L complex as shown in Figure 3; and
(b) displays high solvent accessibility in the crystal structure of the DR5•Apo2L complex as shown in Figure 3. Optionally, such Apo-2 ligand variant polypeptides have one or more of the following amino acid substitutions at the residue position(s) in Figure 1 (SEQ ID NO:1): S96; S101; S111; V114; R115; E116; N134; N140; E144; N152; S153; R170; K179; D234; E249; R255; E263; H264. Optionally, such Apo-2 ligand variant polypeptides is conjugated or linked to one or more polyol groups.

Further embodiments of the invention include pharmaceutical compositions comprising an effective amount of isolated Apo-2 ligand variant polypeptide comprising an amino acid sequence which differs from the native sequence Apo-2 ligand polypeptide sequence of Figure 1 (SEQ ID NO:1) and has one or more of the following amino acid substitutions at the residue position(s) in Figure 1 (SEQ ID NO:1): S96C; S101C; S111C; V114C; R115C; E116C; N134C; N140C; E144C; N152C; S153C; R170C; R170K; R170S; K179C; D234C; E249C; R255C; E263C; and H264C, in admixture with a pharmaceutically acceptable carrier. Optionally, such pharmaceutical compositions comprise one or more divalent metal ions.

Further embodiments of the invention include methods of inducing apoptosis in mammalian cells comprising exposing mammalian cells expressing a receptor selected from the group consisting of DR4 receptor and DR5 receptor to a therapeutically effective amount of isolated Apo-2 ligand variant polypeptide comprising an amino acid sequence which, differs from the native sequence Apo-2 ligand polypeptide sequence of Figure 1 (SEQ ID NO:1) and has one or more of the following amino acid substitutions at the residue position(s) in Figure 1 (SEQ ID NO:1): S96C; S101C; S111C; V114C; R115C; E116C; N134C; N140C; E144C; N152C; S153C; R170C; R170K; R170S; K179C; D234C; E249C; R255C; E263C; H264C.

Further embodiments of the invention include methods of treating cancer in a mammal, comprising administering to said mammal an effective amount of isolated Apo-2 ligand variant polypeptide comprising an amino acid sequence which differs from the native sequence Apo-2 ligand polypeptide sequence of Figure 1 (SEQ ID NO:1) and has one or more of the following amino acid substitutions at the residue position(s) in Figure 1 (SEQ ID NO:1): S96C; S101C; S111C; V114C; R115C; E116C; N134C; N140C; E144C; N152C; S153C; R170C; R170K; R170S; K179C; D234C; E249C; R255C; E263C; H264C. Optionally, in the methods, the cancer is lung cancer, breast cancer, colon cancer or colorectal cancer.

Further embodiments of the invention include methods of treating an immune-related disease in a mammal comprising administering to said mammal an effective amount of isolated Apo-2 ligand variant polypeptide comprising an amino acid sequence which differs from the native sequence Apo-2 ligand polypeptide sequence of Figure 1 (SEQ ID NO:1) and has one or more of the following amino acid substitutions at the residue position(s) in Figure 1 (SEQ ID NO:1): S96C; S101C; S111C; V114C; R115C; E116C; N134C; N140C; E144C; N152C; S153C; R170C; R170K; R170S; K179C; D234C; E249C; R255C; E263C; H264C. Optionally, in the methods, the immune-related disease is arthritis or multiple sclerosis.

### BRIEF DESCRIPTION OF THE DRAWINGS

Figure 1 shows the nucleotide sequence (SEQ ID NO:2) of human Apo-2 ligand cDNA and its derived amino acid sequence (SEQ ID NO:1). The "N" at nucleotide position 447 is used to indicate the nucleotide base may be a "T" or "G".

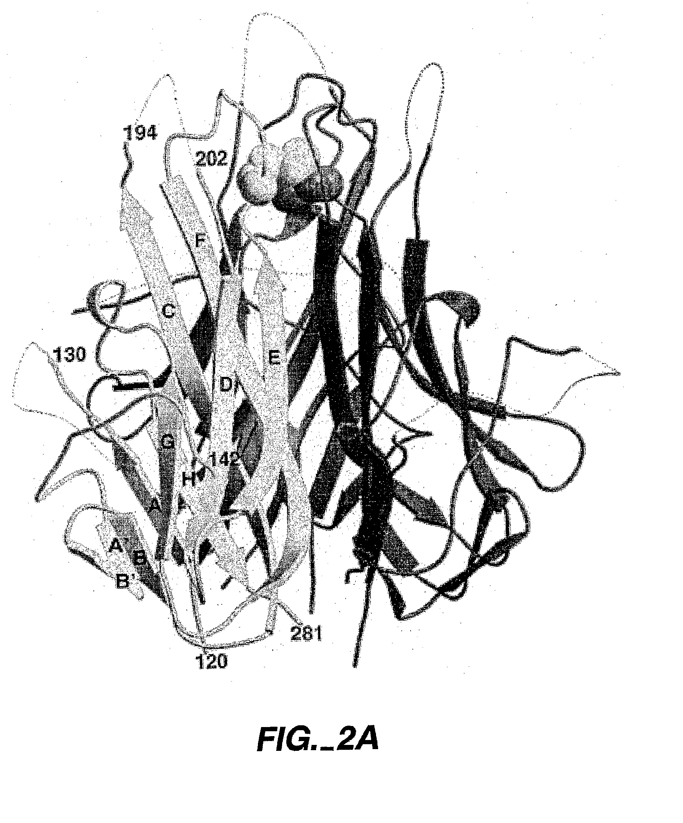

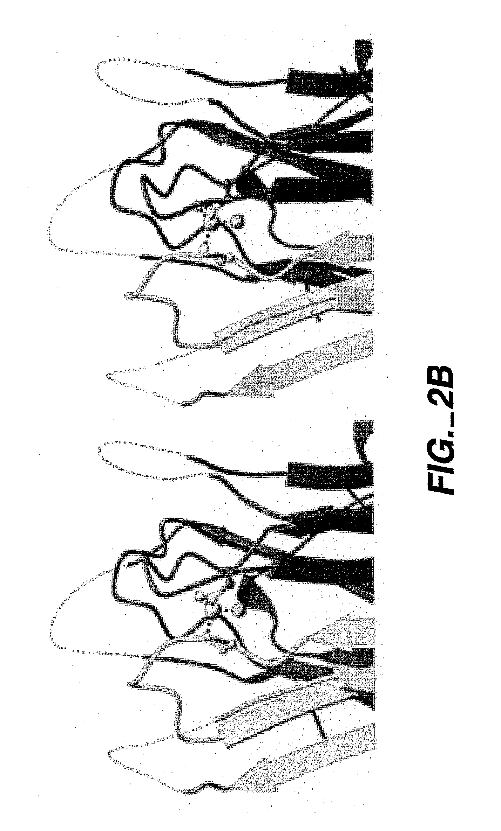

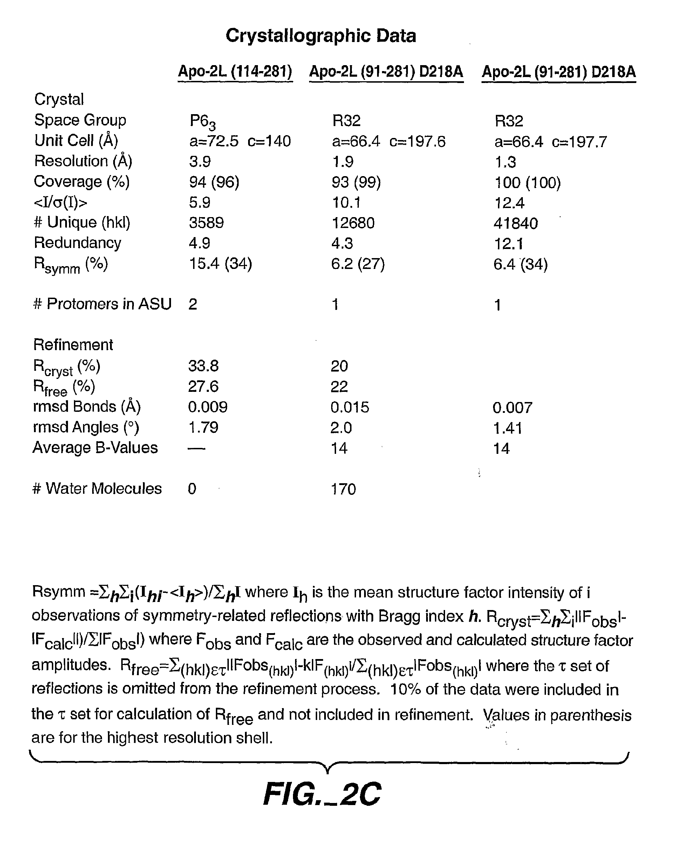

Figures 2A-2C relate to the crystal structure of Apo-2L. Figure 2A shows a view of the trimer along the three fold axis. Each monomer is identical. The ordered protein structure commences at residue 120, residues 131-141 are disordered, as are residues 195-201 (marked as dashed lines). The zinc binding site including the three symmetry related cysteines and the solvent ligand are shown as space filling diagrams. Figure 2B provides cross-eyed stereo close up view of the zinc binding site; the angles between Sγ-zinc-Sγ are 112° and the Sγ-zinc-solvent angles are 107° with 2.3 Angstrom zinc-sulfur and 2.3 Angstrom zinc-solvent bond distances. Figure 2 was made with the programs Molscript [Kraulis et al., J. Appl. Cryst., 24:946-950 (1991)] and Raster3D [Merrit et al., Acta Cryst., D50:869-873 (1994)]. Figure 2C provides a summary of the crystallographic data.
Figure 3 shows a x-ray structure of the DR5•Apo2L complex.
Figure 4 shows the apoptosis-inducing activity of R170C-Apo2L.0 on SK-MES lung carcinoma cells. The increased activity of the R170C variant appears to be related to oxidation of Cys170 during incubation in the bioassay media. Prior alkylation of Cys170 with N-ethylmaleimide (NEM) (Table I) or iodoacetamide blocked the activity increase.

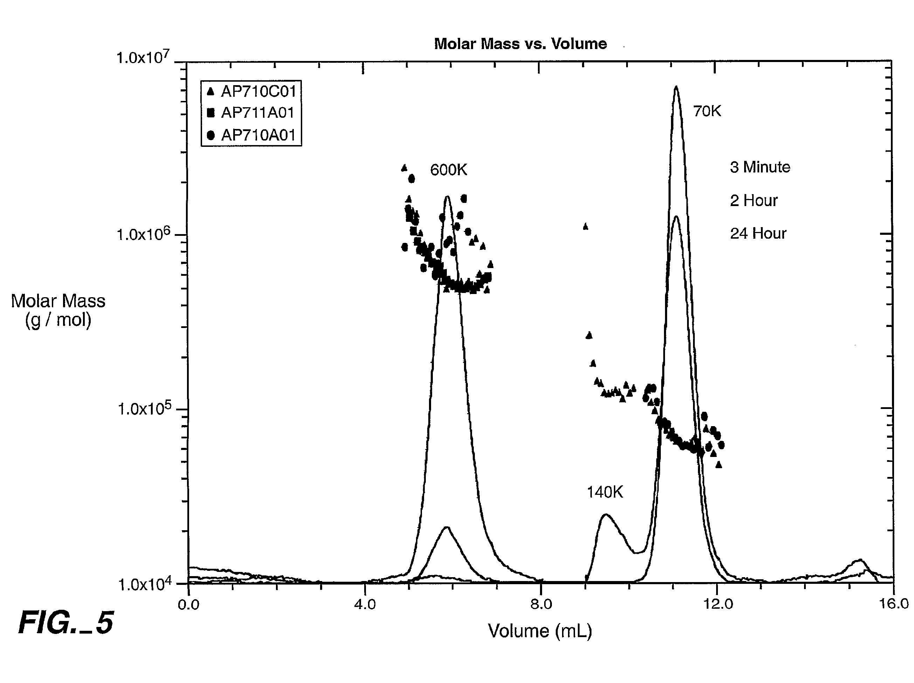

Figure 5 shows an analysis of R170C-Apo2L.0 oligomers by size exclusion chromatography (SEC) on a Superdex 200 column (Amersham Biotech) using a chromatographic system equipped with an on-line light scattering detector (MALS) (Wyatt Technology, Inc.). Solid lines represent the UV trace and symbols indicate the molar mass calculated from the light scattering data. With only 3 minutes of air oxidation R170C-Apo2L.0 is found predominantly in the trimeric form with a calculated molecular weight of 70,000 D (elution volume=11 mLs). At 2 hours significant amounts of higher molecular weight forms are found. The three peaks at 2 hours have calculated molecular weights of 70,000 D, 140,000 D (9.5 mL elution volume) and 600,000 D (6 mL elution volume). After 24 hours only the 600,000 D molecular weight species is found.
Figure 6 shows the kinetics of oligomerization and bioactivity increase for R170C-Apo2L.0. The time course of the increase in bioactivity is concomitant with the accumulation of oligomeric forms.
Figure 7 shows the effects of oxidized R170C-Apo2L.0 on cynomologous monkey hepatocytes.

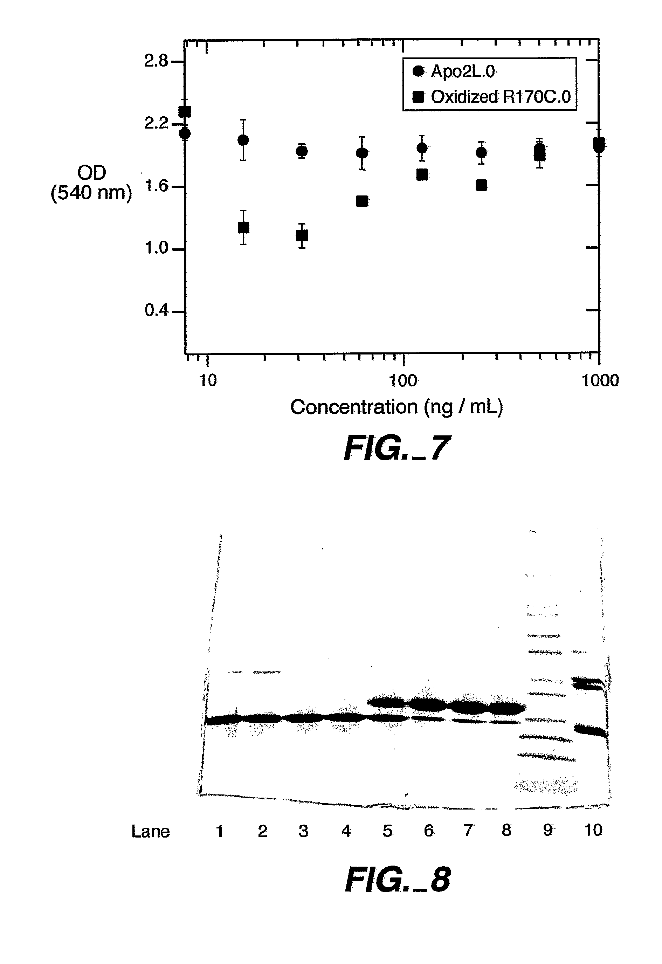

Figure 8 shows a SDS-PAGE analysis of PEGylation reactions. Lanes (left to right) 1,2 - R170C-Apo2L.0, 3 - No PEG-maleimide added, 4 - NEM modified R170C-Apo2L.0, 5 - 1:1 PEG:R170C-Apo2L.0, 6 - 2:1 PEG:R170CApo2L.0, 7 - 5:1 PEG:R170C-Apo2L.0, 8 - 10:1 PEG:R170C-Apo2L.0, 9 - Molecular weight standards, 10 - air oxidized R170C-Apo2L.0. SDS-PAGE indicates an approximately 2000 Dalton shift in the monomer molecular weight upon treatment of R170C-Apo2L.0 with PEG-maleimide. Reactions using PEG:protein ratios of 2:1 or greater gave a similar extent of modification. For these reactions, residual unmodified monomer was observed. Visual inspection of the Coomassie blue-stained gel suggests that unmodified monomer accounts for <10% of the total protein. At PEG:protein molar ratios less than 2:1, less modification was obtained. The reactions appeared to go to completion within 2 hours since no apparent change in the product was observed with a 24 hour reaction time.

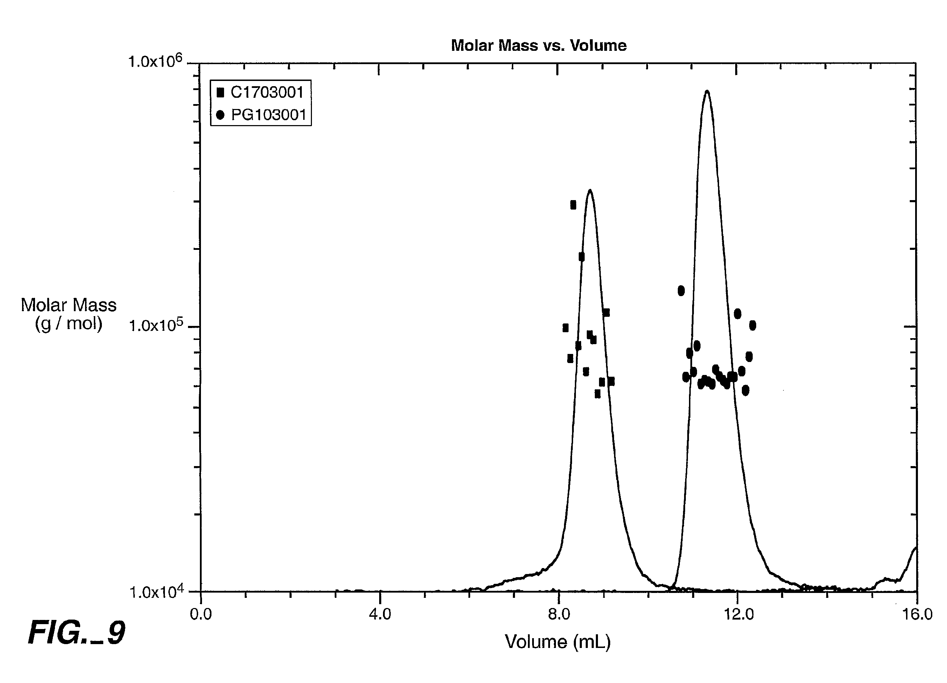

Figure 9 shows the analysis of PEG-R170C-Apo2L.0(32176-87C) by SEC-MALS. A curve for carboxyamidomethyl-R170C-Apo2L.0, with a peak elution volume of 11.3 mL, is shown for comparison. PEGylation causes a decrease in elution volume and increase in apparent molecular weight.

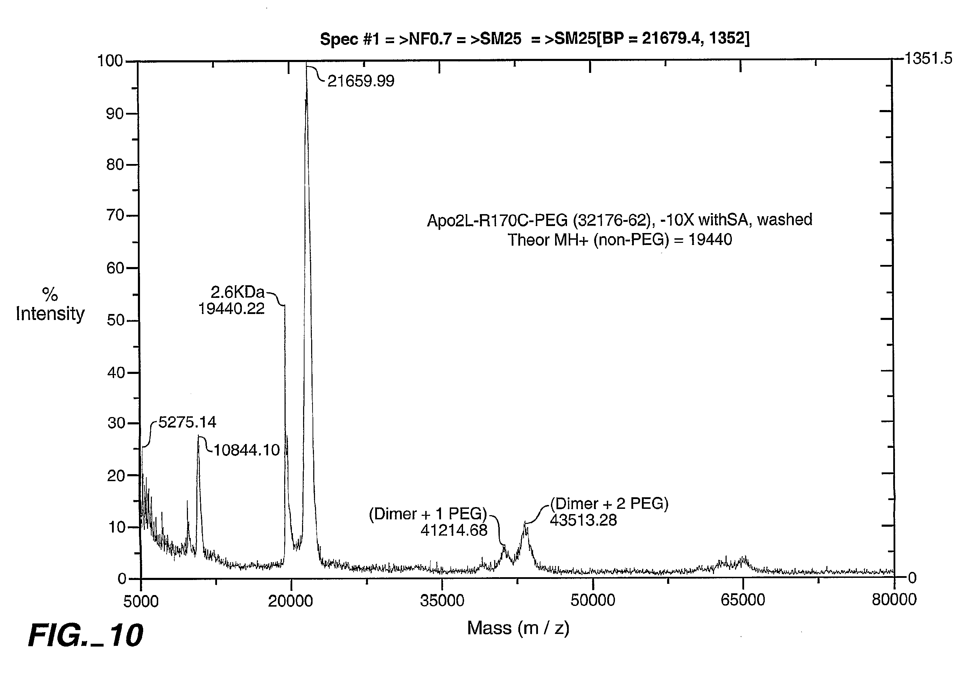

Figure 10 shows the analysis of PEG-R170C-Apo2L.0 by mass spectroscopy. MALDI-TOF-MS indicated the presence of a small amount of unmodified monomer (MW=19,440 D) and a major peak corresponding to protein having a single attached PEG. PEG molecules are well known to have mass heterogeneity, differing in molecular weight by increments of the polymer unit ethylene glycol (MW=44). As a consequence, a broad mass range centered about 21,660 D is observed for the protein with a single PEG attached. The difference in average mass between the pegylated and non-pegylated R170C-Apo2L.0 indicates that the average mass of the PEG is 2200 D.
Figure 11 shows peptide mapping used to confirm the site of PEG attachment. Samples of pegylated and non-pegylated R170C-Apo2L.0 were digested with Lys-C protease and the resulting peptides were separated by reverse phase HPLC. The pattern of peptides produced was compared to the map previously determined for Apo2L.0. A peptide labeled L4, produced by cleavage after Lys15O and Lys179, contains the Cys170 residue in the digest of R170C-Apo2L.0. This peak disappears and is replaced by a broad, later eluting peak (L4*), in the pegylated protein.
Figure 12 shows the pharmacokinetics of PEG-R170C-Apo2L.0(32176-87C) in the mouse. Mice were given tail vein injections of Apo2L.0 (10 mg/kg) or PEG-R170C-Apo2L.0 (10 mg/kg) at time zero. Plasma samples were collected at 1, 20, 40, 60, and 80 minutes. Apo2L concentrations were determined by ELISA. These data show that PEG-R170C-Apo2L.0(32176-87C) has a longer half-life than Apo2L.0.

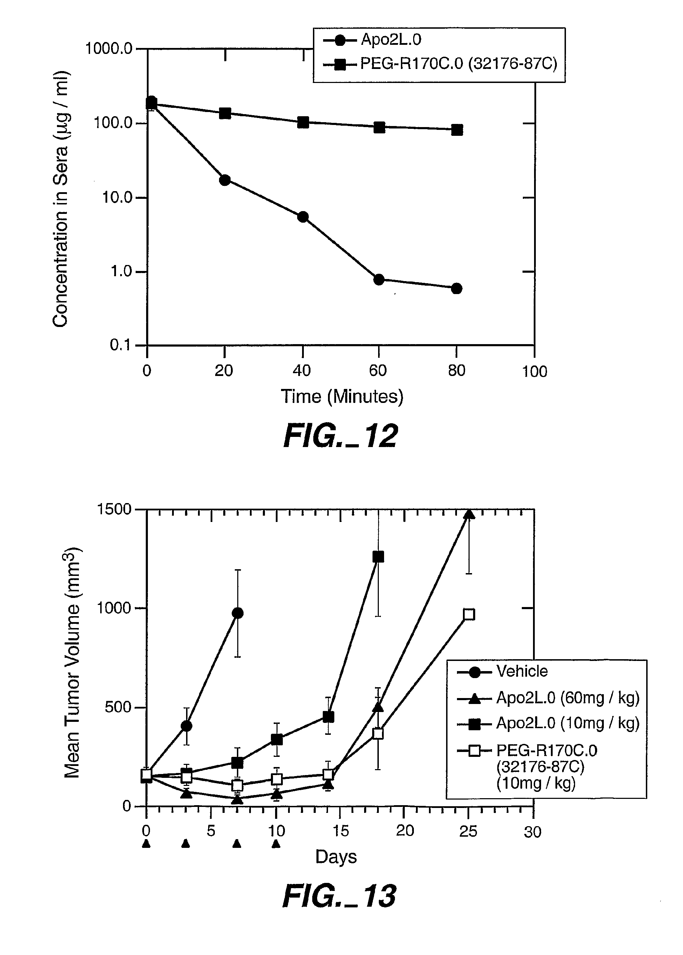

Figure 13 shows the effect of PEG-R170C-Apo2L.0(32176-87C) on the growth of human COLO205 tumors in a mouse xenograft model. Athymic nude mice (Jackson Laboratories) were injected subcutaneously with 5 x 10⁶ COLO205 human colon carcinoma cells (NCI). Tumors were allowed to form and grow to a volume of about 150 mm³ as judged by caliper measurement. Mice (8 per group) were given i.v. injections of vehicle (2x/week), Apo2L.0 (60 mg/kg, 2x/week), Apo2L.0 (10 mg/kg, 2x/week),or PEG-R170C-Apo2L.0(32176-87C) (10 mg/kg, 2x/week). Tumor volume was measured every third day and treatment was stopped after two weeks. Treatment with 10 mg/kg PEG-R170CApo2L.0(32176-87C) caused a greater reduction in tumor volume than an equivalent dose of Apo2L.0.
Figure 14 shows the apoptosis-inducing activity of PEG-R170CApo2L.0(32176-78) on SK-MES lung carcinoma cells. The activity of PEG-R170C-Apo2L.0(32176-78) is increased 39-fold relative to Apo2L.0
Figure 15 shows the analysis of PEG-R170C-Apo2L.0(32176-78) by SEC-MALS. PEG-R170C-Apo2L.0(32176-78) elutes from the column in 3 main peaks. The first peak has a calculated molecular weight of 315,000 D and accounts for 30% of the material injected. The second peak has a calculated molecular weight of 194,000 D and represents 23% of the total. The third peak has a calculated molecular weight of 108,000 D and accounts for 46% of the total mass.
Figure 16 is a schematic drawing of the proposed structure of the "hexameric" component of PEG-R170C-Apo2L.0(32176-78). Two Apo2L trimers are shown in disulfide linkage through Cys170 with the remaining subunits of the trimer having a PEG chain attached to Cys170.
Figure 17 shows the pharmacokinetics of PEG-R170C-Apo2L.0(32176-78) in the mouse. Mice were given tail vein injections of Apo2L.0 (10 mg/kg) or PEG-R170C-Apo2L.0(32176-78) (10 mg/kg) at time zero. Plasma samples were collected at 10 minutes, and 1, 2, 4, 8, and 24 hours. Apo-2L concentrations were determined by ELISA. These data show that PEG-R170CApo2L.0(32176-78) has a 48-fold longer half-life than Apo2L.0.

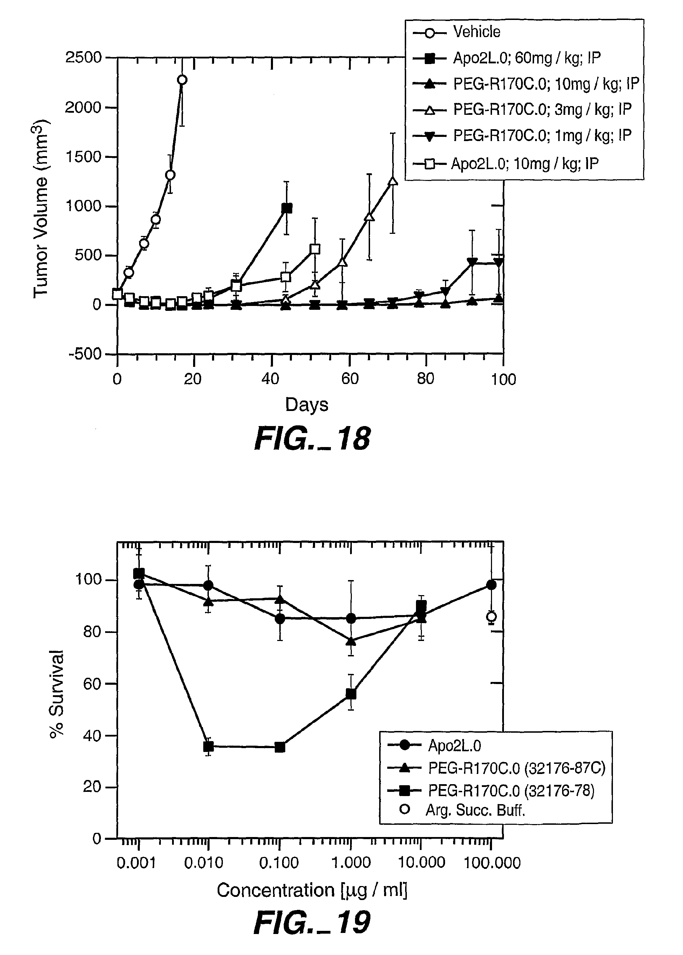

Figure 18 shows the effect of PEG-R170C-Apo2L.0(32176-87C) on the growth of human COLO205 tumors in a mouse xenograft model. Athymic nude mice (Jackson Laboratories) were injected subcutaneously with 5 x 10⁶ COLO205 human colon carcinoma cells (NCI). Tumors were allowed to form and grow to a volume of about 150 mm³ as judged by caliper measurement. Mice (8 per group) were given i.p. injections of vehicle (5x/week), Apo2L.0 (60 mg/kg, 5x/week), Apo2L.0 (10 mg/kg, 2x/week), PEG-R170C-Apo2L.0 (10 mg/kg, 2x/week), PEG-R170C-Apo2L.0 (3 mg/kg, 2x/week), or PEG-R170C-Apo2L.0 (1 mg/kg, 2x/week). Tumor volume was measured every third day and treatment was stopped after two weeks. All three doses of PEG-R170C-Apo2L.0(32176-78) caused complete tumor regression in all 8 animals of each group.
Figure 19 shows the effect of PEG-R170C-Apo2L.0 on survival of normal hepatocytes from the cynomologous monkey. Lot 32176-78 shows the effects at intermediate concentrations whereas lot 32176-87C has no effect on hepatocyte survival.
Figures 20A and 20B show the nucleotide sequence (SEQ ID NO:4) of a cDNA for full length human DR4 and its derived amino acid sequence (SEQ ID NO:3). The respective nucleotide and amino acid sequences for human DR4 are also reported in Pan et al., Science, 276:111 (1997).
Figure 21 shows the 411 amino acid sequence (SEQ ID NO:5) of human DR5 (also referred to as Apo-2) as published in WO 98/51793 on November 19, 1998. A splice variant of human DR5 is known in the art. This DR5 splice variant encodes the 440 amino acid sequence (SEQ ID NO:6) of human DR5 shown in Figures 22A and 22B.
Figures 22A and 22B show the 440 amino acid sequence (SEQ ID NO:6) of human DR5 as published in WO 98/35986 on August 20, 1998.
Figure 23 shows the analysis of 2K PEG-K179C-Apo2L.0 by SEC-MALS.
Figure 24 shows apoptosis-inducing activity of 2K PEG-R179C-Apo2L.0 (referred to in the figures as "2KPEG-K179.0") on SK-MES lung carcinoma cells.
Figure 25 shows the pharmacokinetics of 2KPEG-R179C-Apo2L.0 in the Colo205 mouse model. Plasma samples were collected at the times indicated, and concentrations of the indicated protein were determined by ELISA.
Figure 26 shows the effect of 2K PEG-R179C-Apo2L.0 on the growth of human COLO205 tumors in a mouse xenograft model.

### DETAILED DESCRIPTION OF THE PREFERRED EMBODIMENTS

### I. Definitions

The terms "Apo-2 ligand", "Apo2L", "Apo-2L", and "TRAIL" are used herein to refer to a polypeptide sequence which includes amino acid residues 114-281, inclusive, 95-281, inclusive, residues 92-281, inclusive, residues 91-281, inclusive, residues 41-281, inclusive, residues 15-281, inclusive, or residues 1-281, inclusive, of the amino acid sequence shown in Figure 1, as well as biologically active fragments, deletional, insertional, or substitutional variants of the above sequences. In one embodiment, the polypeptide sequence comprises residues 114-281 of Figure 1 (SEQ ID NO:1). Optionally, the polypeptide sequence comprises residues 95-281, residues 92-281 or residues 91-281 of Figure 1. The Apo-2L polypeptides may be encoded by the native nucleotide sequence shown in Figure 1. Optionally, the codon which encodes residue Proll9 (Figure 1) may be "CCT" or "CCG". In another preferred embodiment, the fragments or variants are biologically active and have at least about 80% amino acid sequence identity, more preferably at least about 90% sequence identity, and even more preferably, at least 95%, 96%, 97%, 98%, or 99% sequence identity with any one of the above sequences. The definition encompasses substitutional variants of Apo-2 ligand in which at least one of its native amino acids are substituted by another amino acid residue, such as a cysteine residue. Preferred substitutional variants include one or more of the residue substitutions identified in Table I below. The definition also encompasses a native sequence Apo-2 ligand isolated from an Apo-2 ligand source or prepared by recombinant or synthetic methods. The Apo-2 ligand of the invention includes the polypeptides referred to as Apo-2 ligand or TRAIL disclosed in WO97/01633 published January 16, 1997, WO97/25428 published July 17, 1997, and WO 01/00832 published January 4, 2001. The terms "Apo-2 ligand", "Apo2L" or "Apo-2L" are used to refer generally to forms of the Apo-2 ligand which include monomer, dimer or trimer forms of the polypeptide. All numbering of amino acid residues referred to in the Apo-2L sequence use the numbering according to Figure 1 (SEQ ID NO:1), unless specifically stated otherwise. For instance, "D203" or "Asp203" refers to the aspartic acid residue at position 203 in the sequence provided in Figure 1 (SEQ ID NO:1).

The term "Apo-2 ligand extracellular domain" or "Apo-2 ligand ECD" refers to a soluble form of Apo-2 ligand which is essentially free of transmembrane and cytoplasmic domains. Ordinarily, the ECD will have less than 1% of such transmembrane and cytoplasmic domains, and preferably, will have less than 0.5% of such domains.

The term "Apo-2 ligand monomer" or "Apo-2L monomer" refers to a covalent chain of an extracellular domain sequence of Apo-2L.

The term "Apo-2 ligand dimer" or "Apo-2L dimer" refers to two Apo-2L monomers joined in a covalent linkage via a disulfide bond. The term as used herein includes free standing Apo-2L dimers and Apo-2L dimers that are within trimeric forms of Apo-2L (i.e., associated with another Apo-2L monomer).

The term "Apo-2 ligand trimer" or "Apo-2L trimer" refers to three Apo-2L monomers that are non-covalently associated.

The term "Apo-2L.0" or "Apo2L.0" refer to a polypeptide consisting of amino acids 114 to 281 of Figure 1 (SEQ ID NO:1) and not linked or conjugated to any epitope tag sequences.

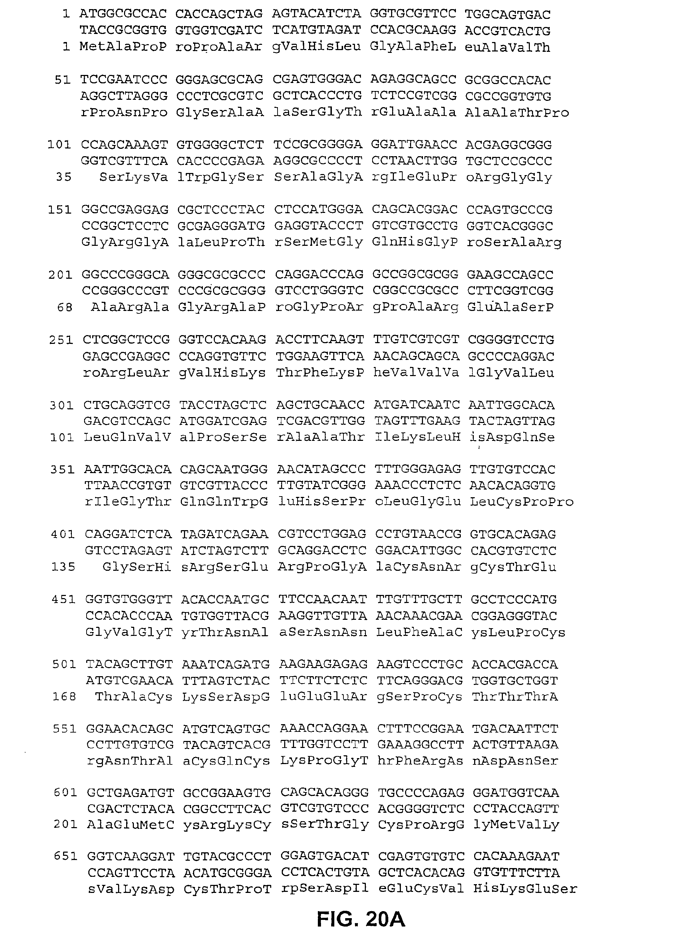

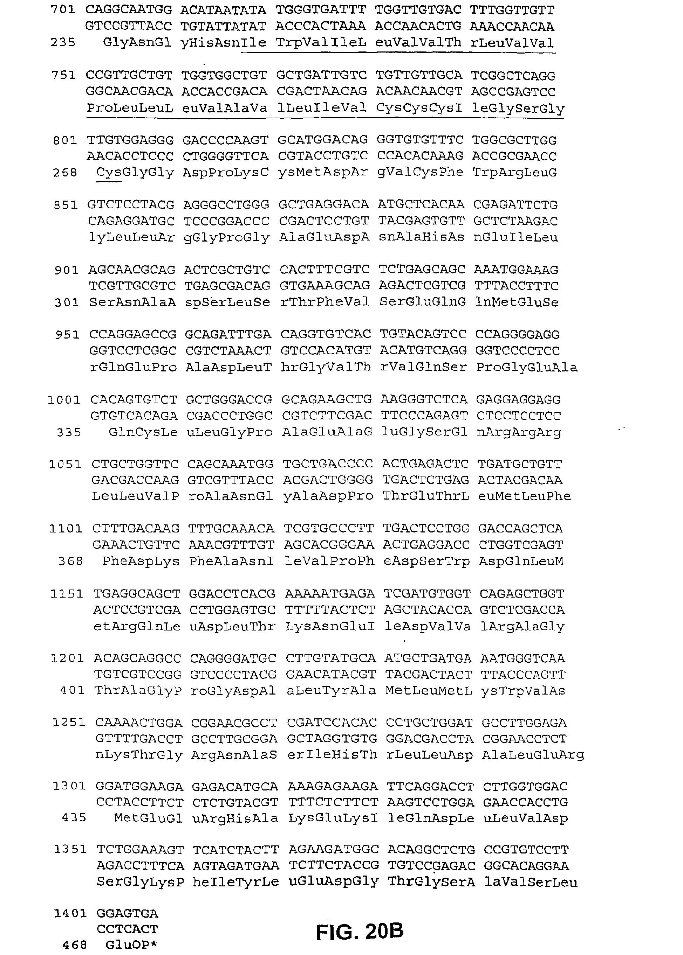

The term "DR4 receptor" as used herein refers to the full length and extracellular domain forms of the receptor described in Pan et al., Science, 276:111-113 (1997)]. The full length amino acid sequence of DR4 receptor is provided in Figs. 20A-20B (SEQ ID NO:4).

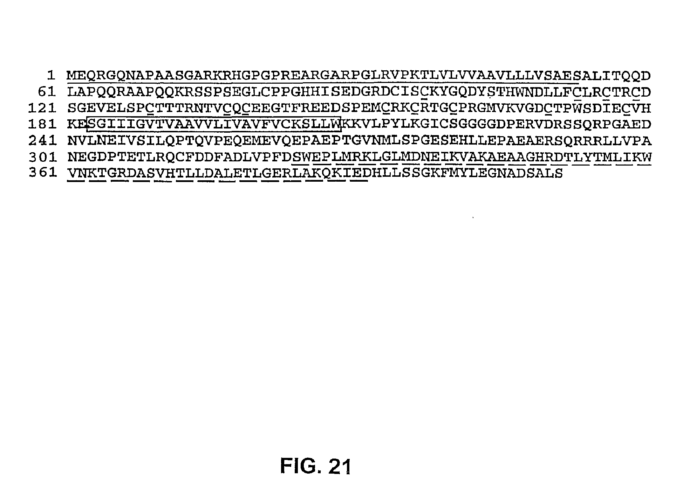

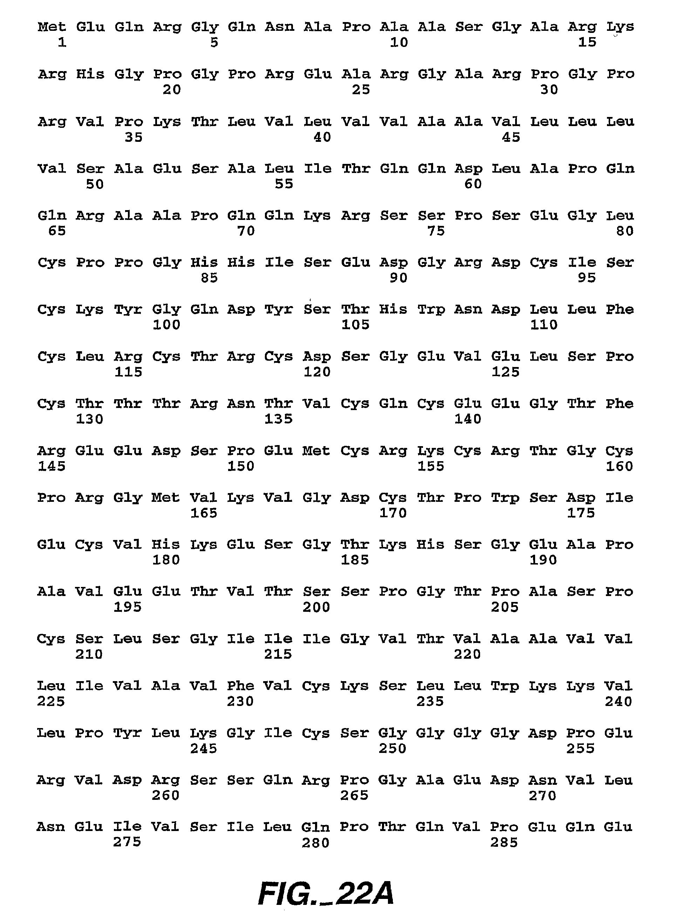

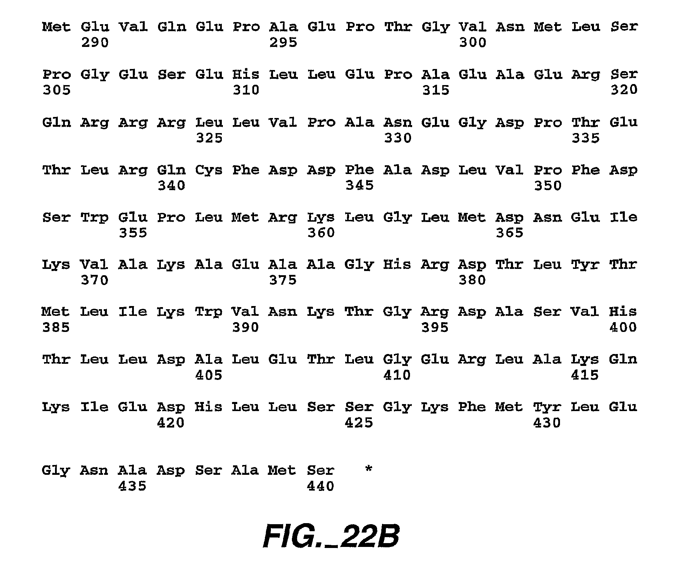

The term "DR5 receptor" as used herein refers to the full length and extracellular domain forms of the receptor described in Sheridan et al., Science, 277:818-821 (1997); Pan et al., Science, 277:815-818 (1997), WO98/51793 published November 19, 1998; WO98/41629 published September 24, 1998; Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP 870,827 published October 14, 1998; WO98/46643 published October 22, 1998; WO99/02653 published January 21, 1999; WO99/091 published February 25, 1999; WO99/11791 published March 11, 1999. The DR5 receptor has also been referred to in the art as Apo-2; TRAIL-R, TR6, Tango-63, hAPO8, TRICK2 or KILLER. The term DR5 receptor used herein includes the full length 411 amino acid polypeptide provided in Fig. 21 (SEQ ID NO:5) and the full length 440 amino acid polypeptide provided in Figs. 22A-B (SEQ ID NO:6).

The term "polyol" when used herein refers broadly to polyhydric alcohol compounds. Polyols can be any water-soluble poly(alkylene oxide) polymer for example, and can have a linear or branched chain. Preferred polyols include those substituted at one or more hydroxyl positions with a chemical group, such as an alkyl group having between one and four carbons. Typically, the polyol is a poly(alkylene glycol), preferably poly(ethylene glycol) (PEG). However, those skilled in the art recognize that other polyols, such as, for example, poly(propylene glycol) and polyethylene-polypropylene glycol copolymers, can be employed using the techniques for conjugation described herein for PEG. The polyols of the invention include those well known in the art and those publicly available, such as from commercially available sources.

The term "conjugate" is used herein according to its broadest definition to mean joined or linked together. Molecules are "conjugated" when they act or operate as if joined.

The term "epitope tagged" when used herein refers to a chimeric polypeptide comprising Apo-2 ligand, or a portion thereof, fused to a "tag polypeptide". The tag polypeptide has enough residues to provide an epitope against which an antibody can be made, yet is short enough such that it does not interfere with activity of the Apo-2 ligand. The tag polypeptide preferably also is fairly unique so that the antibody does not substantially cross-react with other epitopes. Suitable tag polypeptides generally have at least six amino acid residues and usually between about 8 to about 50 amino acid residues (preferably, between about 10 to about 20 residues).

The term "divalent metal ion" refers to a metal ion having two positive charges. Examples of divalent metal ions for use in the present invention include but are not limited to zinc, cobalt, nickel, cadmium, magnesium, and manganese. Particular forms of such metals that may be employed include salt forms (e.g., pharmaceutically acceptable salt forms), such as chloride, acetate, carbonate, citrate and sulfate forms of the above mentioned divalent metal ions. A preferred divalent metal ion for use in the present invention is zinc, and more preferably, the salt form, zinc sulfate. Divalent metal ions, as described herein, are preferably employed in concentrations or amounts (e.g., effective amounts) which are sufficient to, for example, (1) enhance storage stability of Apo-2L trimers over a desired period of time, (2) enhance production or yield of Apo-2L trimers in a recombinant cell culture or purification method, (3) enhance solubility (or reduce aggregation) of Apo-2L trimers, or (4) enhance Apo-2L trimer formation.

"Isolated," when used to describe the various proteins disclosed herein, means protein that has been identified and separated and/or recovered from a component of its natural environment. Contaminant components of its natural environment are materials that would typically interfere with diagnostic or therapeutic uses for the protein, and may include enzymes, hormones, and other proteinaceous or non-proteinaceous solutes. In preferred embodiments, the protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated protein includes protein in situ within recombinant cells, since at least one component of the Apo-2 ligand natural environment will not be present. Ordinarily, however, isolated protein will be prepared by at least one purification step.

An "isolated" Apo-2 ligand nucleic acid molecule is a nucleic acid molecule that is identified and separated from at least one contaminant nucleic acid molecule with which it is ordinarily associated in the natural source of the Apo-2 ligand nucleic acid. An isolated Apo-2 ligand nucleic acid molecule is other than in the form or setting in which it is found in nature. Isolated Apo-2 ligand nucleic acid molecules therefore are distinguished from the Apo-2 ligand nucleic acid molecule as it exists in natural cells. However, an isolated Apo-2 ligand nucleic acid molecule includes Apo-2 ligand nucleic acid molecules contained in cells that ordinarily express Apo-2 ligand where, for example, the nucleic acid molecule is in a chromosomal location different from that of natural cells.

"Percent (%) amino acid sequence identity" with respect to the sequences identified herein is defined as the percentage of amino acid residues in a candidate sequence that are identical with the amino acid residues in the Apo-2 ligand sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art can determine appropriate parameters for measuring alignment, including assigning algorithms needed to achieve maximal alignment over the full-length sequences being compared. For purposes herein, percent amino acid identity values can be obtained using the sequence comparison computer program, ALIGN-2, which was authored by Genentech, Inc. and the source code of which has been filed with user documentation in the US Copyright Office, Washington, DC, 20559, registered under the US Copyright Registration No. TXU510087. The ALIGN-2 program is publicly available through Genentech, Inc., South San Francisco, CA. All sequence comparison parameters are set by the ALIGN-2 program and do not vary.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors; platelet-growth factor; transforming growth factors (TGFs) such as TGF-α and TGF-β; insulin-like growth factor-I and -II; erythropoietin (EPO); osteoinductive factors; interferons such as interferon-α, -β, and - gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-2, IL-3, IL-4, IL-5, IL-6, IL-7, IL-8, IL-9, IL-11, IL-12; and other polypeptide factors including LIF and kit ligand (KL). As used herein, the term cytokine includes proteins from natural sources or from recombinant cell culture and biologically active equivalents of the native sequence cytokines.

The term "cytotoxic agent" as used herein refers to a substance that inhibits or prevents the function of cells and/or causes destruction of cells. The term is intended to include radioactive isotopes *(e.g.,* I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin gamma1I and calicheamicin phiI1, see, e.g., Agnew, Chem Intl. Ed. Engl., 33:183-186 (1994); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (Adriamycin™) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK^{®}; razoxane; rhizoxin; sizofiran; spirogermanium; tenuazonic acid; triaziquone; 2, 2',2 "-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, NJ) and doxetaxel (TAXOTERE^{®}, Rhône-Poulenc Rorer, Antony, France); chlorambucil; gemcitabine (Gemzar™); 6-thioguanine; mercaptopurine; methotrexate; platinum analogs such as cisplatin and carboplatin; vinblastine; platinum; etoposide (VP-16); ifosfamide; mitoxantrone; vincristine; vinorelbine (Navelbine™); novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included in this definition are anti-hormonal agents that act to regulate or inhibit hormone action on tumors such as anti-estrogens and selective estrogen receptor modulators (SERMs), including, for example, tamoxifen (including Nolvadex™), raloxifene, droloxifene, 4-hydroxytamoxifen, trioxifene, keoxifene, LY117018, onapristone, and toremifene (Fareston™); aromatase inhibitors that inhibit the enzyme aromatase, which regulates estrogen production in the adrenal glands, such as, for example, 4(5)-imidazoles, aminoglutethimide, megestrol acetate (Megace™), exemestane, formestane, fadrozole, vorozole (Rivisor™), letrozole (Femara™), and anastrozole (Arimidex™); and anti-androgens such as flutamide, nilutamide, bicalutamide, leuprolide, and goserelin; and pharmaceutically acceptable salts, acids or derivatives of any of the above.

A "growth inhibitory agent" when used herein refers to a compound or composition which inhibits growth of a cell, especially cancer cell overexpressing any of the genes identified herein, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

"Biologically active" or "biological activity" for the purposes herein means (a) having the ability to induce or stimulate apoptosis in at least one type of mammalian cancer cell or virally-infected cell *in vivo* or *ex vivo;* (b) capable of raising an antibody, i.e., immunogenic; (c) capable of binding and/or stimulating a receptor for Apo2L/TRAIL; or (d) retaining the activity of a native or naturally-occurring Apo2L/TRAIL polypeptide. Assays for determining biological activity of the Apo2L/TRAIL can be conducted using methods known in the art, such as cell cytotoxicity, DNA fragmentation (see, e.g., Marsters et al., Curr. Biology, 6: 1669 (1996)), caspase inactivation, DR4 binding, DR5 binding (see, e.g., WO 98/51793, published Nov. 19, 1998), DcR1 (see, e.g., WO 98/58062, published Dec. 23, 1998), DcR2 (see, e.g., WO 99/10484, published March 4, 1999) as well as the assays described in PCT Publication Nos. WO97/01633, WO97/25428, WO 01/00832, and WO 01/22987.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays (such as Alamar blue assays or MTT assays), FACS analysis, DNA fragmentation (see Nicoletti et al., J. Immunol. Methods, 139:271-279 (1991), or poly-ADP ribose polymerase, "PARP", cleavage assay.

As used herein, the term "disorder" in general refers to any condition that would benefit from treatment with the compositions described herein, including any disease or disorder that can be treated by effective amounts of polypeptides such as Apo2L/TRAIL. This includes chronic and acute disorders, as well as those pathological conditions which predispose the mammal to the disorder in question. Non-limiting examples of disorders to be treated herein include benign and malignant cancers; inflammatory, angiogenic, and immunologic disorders, autoimmune disorders, arthritis (including rheumatoid arthritis), multiple sclerosis, and HIV/AIDS.

The terms "cancer", "cancerous", or "malignant" refer to or describe the physiological condition in mammals that is typically characterized by unregulated cell growth. Examples of cancer include but are not limited to, carcinoma, lymphoma, leukemia, blastoma, and sarcoma. More particular examples of such cancers include squamous cell carcinoma, small-cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatoma, breast cancer, colon carcinoma, and head and neck cancer.

The terms "treating", "treatment" and "therapy" as used herein refer to curative therapy, prophylactic therapy, and preventative therapy.

The term "mammal" as used herein refers to any mammal classified as a mammal, including humans, cows, horses, dogs and cats. In a preferred embodiment of the invention, the mammal is a human.

### II. Compositions and Methods of the Invention

A cytokine related to the TNF ligand family, the cytokine identified herein as "Apo-2 ligand" has been described. The predicted mature amino acid sequence of human Apo-2 ligand contains 281 amino acids, and has a calculated molecular weight of approximately 32.5 kDa. The absence of a signal sequence and the presence of an internal hydrophobic region suggests that Apo-2 ligand is a type II transmembrane protein. Soluble extracellular domain Apo-2 ligand polypeptides have also been described. See, e.g., WO97/25428 published July 17, 1997. Apo-2L substitutional variants have further been described. Alanine scanning techniques have been utilized to identify various substitutional variant molecules having biological activity. Particular substitutional variants of the Apo-2 ligand include those in which at least one amino acid is substituted by a cysteine residue. Substitutional variants are identified, for example, as "R115C", "E116C" and "R170C." This nomenclature is used to identify Apo-2 ligand variants wherein the residues at positions 115, 116, and/or 170 (using the numbering shown in Figure 1), respectively, are substituted by cysteine residues. Optionally, the Apo-2L variants may comprise one or more of the substitutions which are recited in Table I below.

The x-ray crystal structure of the extracellular domain of Apo-2 ligand is provided, and alanine-scanning mutagenesis has been performed to provide the mapping of its receptor contact regions. The structure obtained for Apo-2 ligand reveals a homotrimeric protein which contains a novel divalent metal ion (zinc) binding site that coordinates the interaction of the Apo-2 ligand trimer molecule's three subunits.

The x-ray structure of Apo-2L was determined by molecular replacement using a model of TNF-alpha [Eck et al., J. Biol. Chem., 264:17595-17605 (1989)] and refined to 3.9 Angstrom (for the 114-281 residue form) and 1.3 Angstrom (for the D218A variant; 91-281 form). Like other members of the TNF family, Apo-2L appears to comprise a compact trimer formed of three jelly roll monomers which bury approximately 5100 Angstrom² (1700 Angstrom² per monomer) to form the globular trimer (See Figure 2A). The position of the core beta-strands was well conserved compared to the other structurally characterized members of the TNF family, TNF-alpha [Eck et al., supra; Jones et al., Nature, 338:225-228 (1989)], TNF-beta [Eck et al., J. Biol. Chem., 267:2119-2122 (1992)], and CD40L [Karpusas et al., Structure, 3:1031-1039 (1995)], with a r.m.s.d. of 0.8 Angstrom when compared to the core strands of TNF-alpha or TNF-beta. None of the residues in the Apo-2L trimer interface appear to be absolutely conserved across the sequences of the all the presently known human TNF family members; however, the hydrophobic chemical nature of these residues is preserved. The conserved residues in the Apo-2L trimer interface cluster near the base (the widest part of the trimer) and along the three-fold axis. Near the top of the Apo-2L trimer interface in the vicinity of Cys230, the structures appear to diverge, and the conformation of the 190's and 230's loops are variable in each structure.

In contrast to the beta-scaffold core, the structure of the loops and receptor binding surfaces varies considerably among the TNF family members. One difference between the structure of Apo-2 ligand and the structures of TNF-alpha, TNF-beta, and CD40L is the connections between strands A and A'. In TNF-alpha, TNF-beta, and CD40L, strand A is followed by a compact loop. In Apo-2 ligand, a 15-residue insertion lengthens this loop and alters its conformation. The first part of the loop (residues 131 to 141) is disordered while the second part of the loop (residues 142 to 154) crosses the surface of the molecule from one monomer-monomer interface to the next (see Figure 2A) with a conformation that resembles CD40L in its C-terminal portion.

A divalent metal ion (zinc) binding site is buried near the top of the trimerization interface. The TNF family members can be divided by sequence analysis into three groups with respect to Cys230: (1) proteins such as TNF-alpha and Fas ligand in which a cysteine residue at the position corresponding to Cys230 is accompanied by another cysteine in the adjacent loop (the 194-203 loop in Apo-2L) with which it can form a disulfide bridge precluding it from interacting with a metal ion, (2) proteins without a cysteine corresponding to Cys230 (such as TNF-beta and OPGL), and (3) proteins which have only one cysteine residue corresponding to Cys230. Apo-2L and its orthologs in other species meet the latter criteria (i.e., proteins which have only Cys230) and are expected to bind divalent metal ions at the trimer surface. The conformation of the main chain immediately prior to Cys230 in Apo-2L differs from the disulfide containing TNF family members such as TNF-alpha and CD40L. In Apo-2L, the side chain of Cys230 is oriented towards the interface instead of away from it.

The Cys230 residue in each Apo-2L monomer point inward toward the trimer axis and coordinate a divalent metal ion in conjunction with an interior solvent molecule. This divalent metal ion binding site exhibits slightly distorted tetrahedral geometry with bonds and angles appropriate for a zinc binding site and is completely inaccessible to solvent (see Figure 2B). The identity of the bound metal was confirmed using inductively coupled plasma atomic emission spectrometry (ICP-AES). In a quantitative analysis for Cd, Co, Zn, Ni, and Cu using ICP-AES, 0.79 moles of Zn and 0.06 moles of Co per molecule of Apo-2L trimer were detected demonstrating that the bound ion in the structure was zinc at approximately a one to one molar ratio. The importance, of this site was demonstrated by the observation that alanine substitution of Cys230 resulted in a >8-fold decreased apoptotic activity. Furthermore, removal of the bound metal from Apo-2L by dialysis against chelating agents resulted in a 7-fold decrease in DR5 affinity and a >90-fold decrease in apoptotic activity. Upon removal of the Zn, the cysteines became prone to oxidation and disulfide-linked Apo-2L dimers were formed which had decreased apoptotic activity. Since the metal binding site appears to be buried in the Apo-2L trimer structure and is not expected to contact receptor, the data suggests that divalent metal ion binding may be important to maintain the trimer structure and stability of Apo-2L.

The crystal structure of the complex between Apo-2 ligand and an extracellular domain sequence of Apo-2 receptor (DR5) has been determined. (see, Hymowitz et al., Mol. Cell., 4:563-571 (1999)). Apo-2 resembles TNFR1 in overall structure with relatively little defined secondary structure. It is tethered into an elongated shape by a series of seven disulfide bridges, six of which are found in subdomains of Apo-2 (residues 43-84 and 85-130, respectively) that correspond structurally to the second and third CRDs of the TNFR1 receptor.

The interface of the Apo-2 ligand/Apo-2 complex is divided into two patches- patch A and patch B. The dominant characteristic of patch B in the Apo-2L/Apo-2 interface is the interaction between Tyr 216 of Apo-2L (using the numbering of the amino acid sequence for Apo-2L provided in Figure 1) and the 50s loop of the Apo-2 receptor. Residue Tyr 216 is conserved in many of the TNF superfamily ligands (including TNF-alpha, TNF-beta, FasL and OPGL), while other members have a similar large hydrophobic residue at this position. Mutagenesis studies on TNF-alpha, TNF-beta, FasL and Apo-2L have all shown that this residue is critical for binding (Schneider et al., J. Biol. Chem., 272:18827-18833 (1997); Goh et al., Protein Eng., 4:785-791 (1991); Yamagishi et al., Protein Eng., 3:713-719 (1990); Van Ostade et al., Protein Eng., 7:5-22 (1990); Hymowitz et al., personal communication). The interactions of the tyrosine side chain are conserved between the Apo-2L/Apo-2 and TNF-beta-TNFR1 complexes. Moreover, the backbone conformation of the 50s loop of the receptor, which forms the binding pocket for the side chain, is virtually identical between Apo-2 and the TNFR1 (rmsd of only 0.35 between the C-alpha atoms of residues 51 to 62). Additionally, the length of this loop is conserved among the different TNF receptor superfamily members. It is believed that this loop may function as a general hydrophobic binding patch interacting with conserved hydrophobic features on the ligand which may help properly orient the upper part of the receptor for more specific contacts mediated by CRD3.

In contrast to the conserved interactions in patch B, patch A near the bottom of the interface involves interactions made by the 90s loop on CRD3 of Apo-2, which has a completely different conformation than the corresponding loop in the TNFR1.

In patch B, it is believed that the 50s loop of the receptor and Apo-2 ligand residue 216 provide a hydrophobic patch generally important for binding, whereas in patch A, the receptor 90s loop and the Apo-2 ligand residue at or near position 205 control the specificity and cross-reactivity. The 50s loop and the 90s loop of the Apo-2 receptor are believed to carry most of the ligand-binding determinants. The histidine and phenylalanine residues at positions 53 and 59, respectively, of the Apo-2 sequence are both relatively large residues. These two residues are believed to contact residues Asp218 and Ser159 of the Apo-2 ligand; thus introducing larger side chains at the 53 and 59 positions of the Apo-2 sequence may adversely affect Apo-2L affinity for Apo-2 (but improve affinity for DR4).

In order to characterize Apo-2 ligand receptor binding and activity, sites for amino acid substitution were chosen on the basis of examination of the x-ray structure of the DR5•Apo-2L complex (Figure 3). To avoid loss of activity upon mutation or subsequent modification of substituted cysteine amino acid residues, sites outside of the receptor contact region were considered for mutagenesis. In order to ensure efficient chemical modification of the cysteine side chain, residues that displayed high solvent accessibility in the crystal structure were selected. Residues that matched these criteria include, but are not limited to, Glu144, Asn152, Ser153, Arg170, Asp234, Glu249, Arg255, Glu263, and His264. In addition, Va1114, Arg115, Glu116, Asn134 and Asn140 were chosen as sites for cysteine substitution. These residues are in disordered parts of the molecule in the Apo-2L-DR5 crystal structure and thus are presumed to be solvent accessible and do not contribute to receptor binding. As shown in Figure 3, this set of residues spans one face of the Apo-2L monomer from top to bottom. Of the cysteine-substituted Apo-2L proteins experimentally tested, E116C gave significantly reduced apoptotic activity on SK-MES cells (see Table I). The R170C variant exhibited about a 10-fold increased potency. In addition, Apo-2L variants having Arg170 substituted with either Ala, Lys, or Ser residues had activities comparable to the Apo-2L.0 polypeptide. It is believed that in certain embodiments of the invention, preferred Apo-2L variants will comprise native residues (i.e., will not be mutated) at positions corresponding to E116, N134, N140 and/or R255 in the Apo-2L sequence of Figure 1.

The description below relates to methods of producing Apo-2 ligand variants by culturing host cells transformed or transfected with a vector containing Apo-2 ligand variant encoding nucleic acid and recovering the polypeptide from the cell culture.

The DNA encoding Apo-2 ligand may be obtained from any cDNA library prepared from tissue believed to possess the Apo-2 ligand mRNA and to express it at a detectable level. Accordingly, human Apo-2 ligand DNA can be conveniently obtained from a cDNA library prepared from human tissues, such as the bacteriophage library of human placental cDNA as described in WO97/25428. The Apo-2 ligand-encoding gene may also be obtained from a genomic library or by oligonucleotide synthesis.

Libraries can be screened with probes (such as antibodies to the Apo-2 ligand or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding Apo-2 ligand is to use PCR methodology [Sambrook et al., supra; Dieffenbach et al., PCR Primer:A Laboratory Manual (Cold Spring Harbor Laboratory Press, 1995)] .

Amino acid sequence fragments or variants of Apo-2 ligand can be prepared by introducing appropriate nucleotide changes into the Apo-2 ligand DNA, or by synthesis of the desired Apo-2 ligand polypeptide. Such fragments or variants represent insertions, substitutions, and/or deletions of residues within or at one or both of the ends of the intracellular region, the transmembrane region, or the extracellular region (such as the 114-281 amino acid form), or of the amino acid sequence shown for the full-length Apo-2 ligand in Figure 1. Any combination of insertion, substitution, and/or deletion can be made to arrive at the final construct, provided that the final construct possesses, for instance, a desired biological activity or apoptotic activity as defined herein. In a preferred embodiment, the fragments or variants have at least about 80% amino acid sequence identity, more preferably, at least about 90% sequence identity, and even more preferably, at least 95%, 96%, 97%, 98% or 99% sequence identity with the sequences identified herein for the intracellular, transmembrane, or extracellular domains of Apo-2 ligand, or the full-length sequence for Apo-2 ligand. The amino acid changes also may alter post-translational processes of the Apo-2 ligand, such as changing the number or position of glycosylation sites or altering the membrane anchoring characteristics.

Variations in the Apo-2 ligand sequence as described above can be made using any of the techniques and guidelines for conservative and non-conservative mutations set forth in U.S. Pat. No. 5,364,934. These include oligonucleotide-mediated (site-directed) mutagenesis, alanine scanning, and PCR mutagenesis.

Scanning amino acid analysis can be employed to identify one or more amino acids along a contiguous sequence. Among the preferred scanning amino acids are relatively small, neutral amino acids. Such amino acids include alanine, glycine, serine and cysteine. Alanine is typically a preferred scanning amino acid among this group because it eliminates the side-chain beyond the beta-carbon and is less likely to alter the main-chain conformation of the variant. [Cunningham et al., Science, 244:1081 (1989)]. Alanine is also typically preferred because it is the most common amino acid. Further, it is frequently found in both buried and exposed positions [Creighton, The Proteins, (W.H. Freeman & Co., NY); Chothia, J. Mol. Biol., 150:1 (1976)].

Particular Apo-2L variants of the present invention include those Apo-2L polypeptides which include one or more of the recited substitutions provided in TABLE I below. Such Apo-2L variants will typically comprise a non-naturally occurring amino acid sequence which differs from a native sequence Apo-2L (such as provided in Figure 1; for a full length or mature form of Apo-2L or an extracellular domain sequence thereof) in at least one or more amino acids. Optionally, the one or more amino acids which differ in the Apo-2L variant as compared to a native sequence Apo-2L will comprise amino acid substitution(s) such as those indicated in Table I. Apo-2L variants of the invention include soluble Apo-2L variants comprising residues 91-281, 92-281, 95-281 or 114-281 of Figure 1 and having one or more amino acid substitutions recited in TABLE I. Preferred Apo-2L variants will include those variants comprising residues 91-281, 92-281, 95-281 or 114-281 of Figure 1 and having one or more amino acid substitutions recited in TABLE I, and which further have a desired biological activity, such as described herein.

Variations in the Apo-2 ligand sequence also included within the scope of the invention relate to amino-terminal derivatives or modified forms. Such Apo-2 ligand sequences include any of the Apo-2 ligand variants described herein having a methionine or modified methionine (such as formyl methionyl or other blocked methionyl species) at the N-terminus of the polypeptide sequence.

The nucleic acid (e.g., cDNA or genomic DNA) encoding native or variant Apo-2 ligand may be inserted into a replicable vector for further cloning (amplification of the DNA) or for expression.' Various vectors are publicly available. The vector components generally include, but are not limited to, one or more of the following: a signal sequence, an origin of replication, one or more marker genes, an enhancer element, a promoter, and a transcription termination sequence, each of which is described below. Optional signal sequences, origins of replication, marker genes, enhancer elements and transcription terminator sequences that may be employed are known in the art and described in further detail in W097/25428.

Expression and cloning vectors usually contain a promoter that is recognized by the host organism and is operably linked to the Apo-2 ligand variant nucleic acid sequence. Promoters are untranslated sequences located upstream (5') to the start codon of a structural gene (generally within about 100 to 1000 bp) that control the transcription and translation of a particular nucleic acid sequence, such as the Apo-2 ligand variant nucleic acid sequence, to which they are operably linked. Such promoters typically fall into two classes, inducible and constitutive. Inducible promoters are promoters that initiate increased levels of transcription from DNA under their control in response to some change in culture conditions, e.g., the presence or absence of a nutrient or a change in temperature. At this time a large number of promoters recognized by a variety of potential host cells are well known. These promoters are operably linked to Apo-2 ligand variant encoding DNA by removing the promoter from the source DNA by restriction enzyme digestion and inserting the isolated promoter sequence into the vector. Both the native Apo-2 ligand promoter sequence and many heterologous promoters may be used to direct amplification and/or expression of the Apo-2 ligand DNA.

Promoters suitable for use with prokaryotic and eukaryotic hosts are known in the art, and are described in further detail in WO97/25428.

A preferred method for the production of Apo-2L in E. coli employs an inducible promoter for the regulation of product expression. The use of a controllable, inducible promoter allows for culture growth to the desirable cell density before induction of product expression and accumulation of significant amounts of product which may not be well tolerated by the host.

Three inducible promoter systems (T7 polymerase, trp and alkaline phosphatase (AP)) have been evaluated by Applicants for the expression of Apo-2L (form 114-281). The use of each of these three promoters resulted in significant amounts of soluble, biologically active Apo-2L trimer being recovered from the harvested cell paste. The AP promoter is preferred among these three inducible promoter systems tested because of tighter promoter control and the higher cell density and titers reached in harvested cell paste.

Construction of suitable vectors containing one or more of the above-listed components employs standard ligation techniques. Isolated plasmids or DNA fragments are cleaved, tailored, and re-ligated in the form desired to generate the plasmids required.

For analysis to confirm correct sequences in plasmids constructed, the ligation mixtures can be used to transform E. *coli* K12 strain 294 (ATCC 31,446) and successful transformants selected by ampicillin or tetracycline resistance where appropriate. Plasmids from the transformants are prepared, analyzed by restriction endonuclease digestion, and/or sequenced using standard techniques known in the art. [See, e.g., Messing et al., Nucleic Acids Res., 9:309 (1981); Maxam et al., Methods in Enzymology, 65:499 (1980)].

Expression vectors that provide for the transient expression in mammalian cells of DNA encoding Apo-2 ligand variant may be employed. In general, transient expression involves the use of an expression vector that is able to replicate efficiently in a host cell, such that the host cell accumulates many copies of the expression vector and, in turn, synthesizes high levels of a desired polypeptide encoded by the expression vector [Sambrook et al., supra]. Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying analogs and variants of Apo-2 ligand that are biologically active.

Other methods, vectors, and host cells suitable for adaptation to the synthesis of Apo-2 ligand variant in recombinant vertebrate cell culture are described in Gething et al., Nature, 293:620-625 (1981); Mantei et al., Nature, 281:40-46 (1979); EP 117,060; and EP 117,058.

Suitable host cells for cloning or expressing the DNA in the vectors herein include prokaryote, yeast, or higher eukaryote cells. Suitable prokaryotes for this purpose include but are not limited to eubacteria, such as Gram-negative or Gram-positive organisms, for example, *Enterobacteriaceae* such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* Preferably, the host cell should secrete minimal amounts of proteolytic enzymes.

*E. coli* is the preferred host cell for use in the present invention. *E. coli* is particularly well suited for the expression of Apo-2 ligand (form 114-281) , a polypeptide of under 20kd in size with no glycosylation requirement. As a production host, *E. coli* can be cultured to relatively high cell density and is capable of producing relatively high levels of heterologous proteins.

In addition to prokaryotes, eukaryotic microbes such as filamentous fungi or yeast are suitable cloning or expression hosts for Apo-2 ligand-encoding vectors. Suitable host cells for the expression of glycosylated Apo-2 ligand are derived from multicellular organisms. Examples of all such host cells, including CHO cells, are described further in WO97/25428.

Host cells are transfected and preferably transformed with the above-described expression or cloning vectors for Apo-2 ligand production and cultured in nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in Sambrook et al., supra, or electroporation is generally used for prokaryotes or other cells that contain substantial cell-wall barriers. Infection with Agrobacterium tumefaciens is used for transformation of certain plant cells, as described by Shaw et al., Gene, 23:315 (1983) and WO 89/05859 published 29 June 1989. In addition, plants may be transfected using ultrasound treatment as described in WO 91/00358 published 10 January 1991.

For mammalian cells without such cell walls, the calcium phosphate precipitation method of Graham and van der Eb, Virology, 52:456-457 (1978) may be employed. General aspects of mammalian cell host system transformations have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Prokaryotic cells used to produce Apo-2 ligand variant may be cultured in suitable culture media as described generally in Sambrook et al., supra. Mammalian host cells used to produce Apo-2 ligand may be cultured in a variety of culture media.

Examples of commercially available culture media include Ham's F10 (Sigma), Minimal Essential Medium ("MEM", Sigma), RPMI-1640 (Sigma), and Dulbecco's Modified Eagle's Medium ("DMEM", Sigma). Any such media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

In general, principles, protocols, and practical techniques for maximizing the productivity of mammalian cell cultures can be found in Mammalian Cell Biotechnology: A Practical Approach, M. Butler, ed. (IRL Press, 1991).

Optionally, the Apo-2 ligand polypeptide compositions described herein include divalent metal ions such as Zinc. The presence of divalent metal ions in the methods and formulations described herein may protect against disulfide bond formation. It appears that inclusion of divalent metal ions during the process of synthesis and assembly of Apo-2L trimers may further improve accumulation and recovery of properly folded, homotrimeric Apo-2L. Accordingly, in accordance with another aspect of the present invention, one or more divalent metal ions will typically be added to or included in the culture media for culturing or fermenting the host cells. The divalent metal ions are preferably present in or added to the culture media at a concentration level sufficient to enhance storage stability, enhance solubility, or assist in forming stable Apo-2L trimers coordinated by one or more zinc ions. The amount of divalent metal ions which may be added will be dependent, in part, on the host cell density in the culture or potential host cell sensitivity to such divalent metal ions. At higher host cell densities in the culture, it may be beneficial to increase the concentration of divalent metal ions. If the divalent metal ions are added during or after product expression by the host cells, it may be desirable to adjust or increase the divalent metal ion concentration as product expression by the host cells increases. It is generally believed that trace levels of divalent metal ions which may be present in typical commonly available cell culture media may not be sufficient for stable trimer formation. Thus, addition of further quantities of divalent metal ions is preferred.

The divalent metal ions are preferably added to the culture media at a concentration which does not adversely or negatively affect host cell growth, if the divalent metal ions are being added during the growth phase of the host cells in the culture. In shake flask cultures, it was observed that ZnSO₄ added at concentrations of greater than 1 mM can result in lower host cell density. Those skilled in the art appreciate that bacterial cells can sequester metal ions effectively by forming metal ion complexes with cellular matrices. Thus, in the cell cultures, it is preferable to add the selected divalent metal ions to the culture media after the growth phase (after the desired host cell density is achieved) or just prior to product expression by the host cells. To ensure that sufficient amounts of divalent metal ions are present, additional divalent metal ions may be added or fed to the cell culture media during the product expression phase.

The divalent metal ion concentration in the culture media should not exceed the concentration which may be detrimental or toxic to the host cells. In the methods of the invention employing the host cell, *E. coli,* it is preferred that the concentration of the divalent metal ion concentration in the culture media does not exceed about 1mM (preferably, ≤ 1mM). Even more preferably, the divalent metal ion concentration in the culture media is about 50 micromolar to about 250 micromolar. Most preferably, the divalent metal ion used in such methods is zinc sulfate. It is desirable to add the divalent metal ions to the cell culture in an amount wherein the metal ions and Apo-2 ligand trimer can be present at a one to one molar ratio.

The divalent metal ions can be added to the cell culture in any acceptable form. For instance, a solution of the metal ion can be made using water, and the divalent metal ion solution can then be added or fed to the culture media.

In one embodiment of the invention, the selected Apo-2L variant is expressed in *E. coli,* and during the culturing or fermentation of the cell culture, the process parameters are set such that cellular activities are conducted at oxygen uptake rates of approximately 1.0 to 3.0 mmoles/L-min for cultures at approximately 40-50 gm/L dry cell weight. It is preferred that the newly synthesized nascent Apo-2L polypeptides have sufficient time for proper protein folding and trimerization of Apo-2L monomers. The growth phase of the fermentation process is preferably conducted at 30°C. Just prior to the commencement of product expression, the process temperature control set-point may remain at 30°C or be down-shifted to 25°C for the rest of the fermentation. Optionally, it may be desired to increase cell density in the cell culture, and the above-mentioned parameters may be adjusted (or increased) accordingly. For instance, it may be advantageous to increase cell density in the cell culture to increase volumetric yield. One skilled in the art can, by using routine techniques known in the art, incrementally increase the cell density and incrementally increase the above-mentioned parameters, if desired.

Expression of the Apo-2L may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or in situ hybridization, using an appropriately labeled probe, based on the sequences provided herein. Various labels may be employed, most commonly radioisotopes, and particularly ³²P. However, other techniques may also be employed, such as using biotin-modified nucleotides for introduction into a polynucleotide. The biotin then serves as the site for binding to avidin or antibodies, which may be labeled with a wide variety of labels, such as radionucleotides, fluorescers or enzymes. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like.

Antibodies useful for immunohistochemical staining and/or assay of sample fluids may be either monoclonal or polyclonal, and may be prepared in any mammal. Conveniently, the antibodies may be prepared against a native Apo-2 ligand polypeptide or against a synthetic peptide based on the DNA sequences provided herein or against exogenous sequence fused to Apo-2 ligand DNA and encoding a specific antibody epitope.

Apo-2 ligand preferably is recovered from the culture medium as a secreted polypeptide, although it also may be recovered from host cell lysates when directly produced without a secretory signal. If the Apo-2 ligand is membrane-bound, it can be released from the membrane using a suitable detergent solution (e.g. Triton-X 100) or its extracellular region may be released by enzymatic cleavage.

When Apo-2 ligand is produced in a recombinant cell other than one of human origin, the Apo-2 ligand is free of proteins or polypeptides of human origin. However, it is usually necessary to recover or purify Apo-2 ligand from recombinant cell proteins or polypeptides to obtain preparations that are substantially homogeneous as to Apo-2 ligand. As a first step, the culture medium or lysate may be centrifuged to remove particulate cell debris. Apo-2 ligand thereafter is purified from contaminant soluble proteins and polypeptides, with the following procedures being exemplary of suitable purification procedures: by fractionation on an ion-exchange column; ethanol precipitation; reverse phase HPLC; chromatography on silica or on a cation-exchange resin such as DEAE or CM; chromatofocusing; SDS-PAGE; ammonium sulfate precipitation; gel filtration using, for example, Sephadex G-75; diafiltration and protein A Sepharose columns to remove contaminants such as IgG.

In a preferred embodiment, the Apo-2 ligand can be isolated by affinity chromatography. Apo-2 ligand fragments or variants in which residues have been deleted, inserted, or substituted are recovered in the same fashion as native Apo-2 ligand, taking account of any substantial changes in properties occasioned by the variation. For example, preparation of an Apo-2 ligand fusion with another protein or polypeptide, e.g., a bacterial or viral antigen, facilitates purification; an immunoaffinity column containing antibody to the antigen can be used to adsorb the fusion polypeptide.

A protease inhibitor such as phenyl methyl sulfonyl fluoride (PMSF) also may be useful to inhibit proteolytic degradation during purification, and antibiotics may be included to prevent the growth of adventitious contaminants. One skilled in the art will appreciate that purification methods suitable for native Apo-2 ligand may require modification to account for changes in the character of Apo-2 ligand or its variants upon expression in recombinant cell culture.

During any such purification steps, it may be desirable to expose the recovered Apo-2L to a divalent metal ion-containing solution or to purification material (such as a chromatography medium or support) containing one or more divalent metal ions. In a preferred embodiment, the divalent metal ions and/or reducing agent is used during recovery or purification of the Apo-2L. Optionally, both divalent metal ions and reducing agent, such as DTT or BME, may be used during recovery or purification of the Apo-2L. It is believed that use of divalent metal ions during recovery or purification will provide for stability of Apo-2L trimer or preserve Apo-2L trimer formed during the cell culturing step.

A preferred method of recovering and purifying the expressed Apo-2L from prokaryotic host cells (most preferably from bacterial host cells) comprises the following steps: (a) extracting Apo-2L (intracellular) from *E. coli* cells; (b) stabilizing the properly folded Apo-2L in a buffer solution comprising divalent metal ions and/or reducing agent; (c) purifying the Apo-2L by chromatography using, sequentially, a cationic exchanger, a hydroxyapatite and a hydrophobic interaction chromatograph, and (d) selectively eluting Apo-2L in a buffer solution comprising divalent metal ions and/or reducing agent from each such chromatographic support. The divalent metal ions and the reducing agent utilized in such methods may include a Zn sulfate, Zn chloride, Co sulfate, DTT and BME, and more preferably, a Zn sulfate or DTT.

The description below also relates to methods of producing Apo-2 ligand variants covalently attached (hereinafter "conjugated") to one or more chemical groups. Chemical groups suitable for use in an Apo-2L conjugate of the present invention are preferably not significantly toxic or immunogenic. The chemical group is optionally selected to produce an Apo-2L variant conjugate that can be stored and used under conditions suitable for storage. A variety of exemplary chemical groups that can be conjugated to polypeptides are known in the art and include for example carbohydrates, such as those carbohydrates that occur naturally on glycoproteins, and non-proteinaceous polymers, such as polyols (see, e.g., U.S. Patent No. 6,245,901).

A polyol, for example, can be conjugated to polypeptides such as an Apo-2L variant at one or more amino acid residues, including lysine residues, as is disclosed in WO 93/00109, supra.The polyol employed can be any water-soluble poly(alkylene oxide) polymer and can have a linear or branched chain. Suitable polyols include those substituted at one or more hydroxyl positions with a chemical group, such as an alkyl group having between one and four carbons. Typically, the polyol is a poly(alkylene glycol), such as poly(ethylene glycol) (PEG), and thus, for ease of description, the remainder of the discussion relates to an exemplary embodiment wherein the polyol employed is PEG and the process of conjugating the polyol to a polypeptide is termed "pegylation." If However, those skilled in the art recognize that other polyols, such as, for example, poly(propylene glycol) and polyethylene-polypropylene glycol copolymers, can be employed using the techniques for conjugation described herein for PEG.

The average molecular weight of the PEG employed in the pegylation of the Apo-2L variant can vary, and typically may range from about 500 to about 30,000 daltons (D). Preferably, the average molecular weight of the PEG is from about 1,000 to about 25,000 D, and more preferably from about 2,000 to about 5,000 D. In one embodiment, pegylation is carried out with PEG having an average molecular weight of about 2,000 D. Preferably, the PEG homopolymer is unsubstituted, but it may also be substituted at one end with an alkyl group. Preferably, the alkyl group is a C1-C4 alkyl group, and most preferably a methyl group. PEG preparations are commercially available, and typically, those PEG preparations suitable for use in the present invention are nonhomogeneous preparations sold according to average molecular weight. For example, commercially available PEG(5000) preparations typically contain molecules that vary slightly in molecular weight, usually ± 500 D.

The Apo-2 ligand variants of the invention may be in monomer form or trimer form (comprising three monomers). Optionally, an Apo-2L variant trimer will be pegylated in a manner such that a PEG molecule is linked or conjugated to each of the three monomers that make up the trimeric Apo-2L variant. In such an embodiment, it is preferred that the PEG employed have an average molecular weight of about 2,000 to about 5,000 D. It is also contemplated that the Apo-2L variant trimers may be "partially" pegylated, i.e., wherein only one or two of the three monomers that make up the trimer are linked or conjugated to PEG. In such a "partially" pegylated Apo-2L variant, it is preferred that the PEG employed have an average molecular weight of about 5,000 D or greater than 5,000 D.

A variety of methods for pegylating proteins are known in the art. Specific methods of producing proteins conjugated to PEG include the methods described in U.S. Pat. No. 4,179,337, U.S. Pat. No. 4,935,465 and U.S. Patent No. 5,849,535. Typically the protein is covalently bonded via one or more of the amino acid residues of the protein to a terminal reactive group on the polymer, depending mainly on the reaction conditions, the molecular weight of the polymer, etc. The polymer with the reactive group(s) is designated herein as activated polymer. The reactive group selectively reacts with free amino or other reactive groups on the protein. The PEG polymer can be coupled to the amino or other reactive group on the protein in either a random or a site specific manner. It will be understood, however, that the type and amount of the reactive group chosen, as well as the type of polymer employed, to obtain optimum results, will depend on the particular protein or protein variant employed to avoid having the reactive group react with too many particularly active groups on the protein. As this may not be possible to avoid completely, it is recommended that generally from about 0.1 to 1000 moles, preferably 2 to 200 moles, of activated polymer per mole of protein, depending on protein concentration, are employed. The final amount of activated polymer per mole of protein is a balance to maintain optimum activity, while at the same time optimizing, if possible, the circulatory half-life of the protein.

While the residues may be any reactive amino acids on the protein, such as the N-terminal amino acid group, preferably the reactive amino acid is cysteine, which is linked to the reactive group of the activated polymer through its free thiol group as shown, for example, in W0 99/03887, WO 94/12219, WO 94/22466, U.S. Patent No. 5,206,344, U.S. Patent No. 5,166,322, and U.S. Patent No. 5,206,344. Alternatively the reactive group is lysine, which is linked to the reactive group of the activated polymer through its free epsilon-amino group, or glutamic or aspartic acid, which is linked to the polymer through an amide bond. This reactive group can then react with, for example, the α, and ε amines of proteins to form a covalent bond. Conveniently, the other end of the PEG molecule can be "blocked" with a non-reactive chemical group, such as a methoxy group, to reduce the formation of PEG-crosslinked complexes of protein molecules.

Suitable activated PEGs can be produced by a number of conventional reactions. For example, a N-hydroxysuccinimide ester of a PEG (M-NHS-PEG) can be prepared from PEG-monomethyl ether (which is commercially available from Union Carbide) by reaction with N,N'-dicyclohexylcarbodiimide (DCC) and N-hydroxysuccinimide (NHS), according to the method of Buckmann and Merr, Makromol.Chem.,182:1379-1384 (1981). In addition, a PEG terminal hydroxy group can be converted to an amino group, for example, by reaction with thionyl bromide to form PEG-Br, followed by aminolysis with excess ammonia to form PEG-NH₂. The PEG-NH₂ is then conjugated to the protein of interest using standard coupling reagents, such as Woodward's Reagent K. Furthermore, a PEG terminal -CH₂ OH group can be converted to an aldehyde group, for example, by oxidation with MnO₂. The aldehyde group is conjugated to the protein by reductive alkylation with a reagent such as cyanoborohydride. Alternatively, activated PEGs suitable for use in the present invention can be purchased from a number of vendors. For example, Shearwater Polymers, Inc. (Huntsville, Ala.) sells methoxy-PEG-maleimide, MW 2,000, in addition to a succinimidyl carbonate of methoxy-PEG and methoxy-PEG succinimidyl propionate.

The degree of pegylation of Apo-2L variant of the present invention can be adjusted to provide a desirably increased *in vivo* half-life (hereinafter "half-life"), compared to the corresponding non-pegylated Apo-2L variant. It is believed that the half-life of a pegylated Apo-2L variant typically increases incrementally with increasing degree of pegylation. The degree and sites of pegylation of a protein are determined, e.g., by (1) the number and reactivities of pegylation sites (e.g., primary amines) and (2) pegylation reaction conditions. As some of the pegylation sites in a protein are likely to be relatively unreactive, standard pegylation reactions typically result in less than complete pegylation.

Standard mutagenesis techniques can be used to alter the number of potential pegylation sites in a protein. Thus, to the extent that amino acid substitutions introduce or replace amino acids such as cysteine and lysine, Apo-2L variants of the present invention can contain a greater or lesser number of potential pegylation sites than native sequence Apo-2L (shown in Figure 1). The degree and sites of pegylation can also be manipulated by adjusting reaction conditions, such as the relative concentrations of the activated PEG and the protein as well as the pH. Suitable conditions for a desired degree of pegylation can be determined empirically by varying the parameters of standard pegylation reactions.

Pegylation of Apo-2L variants, such as R170C, is carried out by any convenient method. In an exemplary embodiment, the Cys170 side chain of R170C-Apo2L.0 (i.e., a variant Apo-2L having amino acids 114-281 of Figure 1 and a cysteine residue substituted for the native arginine residue at position 170; such a molecule may alternatively be identified herein as "R170C.0") is covalently modified by reaction with methoxy-PEG-maleimide, MW 2,000 D (Shearwater Polymers). Briefly, R170C-Apo2L.0 is prepared for modification by first reducing with 10 mM DTT at ambient temperature for about 30 minutes followed by passage over a PD-10 gel filtration column, equilibrated and eluted with HIC buffer (0.45 M Na2S04, 25 mM Tris-HCI pH 7.5), to remove the reducing agent. An aliquot of a PEG-maleimide solution (10 MM in dH20) is then added immediately. Conditions of time and reagent concentration necessary to ensure complete reaction can be determined empirically. Molar concentration ratios of PEG-maleimide to R170C-Apo2L.0 monomer ranging from 0.5 to 5-fold and reaction times of 2 or 24 hours can be used. The reactions are terminated by addition of a 10-fold molar excess of iodoacetamide, relative to the R170C-Apo2L.0 monomer concentration, such that any unpegylated Cys170 thiol becomes carboxyamidomethylated. Modification with iodoacetamide is for about 30 minutes and then the excess reagents are removed by gel filtration on a NAP-5 column (Pharmacia) equilibrated and eluted with PBS.

The pegylated proteins can be characterized by SDS-PAGE, gel filtration, NMR, peptide mapping, liquid chromatography-mass spectrophotometry, and in vitro biological assays. The extent of pegylation is typically first shown by SDS-PAGE. Polyacrylamide gel electrophoresis in 10% SDS is typically run in 10 mM Tris-HC1 pH 8.0, 100 mM NaCl as elution buffer. To demonstrate which residue is pegylated, peptide mapping using proteases such as trypsin and' Lys-C protease can be performed. Thus, samples of pegylated and non-pegylated R170C-Apo2L.0 can be digested with a protease such as Lys-C protease and the resulting peptides separated by a technique such as reverse phase HPLC. The chromatographic pattern of peptides produced can be compared to a peptide map previously determined for the Apo-2L.0 polypeptide. Each peak can then be analyzed by mass spectrometry to verify the size of the fragment in the peak. The fragment(s) that carried PEG groups are usually not retained on the HPLC column after injection and disappear from the chromatograph. Such disappearance from the chromatograph is an indication of pegylation on that particular fragment that should contain at least one pegylatable amino acid residue. Pegylated Apo-2L variants may further be assayed for ability to interact with an Apo-2L receptor and/or induce apoptosis in mammalian cells and/or other biological activities using known methods in the art.

Formulations comprising such Apo-2 ligand variant polypeptides are also provided by the present invention. It is believed that such formulations will be particularly suitable for storage (and maintain Apo-2L trimerization), as well as for therapeutic administration. Optional formulations will comprise Apo-2L variants and zinc or cobalt. More preferably, the formulation will comprise an Apo-2L variant and zinc or cobalt solution in which the metal is at a <2X molar ratio to the protein. If an aqueous suspension is desired, the divalent metal ion in the formulation may be at a >2X molar ratio to the protein. Those skilled in the art will appreciate that at a >2X molar ratio, there may be an upper range of concentration of the divalent metal ion in the formulation at which the metal can become deleterious to the formulation or would be undesirable as a therapeutic formulation.

The formulations may be prepared by known techniques. For instance, the Apo-2L variant formulation may be prepared by buffer exchange on a gel filtration column.

Typically, an appropriate amount of a pharmaceutically-acceptable salt is used in the formulation to render the formulation isotonic. Examples of pharmaceutically-acceptable carriers include saline, Ringer's solution and dextrose solution. The pH of the formulation is preferably from about 6 to about 9, and more preferably from about 7 to about 7.5. Preferably, the pH is selected so as to ensure that the zinc remains bound to the Apo-2L. If the pH is too high or too low, the zinc does not remain bound to the Apo-2L variant and as a result, dimers of Apo-2L variant will tend to form. It will be apparent to those persons skilled in the art that certain carriers may be more preferable depending upon, for instance, the route of administration and concentrations of Apo-2 ligand variant and divalent metal ions.

Therapeutic compositions of the Apo-2L variant can be prepared by mixing the desired Apo-2L variant molecule having the appropriate degree of purity with optional pharmaceutically acceptable carriers, excipients, or stabilizers (Remington's PharmaceuticalSciences,16th edition, Osol, A. ed. (1980)), in the form of lyophilized formulations, aqueous solutions or aqueous suspensions. Acceptable carriers, excipients, or stabilizers are preferably nontoxic to recipients at the dosages and concentrations employed, and include buffers such as Tris, HEPES, PIPES, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; sugars such as sucrose, mannitol, trehalose or sorbitol; salt-forming counter-ions such as sodium; and/or non-ionic surfactants such as TWEEN™, PLURONICS™ or polyethylene glycol (PEG).

Additional examples of such carriers include ion exchangers, alumina, aluminum stearate, lecithin, serum proteins, such as human serum albumin, buffer substances such as glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, colloidal silica, magnesium trisilicate, polyvinyl pyrrolidone, and cellulose-based substances. Carriers for topical or gel-based forms include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations.

Effective dosages of Apo-2 ligand variant in the formulations may be determined empirically, and making such determinations is within the skill in the art. It is presently believed that an effective dosage or amount of Apo-2 ligand variant may range from about 1 microgram/kg to about 100 mg/kg of body weight or more per day. Interspecies scaling of dosages can be performed in a manner known in the art, e.g., as disclosed in Mordenti et al., Pharmaceut. Res., 8:1351 (1991). Those skilled in the art will understand that the dosage of Apo-2 ligand variant that must be administered will vary depending on, for example, the mammal which will receive the Apo-2 ligand variant, the route of administration, and other drugs or therapies being administered to the mammal.

Apo-2L variants to be used for in vivo administration should be sterile. This is readily accomplished by filtration through sterile filtration membranes, prior to or following lyophilization and reconstitution. Apo-2L variant ordinarily will be stored in lyophilized form or in solution if administered systemically. If in lyophilized form, Apo-2L variant is typically formulated in combination with other ingredients for reconstitution with an appropriate diluent at the time for use. An example of a liquid formulation of Apo-2L variant is a sterile, clear, colorless unpreserved solution filled in a single-dose vial for subcutaneous injection.

Therapeutic Apo-2L variant formulations generally are placed into a container having a sterile access port, for example, an intravenous solution bag or vial having a stopper pierceable by a hypodermic injection needle. The formulations are preferably administered as repeated intravenous (i.v.), subcutaneous (s.c.), intramuscular (i.m.) injections or infusions, or as aerosol formulations suitable for intranasal or intrapulmonary delivery (for intrapulmonary delivery see, e.g., EP 257, 956) .

Apo-2L variants can also be administered in the form of sustained-release preparations. Suitable examples of sustained-release preparations include semipermeable matrices of solid hydrophobic polymers containing the protein, which matrices are in the form of shaped articles, e.g., films, or microcapsules. Examples of sustained-release matrices include polyesters, hydrogels (e.g., poly(2-hydroxyethyl-methacrylate) as described by Langer et al., J. Biomed. Mater. Res., 15: 167-277 (1981) and Langer, Chem. Tech., 12: 98-105 (1982) or poly(vinylalcohol)), polylactides (U.S. Patent No. 3,773,919, EP 58,481), copolymers of L-glutamic acid and gamma ethyl-L-glutamate (Sidman et al., Biopolymers, 22: 547-556 (1983)), non-degradable ethylene-vinyl acetate (Langer et *al., supra),* degradable lactic acid-glycolic acid copolymers such as the Lupron Depot (injectable microspheres composed of lactic acid-glycolic acid copolymer and leuprolide acetate), and poly-D-(-)-3-hydroxybutyric acid (EP 133,988).

The Apo-2L variants and its formulations described herein can be employed in a variety of therapeutic and non-therapeutic applications. Among these applications are methods of treating various cancers (provided above), immune related conditions, and viral conditions. Such therapeutic and non-therapeutic applications are described, for instance, in W097/25428, W097/01633, WO 01/00832, and WO 01/22987.

The Apo2L variants described herein are useful in treating various pathological conditions, such as immune related diseases or cancer. Diagnosis in mammals of the various pathological conditions described herein can be made by the skilled practitioner. Diagnostic techniques are available in the art which allow, e.g., for the diagnosis or detection of cancer or immune related disease in a mammal. For instance, cancers may be identified through techniques, including but not limited to, palpation, blood analysis, x-ray, NMR and the like. Immune related diseases can also be readily identified. In systemic lupus erythematosus, the central mediator of disease is the production of auto-reactive antibodies to self proteins/tissues and the subsequent generation of immune-mediated inflammation. Multiple organs and systems are affected clinically including kidney, lung, musculoskeletal system, mucocutaneous, eye, central nervous system, cardiovascular system, gastrointestinal tract, bone marrow and blood. Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, interstitial pneumonitis with pulmonary fibrosis, keratoconjunctivitis sicca, and rheumatoid nodules.

The Apo2L variants can be administered in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

Effective dosages and schedules for administering Apo2L variants may be determined empirically, and making such determinations is within the skill in the art. Single or multiple dosages may be employed. It is presently believed that an effective dosage or amount of Apo2L variants used alone may range from about 1 µg/kg to about 100 mg/kg of body weight or more per day. Interspecies scaling of dosages can be performed in a manner known in the art, e_{.}g., as disclosed in Mordenti et al., Pharmaceut. Res., 8:1351 (1991).

When *in vivo* administration of an Apo2L variant is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue. Those skilled in the art will understand that the dosage of Apo2L variant that must be administered will vary depending on, for example, the mammal which will receive the Apo2L variant, the route of administration, and other drugs or therapies being administered to the mammal.

It is contemplated that yet additional therapies may be employed in the methods. The one or more other therapies may include but are not limited to, administration of radiation therapy, cytokine(s), growth inhibitory agent(s), chemotherapeutic agent(s), cytotoxic agent(s), tyrosine kinase inhibitors, ras farnesyl transferase inhibitors, angiogenesis inhibitors, and cyclin-dependent kinase inhibitors which are known in the art and defined further with particularity in Section I above. In addition, therapies based on therapeutic antibodies that target tumor antigens such as Rituxan™ or Herceptin™ as well as anti-angiogenic antibodies such as anti-VEGF, or antibodies that target Apo2L receptors, such as DR5 or DR4.

Preparation and dosing schedules for chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the Apo2L variant, or may be given simultaneously therewith.

It may be desirable to also administer antibodies against other antigens, such as antibodies which bind to CD20, CDlla, CD18, CD40, ErbB2, EGFR, ErbB3, ErbB4, vascular endothelial factor (VEGF), or other TNFR family members (such as DR4, DR5, OPG, TNFR1, TNFR2). Alternatively, or in addition, two or more antibodies binding the same or two or more different antigens disclosed herein may be co-administered to the patient. Sometimes, it may be beneficial to also administer one or more cytokines to the patient. In one embodiment, the Apo2L variants herein are co-administered with a growth inhibitory agent. For example, the growth inhibitory agent may be administered first, followed'by an Apo2L variant of the present invention.

The Apo2L variant (and one or more other therapies) may be administered concurrently or sequentially. Following administration of Apo2L variant, treated cells in vitro can be analyzed. Where there has been *in vivo* treatment, a treated mammal can be monitored in various ways well known to the skilled practitioner. For instance, tumor cells can be examined pathologically to assay for necrosis or serum can be analyzed for immune system responses.

An article of manufacture such as a kit containing Apo-2L variants useful for the diagnosis or treatment of the disorders described herein comprises at least a container and a label. Suitable containers include, for example, bottles, vials, syringes, and test tubes. The containers may be formed from a variety of materials such as glass or plastic. The container holds an Apo-2L variant formulation that is effective for diagnosing or treating the condition and may have a sterile access port (for example, the container may be an intravenous solution bag or a vial having a stopper pierceable by a hypodermic injection needle). The label on, or associated with, the container indicates that the formulation is used for diagnosing or treating the condition of choice. The article of manufacture may further comprise a second container comprising a pharmaceutically-acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use. The article of manufacture may also comprise a second or third container with another active agent as described above.

The following examples are offered for illustrative purposes only, and are not intended to limit the scope of the present invention in any way. All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### EXAMPLE 1

### Crystallography Analysis of Apo-2L

Crystals of Apo-2L (amino acid residues 114-281) were grown in 70uL sitting drops containing 40uL protein (at 2.6 mg/mL in 20 mM Tris, pH8.0), 20uL 50 mM Tris pH 8.0, and 10uL 8% peg 2K MME over a well solution of 50% peg 2K MME at 20° C and were members of the spacegroup P63 with two monomer in the asymmetric unit and unit cell constants a=72.5, c=140 Angstrom and diffract to 3.9 Angstrom at room temperature. Crystals of D218A variant grew in 14 uL sitting drops containing 4 uL of 4% MPD and 10 uL protein (1.7 mg/ml in 20 mM Tris pH 7.5) over a well solution of 32% MPD at 4° C and were members of the spacegroup R32 with one monomer per asymmetric unit and unit cell parameters 66.4, c=197.7 Angstrom and diffracted to 1.3 Angstrom at -180° C with synchroton radiation. Data sets diffracting to 3.9 Angstrom for the Apo-2L (residues 114-281) crystals and 1.9 Angstrom for the D218A variant were measured on a Rigaku rotating anode x-ray generator equipped with a MAR detector and processed with DENZO/SCALEPACK [Otwinowski et al., Proceedings of the CCP4 Study Weekend:Data Collection and Processing (eds. Sawyer et al.) pp.56-62 Daresbury Laboratory, Daresbury, England, 1993]. A 1.3 Angstrom data set for the D218A variant was measured at the Advanced Photon Source at Argonne National Labs and was processed with HKL2000/SCALEPACK and had a Rsym of 6.4% (34% in the 1.35-1.30 shell), with 100% completeness and a redundancy of 12-fold, and I/<I>=12.4.

The native Apo-2L structure was solved by molecular placement using a model based on TNF-alpha (pdb code 1TNF) with the program Amore [Acta Cyst., D50:760-763 (1994)] and was refined [Brunger, X-PLOR:version 3.1, Yale Press, New Haven 1987] with strict 2-fold non-crystallographic restraints until a R_{free} of 35%. This structure refined against the 1.9 Angstrom dataset until a R_{free} of 25% and finally was refined against 1.3 Angstrom data with Refmac and SHELXL [Sheldrick et al., Methods in Enzymology, pp. 319-343, Academic Press, San Diego 1997] of R_{free} = 22% and R_{factor} of 20% with good geometry (rmsd bonds 0.011 Angstrom, rmsd angle 1.7° ). All residues fall within the allowed regions of a Ramachandran plot. During refinement, a 28 sigma peak of electron density was observed between symmetry related Cys230 on the trimer axis. This density was modeled as a zinc ion and refined with B-factor of 10. It is believed that a chlorine molecule on the trimer axis is present as the fourth ligand to the zinc. The final model consists of residues 120-130, 142-194, 203-281 with 170 solvent molecules and one zinc ion and one chloride ion. Residues 91-119, 131-141, and 195-202 are disordered. N-terminal sequencing of several crystals confirmed that the N-terminus is intact while mass spectrometry of the starting material shows that it is full length.

A summary of the crystallographic data is provided in Figure 2C.

### EXAMPLE 2

### Design and production of Apo2L cysteine substitution variants

Sites for cysteine substitution were chosen on the basis of examination of the x-ray structure of the DR5-Apo2L complex (Figure 3). To avoid loss of activity upon mutation or subsequent modification of the introduced cysteine residue, only sites outside of the receptor contact region were considered for mutagenesis. In order to ensure efficient chemical modification of the cysteine side chain, only residues that displayed high solvent accessibility in the crystal structure were selected. Residues that matched these criteria include, but are not limited to, Vall14, Arg115, Glu116, Asn134, Asn140, Glu144, Asn152, Ser153, Arg170, Asp234, Glu249, Arg255, Glu263 and His264. As shown in Figure 3, this set of residues spans one face of the Apo2L monomer from top to bottom.

Cysteine substitution variants of Apo-2L were constructed by oligonucleotide-directed mutagenesis (Kunkel et al., Proc. Natl. Acad. Sci., 82:488-492 (1985); Kunkel, Methods in Enzymology, 154:367-382 (1987)) on the single-stranded form of the plasmid pAPOK5.0. This plasmid was designed for the intracellular *E. coli* expression of the 114-281 amino acid form of Apo2L driven by the tryptophan (trp) promoter. PAPOK5.0 0 was constructed from pAPOK5 (WO 99/36535 published July 22, 1999) by deletion mutagenesis of the DNA segment encoding residues 91-113 of Apo-2L (Figure 1). pAPOK5 was constructed by using PCR to clone the Apo-2L cDNA (encoding residues 91-281 of Figure 1) into plasmid pS1162 which carries the trp promoter. After mutagenesis, the identity of the plasmids was confirmed by dideoxynucleotide sequencing (Sanger) of the entire Apo2L portion of the plasmid.

Plasmids encoding the cysteine-substituted proteins were then transformed into *E. coli* strain 294 for expression. Cultures were grown overnight to saturation at 37°C in Luria broth plus carbenecillin at 50 µg/mL. The saturated cultures were subsequently seeded at a 50-fold dilution into sterile-filtered media comprised of Na₂HPO₄ (6 g/L), KH₂PO₄ (3 g/L), NaC1 (0.5 g/L), NH₄C1 (1 g/L), glucose (4.9 g/L), Casamino acids (4.9 g/L), 27 mM MgSO₄, 0.003% Thiamine HC1 and q.s. with distilled water plus carbenicillin at 40 µg/mL. The cultures were grown at 37°C until the A500 was 0.5 - 0.8 and then expression was induced by addition of 3-α-indoleacrylic acid (IAA) (Sigma, St. Louis, MO) to a final concentration of 25 µg/mL. Cells were grown overnight at 30°C with shaking, harvested by centrifugation and stored frozen at -20°C for subsequent recovery of Apo2L as described below.

The Apo-2L proteins were extracted from the frozen *E. coli* cell pellets by homogenization in 10 volumes (wt/vol) of 100mM Tris, pH8.0/200mM NaCl/5mM EDTA/1mM DTT using a model M110-F Microfluidizer (Microfluidics Corporation, Newton, MA). Polyethyeneimine (PEI) was added to a final concentration of 0.5% (vol/vol) to the homogenate which was then centrifuged to remove cell debris. Solid ammonium sulfate was added to the extraction supernatant to a final concentration of 45% saturation at ambient temperature with stirring, and the pellet was recovered by centrifugation. The ammonium sulfate pellet was washed with 50% ammonium sulfate solution to remove residual EDTA, then resuspended in 50 volumes (wt/vol) of 50mM HEPES, pH 7.5/0.1% Triton X-100. The resulting solution was clarified by centrifugation and purified by immobilized metal affinity chromatography (IMAC) using a 5 mL HiTrap Chelating Sepharose column (Pharmacia, Piscataway, NJ). The column was charged with nickel in 100mM NiS0_{4/}300mM Tris, pH7.5 and equilibrated with 350mM NaC1 in phosphate-buffered saline (PBS). After loading, the column was washed with 350mM NaC1 in PBS and eluted with 50mM Imidazole/350mM NaC1 in PBS. The IMAC eluent was dialyzed against 20mM Tris, pH7.5, clarified by centrifugation, and further purified by cation exchange chromatography using a 5mL HiTrap SP Sepharose column (Pharmacia), which was equilibrated and washed with 20mM Tris, pH7.5. The HiTrap SP column was eluted with 20mM Tris, pH7.5/0.5M NaC1. The SP column eluent was reduced with 2mM DTT and subsequently precipitated by adding solid ammonium sulfate with stirring to a final concentration of 45% saturation at ambient temperature. The ammonium sulfate pellet was resuspended in 3.5 mL of 20mM Tris, pH7.5/100mM NaC1 and exchanged into the final buffer of 20mM Tris, pH7.5/100mM NaCI/2mm DTT by gel filtration chromatography using a PD10 column (Pharmacia). The purified Apo-2L cysteine-substituted proteins were characterized by Coomassie-stained SDS-PAGE and mass spectroscopy, and stored frozen at - 20°C.

### EXAMPLE 3

### Apoptotic activity of Apo2L variants in vitro

A bioassay which measures cell viability from the metabolic conversion of a fluorescent dye was used to determine the apoptotic activity of Apo2L variants. Serial 2-fold dilutions of Apo-2L.0, Apo2L.2, or Apo2L variants were made in RPMI-1640 media (Gibco) containing 0.1% BSA, and 50 µL of each dilution was transferred to individual wells of 96-well Falcon tissue culture microplates. 50 µL of SK-MES-1 human lung carcinoma cells (ATCC HTB58) (in RMPI-1640, 0.1% BSA) were added at a density of 2 x 10⁴ cells/well. These mixtures were incubated at 37°C for 24 hours. At 20 hours, 25 µL of alamarBlue (AccuMed, Inc., Westlake, Ohio) was added. Cell number was determined by measuring the relative fluorescence at 590 nm upon excitation at 530 nm. These data were analyzed by using a 4 parameter fit to calculate ED₅₀, the concentration of Apo2L.0 giving a 50% reduction in cell viability.

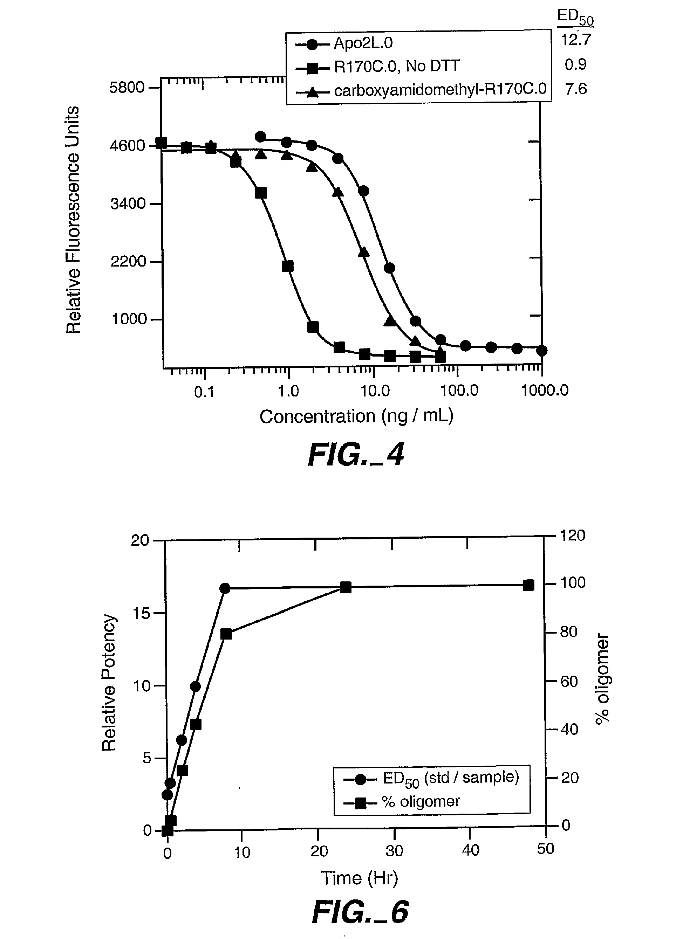

Of the cysteine-substituted Apo2L variants tested, E116C had a significant (>2-fold) reduction in apoptotic activity on SK-MES cells (Table I). The R170C variant had about a 10-fold increased potency. The increased activity of the R170C variant appears to be related to oxidation of Cys170 during incubation in the bioassay media. In this assay, the protein is diluted in the assay media with concomitant dilution of the reducing agent (2 mM DTT) included in the storage buffer. A decrease in the concentration of the reducing agent could allow disulfide bonds to form. Prior alkylation of Cys170 with N-ethylmaleimide (NEM) (Table I) or iodoacetamide (Figure 4) blocked the activity increase. In addition, Apo2L variants having Arg170 replaced with either Ala, Lys, or Ser residues had activities more comparable to the Apo-2L.0 polypeptide.

**Table I. Effect Of Apo2L Cysteine Substitutions On Apoptosis-Inducing Activity.**

| Variant | ED50 ratio |
|---|---|
| Apo2L.2 | 15.3 |
| S96C.2 | 21.1 |
| S101C.2 | 8.7 |
| S111C.2 | 2.1 |
| V114C | 1.4 |
| R115C | 1.2 |
| E116C | 3.5 |
| N134C | 0.7 |
| N140C | 0.7 |
| E144C | 1.5 |
| N152C | 1.0 |
| S153C | 1.3 |
| R170C | 0.1 |
| R170C-NEM | 1.1 |
| R170K | 1.0 |
| R170S | 0.4 |
| K179C | 1.2 |
| D234C | 1.5 |
| E249C | 1.6 |
| R255C | 1.9 |
| E263C | 0.6 |
| H264C | 2.1 |
| S96C.2-PEG-2K | 51 |
| S101C.2-PEG-2K | 14.4 |
| S111C.2-PEG-2K | 5.1 |
| V114C-PEG-2K | 2.1 |
| R115C- PEG-2K | 14.3 |
| E116C- PEG-2K | ND |
| N134C- PEG-2K | 134 |
| N140C- PEG-2K | 38 |
| E144C- PEG-2K | 7 |
| N152C- PEG-2K | 17.1 |
| S153C- PEG-2K | 65 |
| R170C- PEG-2K | 5.2 |
| K179C-PEG-2K | 1.9 |
| D234C- PEG-2K | 43 |
| E249C- PEG-2K | 13.2 |
| R255C- PEG-2K | ND |
| E263- PEG-2K | 23 |
| H264C- PEG-2K | 54 |

| | |
|---|---|
| "ND" = Not Determined; All of the Apo-2 ligand variants were produced as the 114-281 form of the protein (Apo2L.0) except for variants S96C.2, S101C.2, and S111C.2 which were produced from the 91-281 form of the protein ("Apo2L.2"). | |

The potential for formation of disulfide bonds between R170C-Apo-2L.0 trimers was further examined by measuring the kinetics of air oxidation. A portion of a 1.4 mg/mL solution of R170C-Apo-2L.0, stored in the presence of 2 mM DTT, was passed over a PD-10 column equilibrated with HIC buffer (0.45 M Na₂SO₄, 25 mM Tris-HC1 pH 7.5) in order to remove the DTT. This solution (3.5 mL of 1.1 mg/mL R170C-Apo2L.0) was incubated at ambient temperature in a 15 mL Falcon tube with gentle agitation. Aliquots were removed at varied times and any solvent accessible thiols remaining on R170C-Apo-2L.0 were alkylated with a 10-fold molar excess of iodoacetamide. The first time point was at 3 minutes because this is the amount of time required to elute the protein from the PD-10 column. These samples were assayed for bioactivity on SK-MES cells as described above and were also characterized for molecular weight by size exclusion chromatography with multi-angle light scattering detection (SEC-MALS). Chromatography was performed by using a Superdex 200 column (1.6 x 30 cm), equilibrated and eluted with PBS, operated on a FPLC system equipped with both a UV detector and a light scattering detector (Wyatt Technology, Inc.).

As shown in Figure 5, with only 3 minutes of air oxidation R170C-Apo-2L.0 is found predominantly in the trimeric form with a calculated molecular weight of 70,000 D. At 2 hours, significant amounts of higher molecular weight forms are found. The three peaks at 2 hours have calculated molecular weights of 70,000, 140,000 and 600,000 D. After 24 hours of air oxidation most of the R170C-Apo2L.0 is found as the 600,000 D molecular weight species. A further 24 hour incubation does not result in production of species greater than 600,000 D. Upon SDS-PAGE, the higher molecular weight forms migrate as disulfide-linked dimers. These results suggest that the R170C-Apo2L.0 protein forms oligomeric species in which trimers are linked together via disulfide bonds. The 140,000 D form corresponds to 2 trimers joined together whereas the 600,000 D form has 8-10 trimers covalently linked. Upon denaturation in SDS, these oligomers are resolved into disulfide-linked dimers. After 24 hours of air oxidation, R170C-Apo2L.0 gave a nearly 20-fold decreased ED50 on SK-MES cells in the apoptosis bioassay. The time course of the increase in bioactivity is concomitant with the accumulation of oligomeric forms (Figure 6). Oligomerization through Cys170 disulfide bonds also results in increased affinity for the DR5 receptor.

These results suggest that oligomerization of Apo2L in a fashion that does not preclude receptor binding yields a molecule which produces a more potent death signal on tumor cells. However, oligomerization through Cys170 may render the molecule toxic to some normal cells. In certain in vitro testing on human or cynomologous monkey hepatocytes, oxidized R170CApo2L.0 was more toxic than Apo2L.0 (Figure 7).

### EXAMPLE 4

### Pegylation of Apo-2L on Cys residues

Cysteine-substituted Apo2L proteins were covalently modified by reaction with methoxy-PEG-maleimide, MW 2,000 D (Shearwater Polymers). The Apo2L variants were prepared for modification by first removing the DTT contained in the storage buffer by passage over a PD-10 gel filtration column. The column was equilibrated and eluted with HIC buffer (0.45 M Na₂S0₄, 25 mM Tris-HCI pH 7.5), or arginine formulation buffer (0.5 M Arg-succinate, 20 mM Tris-HC1 pH 7.5). An aliquot of a PEG-maleimide solution (10 mM in dH₂0) was added immediately. Initial experiments used the R170C variant to determine the reaction time and reagent concentration necessary to ensure complete reaction. Molar concentration ratios of PEG-maleimide to R170C-Apo2L.0 monomer of 1:1, 2:1, 5:1 or 10:1 and reaction times of 2 or 24 hours were used. The reactions were terminated by addition of DTT to 2mM, followed by a 30 minute incubation at ambient temperature, and then iodoactemide was added to 10mM. This quenching procedure ensured that any disulfide bonds formed during the reaction procedure were reduced and any unpegylated Cys170 thiol became carboxyamidomethylated. Modification with iodoacetamide was for 30 minutes and then the excess reagents were removed by gel filtration on a NAP-5 column (Pharmacia) equilibrated and eluted with PBS. These samples were analyzed by SDS-PAGE and SEC-MALS. Apoptosis-inducing activity on SK-MES cells was also assayed as described above. As shown in Figure 8, SDS-PAGE indicates an approximately 2000 Dalton shift in the monomer molecular weight upon treatment of R170CApo2L.0 with PEG-2K-maleimide. Reactions using PEG:protein ratios of 2:1 or greater gave a similar extent of modification. For these reactions, residual unmodified monomer was observed. Visual inspection of the Coomassie blue-stained gel suggests that unmodified monomer accounts for <10% of the total protein. At PEG:protein molar ratios less than 2:1, less modification was obtained. The reactions appeared to go to completion within 2 hours since no apparent change in the product was observed with a 24 hour reaction time.

The hydrodynamic properties of PEGylated R170C-Apo2L.0 were evaluated by SEC-MALS as described above except that the running buffer used for the Superdex 200 column was 0.4 M ammonium sulfate, 15 mM sodium phosphate pH 6.5. Use of this higher ionic strength buffer reduces interaction of Apo2L with the column material. R170C-Apo2L.0 having Cys170 blocked with iodoacetamide eluted as a single, symmetrical peak centered at 10.65 mL with a molar mass calculated from light scattering as 60,000 g/mol. PEG-2K-R170C-Apo2L.0, prepared using a 2:1 PEG ratio and 2 hour reaction time, also eluted as a single, symmetrical peak, but with an elution volume of 9.25 mL and a calculated molar mass of 69,000 g/mol. Taken together with the results from SDS-PAGE these data suggest that this sample has 3 covalently attached PEG chains per trimer with 1 PEG chain per monomer. The calculated molar mass of PEG-2K-R170C-Apo2L.0 (69,000 D) agrees with the expected mass (66,000 D) given the standard error (±10%) in the measurement. This trimeric form of APO-2L having 3 covalently attached PEG chains per trimer with 1 PEG chain per monomer yields a complex represents a preferred embodiment of the invention by exhibiting a number of coexisting optimal characteristics including a significant bioactivity profile and a MWₐₚₚ that is greater than the kidney filtration cutoff. Apparent molecular weights of 50,000 D for IAM-RI70C-Apo2L.0 and 100,000 D for PEG-2K-R170C-Apo2L.0 were calculated on the basis of the relative elution volume. A series of proteins of known molecular weight were used to construct a calibration curve relating elution volume to apparent molecular weight. IAM-R170C-Apo2L.0, and also unmodified Apo2L.0, gave molecular weights somewhat smaller than expected but consistent with the compact shape of the trimer. The larger apparent molecular weight calculated for PEG-2K-R170C-Apo2L.0 results from the hydrophilic and extended PEG chain causing a large increase in the hydrodynamic radius of the modified protein.

On the basis of the above results, initial PEGylation experiments with the other cysteine-substituted variants used a 2:1 molar ratio of PEG to monomer and a 2 hour reaction time. These reaction conditions usually produced trimers having 3 attached PEG chains. However, the V114C and R115C variants were found in the experiment to be poorly reactive and required higher PEG ratios and longer reaction times to get a more complete reaction. The R255C variant could not be modified even with higher PEG ratios and longer reaction times. Modification experiments were not performed on the E116C variant.

Many of the cysteine-substituted proteins displayed decreased bioactivity (Table I) when PEGylated as described above. The decrease in bioactivity ranged from 2.1-fold for PEG-V114C to 134-fold for PEG-N134C. R170C-Apo2L.0 retained a relatively high level of activity upon PEGylation, and because the modification proceeded rapidly to completion at low PEG:protein ratios, this variant was chosen for further study.

For production of larger amounts of PEG-R170C-Apo2L.0, a 2:1 molar ratio of PEG:Apo2L monomer and a 24 hour reaction time was used. 70 mg of R170C-Apo2L.0 was gel filtered and then reacted with PEG-maleimide at ambient temperature for 24 hours. The reaction was quenched with a 10-fold molar excess of iodoacetamide and then protein was separated from free PEG by gel filtration chromatography on a column of Sephadex G-25 equilibrated with formulation buffer (Arg-succinate). This preparation had lot number 32176-87C. Purified PEG-R170C-Apo2L.0 (32176-87C) displayed binding affinities for DR4, DR5, DcR1, DcR2, and OPG equivalent to that measured for Apo2L.0 (Table II).

**Table II. Receptor binding affinities measured for PEG-R170C-Apo2L.0 by ELISA**

| Sample | DcR1 | DcR2 | OPG | DR4 | DR5 |
|---|---|---|---|---|---|
| Apo2L.0 | 10.8 | 6.0 | 85.2 | 94.3 | 42.3 |
| PEG-R170C-Apo2L.0(32 176-87C) | 12.4 | 4.8 | 55.7 | 42.2 | 43.8 |
| PEG-R170C-Apo2L.0(32 176-78) | 2.7 | 0.8 | 6.7 | 4.5 | 9.0 |

PEG-R170C-Apo2L.0 (32176-87C) was then analyzed by mass spectroscopy and peptide mapping. MALDI-TOF-MS (Figure 10) indicated the presence of a small amount of unmodified monomer (MW=19,440) and a major peak corresponding to protein having a single attached PEG. PEG molecules are well known to have mass heterogeneity, differing in molecular weight by increments of the polymer unit ethylene glycol (MW=44). As a consequence, a broad mass range centered about 21,680 is observed for the protein with a single PEG attached. The difference in average mass between the pegylated and non-pegylated R170C-Apo2L.0 indicates that the average mass of the PEG is 2200 D.

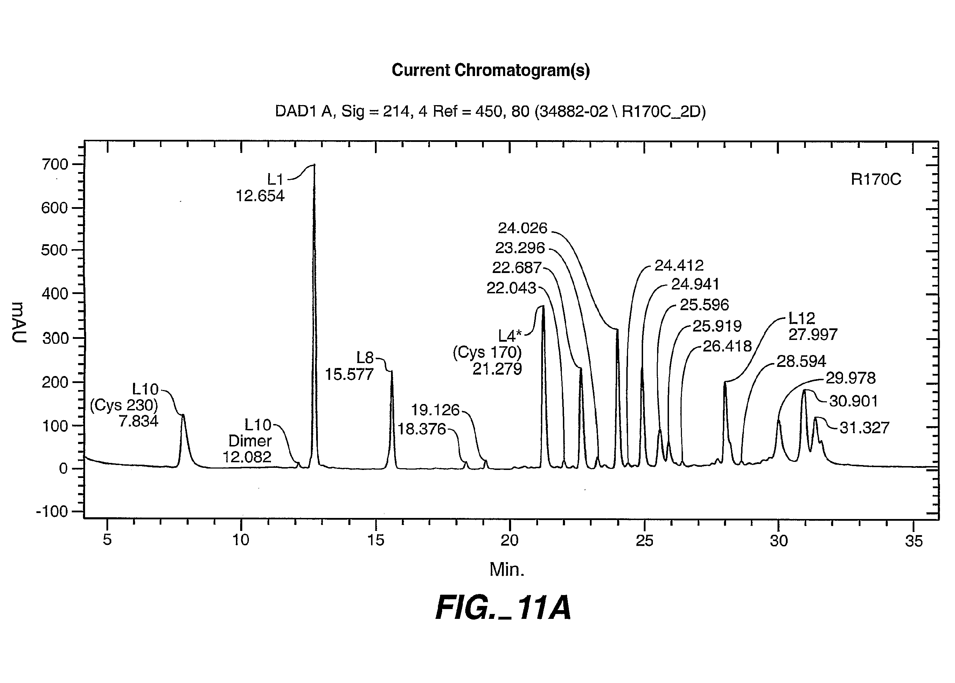

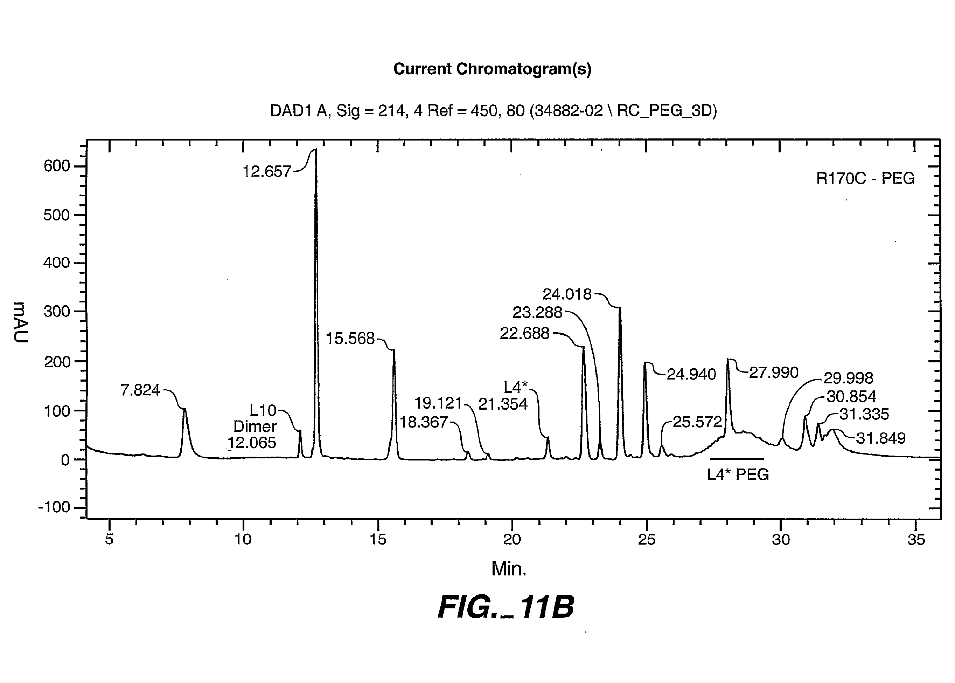

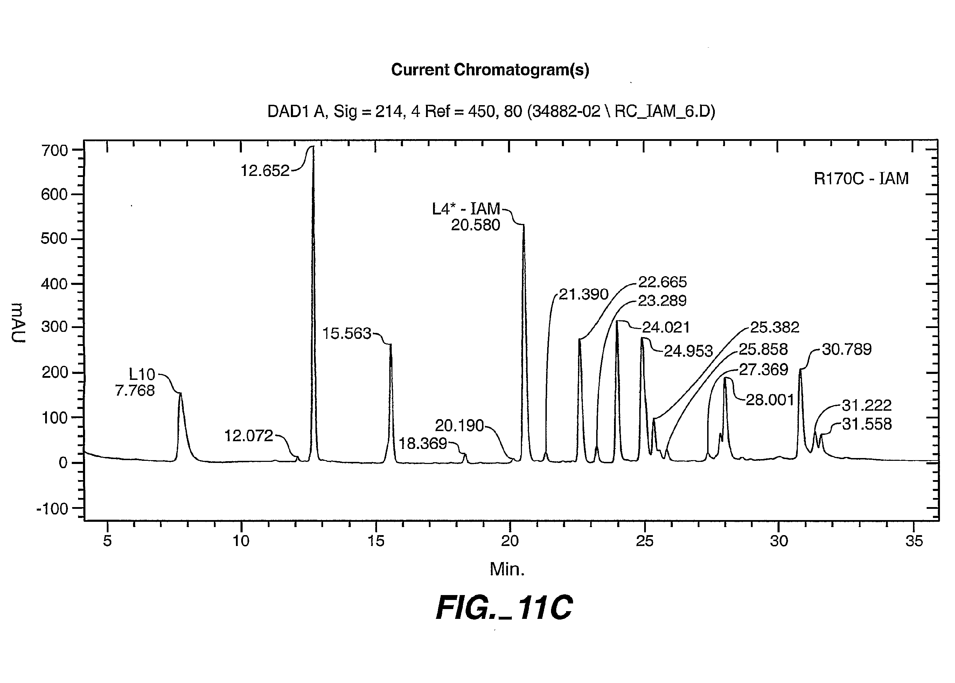

The site of PEG attachment was confirmed by peptide mapping. Samples of pegylated and non-pegylated R170C-Apo2L.0 were digested with Lys-C protease and the resulting peptides were separated by reverse phase HPLC (Figure 11). The pattern of peptides produced was compared to the map previously determined for Apo2L.0. A peptide labeled L4, produced by cleavage after Lys150 and Lys179, contains the Cys170 residue in the digest of R170C-Apo2L.0. This peak disappears and is replaced by a broad, later eluting peak (L4^{*}), in the pegylated protein. A MALDI-TOF mass spectrum of this fraction shows a series of peaks separated by 44 Da with a distribution of 1.9-2.6 kDa higher than the predicted peptide mass. Further analysis by in-source fragmentation in MALDI-TOF confirmed L4^{*} as the 151-179 peptide modified on Cys170 with PEG. In contrast to these results, the L10 peptide (225-233) shows a similar peak area in both unmodified and pegylated R170C-Apo2L.0. This indicates that the native Cys230 residue, which is buried and participates in chelation of the zinc ion, is not modified by PEG-maleimide. Significant modification of other functional groups, such as the side chains of Lys residues, was not observed. Taken together with the SDS-PAGE and MALDI-TOF mass spectrum of one or more of the following amino acid substitutions at the residue position(s) in Figure 1 (SEQ ID NO:1): S96C; S101C; S111C; V114C; R115C; E116C; N134C; N140C; E144C; N152C; S153C; R170C; R170K; R170S; K179C; D234C; E249C; R255C; E263C; H264C.

### EXAMPLE 5

### Pharmacokinetics of PEG-R170C-Apo2L.0

The effect of PEGylation on the clearance of Apo2L was tested in the mouse. Mice were given tail vein injections of Apo2L.0 (10 mg/kg) or PEG-R170C-Apo2L.0 (10 mg/kg) at time zero. Plasma samples were collected at 1, 20, 40, 60, and 80 minutes. Apo2L concentrations were determined by ELISA.

As shown in Figure 12, Apo2L.0 was rapidly cleared from the circulation whereas PEG-R170C-Apo2L.0(32176-87C) was cleared more slowly. At 60 minutes after injection, the plasma concentration of Apo2L.0 was less than 1% of the concentration at 1 minute. In contrast, the plasma concentration of PEG-R170C-Apo2L.0(32176-87C) only decreased by 50% in this time period. Site-specific attachment of PEG-2000 to Apo2L thus resulted in a significant decrease in the rate of clearance.

### EXAMPLE 6

### Effect of PEG-R170C-Apo2L.0(32176-87C) on the growth of human COLO205 tumors in a mouse xenograft model

Athymic nude mice (Jackson Laboratories) were injected subcutaneously with 5 x 10⁶ COLO205 human colon carcinoma cells (NCI). Tumors were allowed to form and grow to a volume of about 150 mm³ as judged by caliper measurement. Mice (8 per group) were given i.v. injections of vehicle (2x/week), Apo2L.0 (60 mg/kg, 2x/week), Apo2L.0 (10 mg/kg, 2x/week),or PEG-R170C-Apo2L.0(32176-87C) (10 mg/kg, 2x/week). Tumor volume was measured every third day and treatment was stopped after two weeks. As shown in Figure 13, treatment with 10 mg/kg PEG-R170C-Apo2L.0(32176-87C) caused a greater reduction in tumor volume than an equivalent dose of Apo2L.0. The anti-tumor effect of 10 mg/kg PEG-R170C-Apo2L.0(32176-87C) was similar to that observed for the higher dose (60 mg/kg) of Apo2L.0. PEGylation of Apo2L on Cys170 lowers the dose required to achieve efficacy in this xenograft model of human cancer.

### EXAMPLE 7

### Preparation of partially PEGylated and disulfide crosslinked R170C-Apo2L.0

As described above, overnight air oxidation of R170C-Apo2L.0 yields a 600,000 D molecular weight species that has significantly increased in vitro bioactivity on SK-MES cells. However, preliminary results show that this higher molecular weight form does not have a significantly increased half-life in mice. Also, oxidized R170C-Apo2L.0 does not have increased anti-tumor activity in the mouse xenograft model and appears to be toxic towards some normal hepatocytes. In an effort to combine the increased bioactivity of the disulfide-linked form with the slower clearance of PEGylated Apo2L, PEGylation experiments were conducted using substoichiometric ratios of PEG-maleimide:R170C-Apo2L.0 monomer. This should allow both crosslinking and PEGylation on the same molecule.

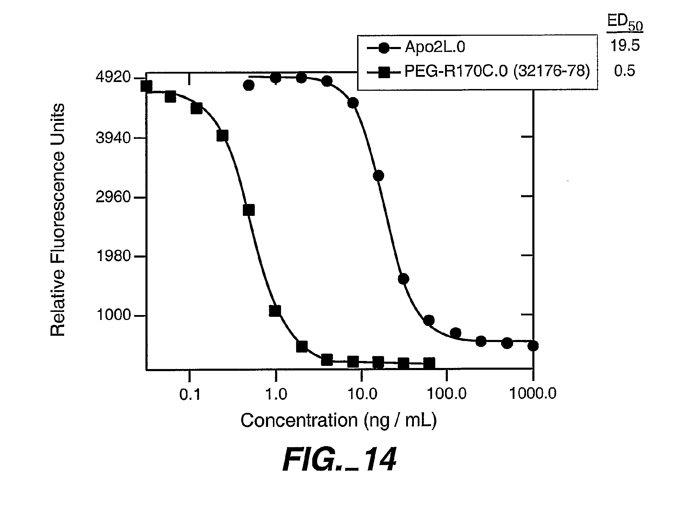

R170C-Apo2L.0 (95 mg) was prepared for the PEGylation reaction by removing the DTT on a Sephadex G-25 column equilibrated in HIC buffer. methoxy-PEG-maleimide, MW 2,000 D (Shearwater Polymers) was added to a final ratio of 0.75:1 PEG:Rl70C-Apo2L.0-monomer. The monomer concentration was 55 µM. This solution was incubated overnight at ambient temperature and then the reaction was quenched by addition of iodoacetamide to 100 µM. Excess reagents were removed, and the buffer was exchanged, by gel filtration of the modified protein on a Sephadex G-25 column equilibrated with arginine-succinate formulation buffer. This material is designated PEG-R170C-Apo2L.0(32176-78) and displayed increased receptor affinity (Table II) and *in vitro* bioactivity (Figure 14).

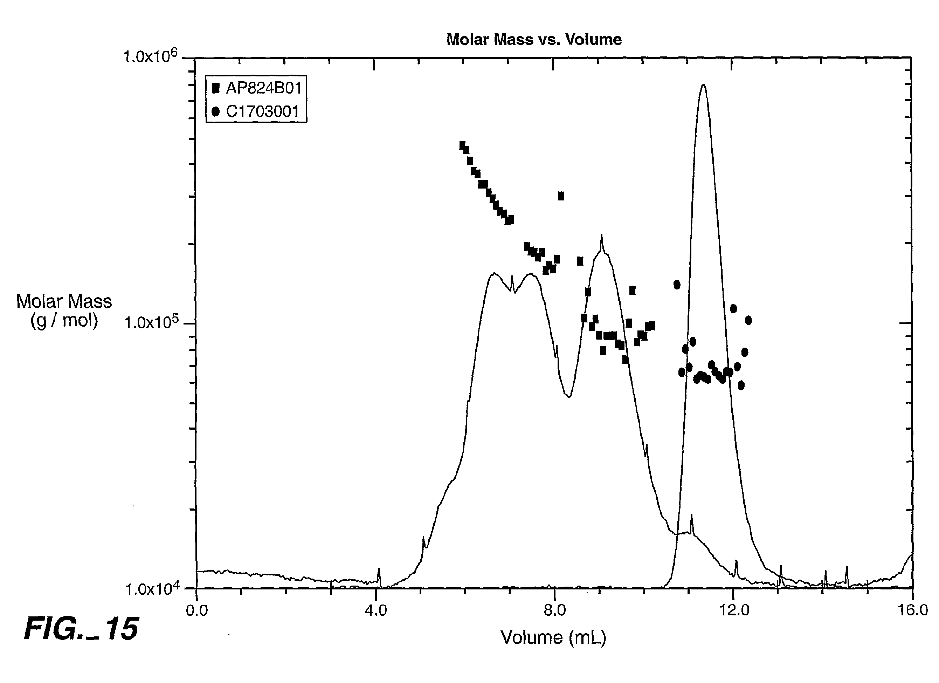

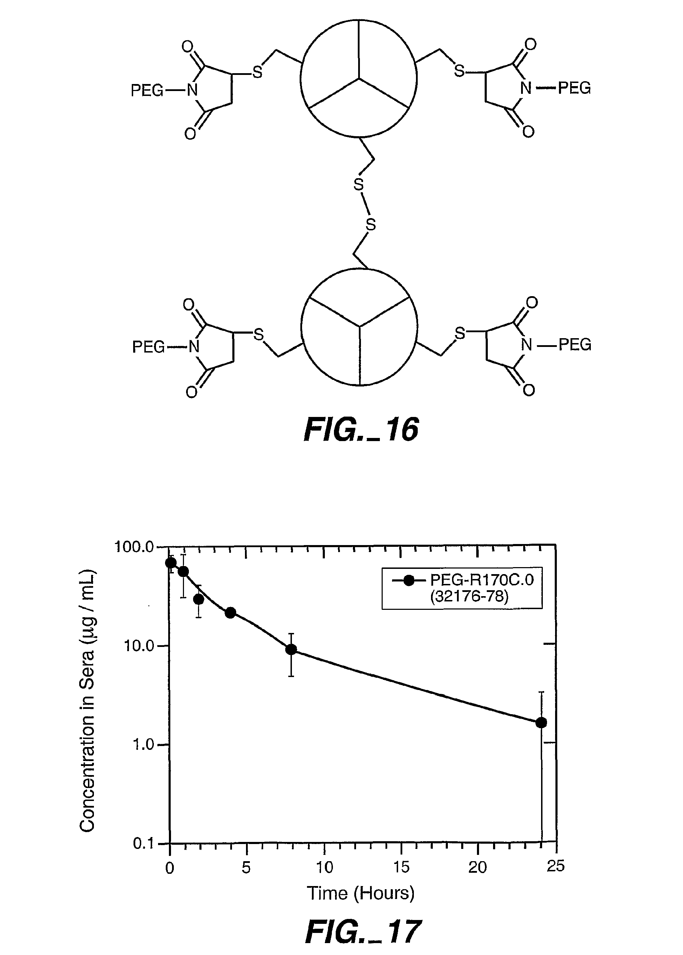

The hydrodynamic properties of PEG-R170C-Apo2L.0(32176-78) were examined by gel filtration chromatography as described above for lot 32176-87C except that the column was equilibrated and eluted with PBS. PEG-R170C-Apo2L.0(32176-78) elutes from the column in 3 main peaks (Figure 15). The first peak has a calculated molecular weight of 315,000 D and accounts for 30% of the material injected. The second peak has a calculated molecular weight of 194,000 D and represents 23% of the total. The third peak has a calculated molecular weight of 108,000 D and accounts for 46% of the total mass. Analysis by SDS-PAGE indicates that all three peaks contain PEGylated monomers as well as disulfide-linked dimers. The ratios of these components suggests that Peak 3 is predominately composed of fully PEGylated trimer, Peak 2 appears to be mostly "hexamer" - 2 trimers joined via a disulfide bond -, and Peak 1 is a "nonamer" having 3 trimers joined via disulfide bonds. A schematic diagram of the hexameric form is shown in Figure 16. Peak 1 has the highest activity in the apoptosis assay giving a relative potency of 50. Peak 2 gave a relative potency of 17 and peak 1 had a relative potency of 3.

The pharmacokinetics of PEG-R170C-Apo2L.0(32176-78) in the mouse were determined as described above for lot 32176-87C except that plasma samples were taken at 10 minutes, and 1, 2, 4, 8, and 24 hours. Plasma concentrations of PEG-R170C-Apo2L.0(32176-78) are plotted in Figure 17. Analysis of these data according to a two compartment model (Table III) shows that PEG-R170C-Apo2L.0(32176-78) has a 48-fold increased half-life and a 15-fold decreased rate of clearance relative to Apo2L.0.

**Table III. Analysis of pharmacokinetic data according to a two compartment model**

| Parameter | Apo2L.0 | PEG-R170C-Apo2L.0 (32176-78) |
|---|---|---|
| AUC (µg^{*}hour/mL) | 18.6 | 283 |
| K10(hour-1) | 12.5 | 0.258 |
| K12(hour-1) | 0.53 | 0.166 |
| Cmax (µg/mL) Observed | 87.4 | 67.3 |
| Cmax (µg/mL) Predicted | 232 | 73 |
| Cl (mL/hour/kg) | 536 | 35.3 |
| Vc(mL/kg) | 43.0 | 137 |
| Vss(mL/kg) | 66.5 | 244 |
| K10 half-life(hour) | 0.056 | 2.69 |

PEG-R170C-Apo2L.0 (32176-78) was tested in the mouse xenograft model as described above with the following modifications: 1) All injections were made i.p.; 2) PEG-R170C-Apo2L.0 (32176-78) injections were made 2x/week at 1, 3, or 10 mg/kg for 2 weeks; 3) Apo2L.0 injections were made 5x/week at 60 mg/kg or 2x/week at 10 mg/kg, both for 2 weeks. As shown in Figure 18, all three doses of PEG-R170C-Apo2L.0 (32176-78) caused complete tumor regression in all 8 animals of each group. Tumor volume was reduced to zero and maintained at that level after treatment was stopped. Both doses of Apo2L.0 caused a reduction in tumor volume but did not cause complete tumor regression in all treated animals. In the three groups of PEG-R170CApo2L.0 treatment, none of the animals had tumors after completion of the dosing regimen. There was a dose response in that the higher doses gave a faster elimination of the tumors. The groups treated with Apo2L.0 gave a smaller % of loss of tumors and tumors began to regrow upon cessation of treatment in 8/8 animals given the 60 mg/kg dose and 4/6 that received the 10 mg/kg dose. In the PEG-R170C-Apo2L.0 (32176-78) treated groups, tumors took longer to reappear and grew slowly. In the 1, 3, and 10 mg/kg PEG-R170C-Apo2L.0 (32176-78) treatment groups tumors reappeared in 2/7, 4/8, and 3/8 animals, respectively. These data show that partially PEGylated and crosslinked R170C-Apo2L.0 has a greater anti-tumor effect at a lower dose than observed with unmodified Apo2L.0.

The effects of lot nos. 32176-78 and 32176-87C of PEG-R170C-Apo2L.0 on normal hepatocytes from cynomologous monkeys are compared in Figure 19. Lot 32176-87C showed little toxicity towards hepatocytes but lot 32176-78 displayed some toxicity at intermediate, but not high, concentration. It is believed this concentration dependence is consistent with toxicity resulting from a higher order clustering of receptors on the cell surface.

### EXAMPLE 8

### Preparation of PEGylated K179C-Apo2L.0

Pharmacokinetic and efficacy experiments were also performed with a cysteine variant having a decreased tendency towards oligomerization. The K179C variant (prepared as described in the above Examples) was chosen for these experiments since this variant in its native form has comparable activity to the wild-type (native) Apo-2 ligand molecule and preliminary pegylation studies indicated only a 2-fold loss in activity upon Cys179 modification (see Table I). This variant did not appear to readily form disulfide-linked oligomers (data not shown). K179C-Apo2L.0 (40 mg) was concentrated to 8 mg/mL and reduced with 10 mM DTT for 2 hours at ambient temperature. The reducing agent was removed by gel filtration on a PD-10 column equilibrated with arginine-succinate formulation buffer. Protein concentration was determined by absorbance measurements and then 2K-methoxyPEG-maleimide was added to a final molar concentration ratio of 5:1 PEG:Apo2L monomer. This mixture was incubated overnight at ambient temperature and then the reaction was quenched by adding DTT to 5 mM. After 90 minutes, the reducing agent was blocked by addition of iodoacetamide to a concentration of 20 mM. Alkylation was allowed to proceed for 60 minutes and then the mixture was exchanged into arginine-succinate formulation buffer on a PD-10 column.

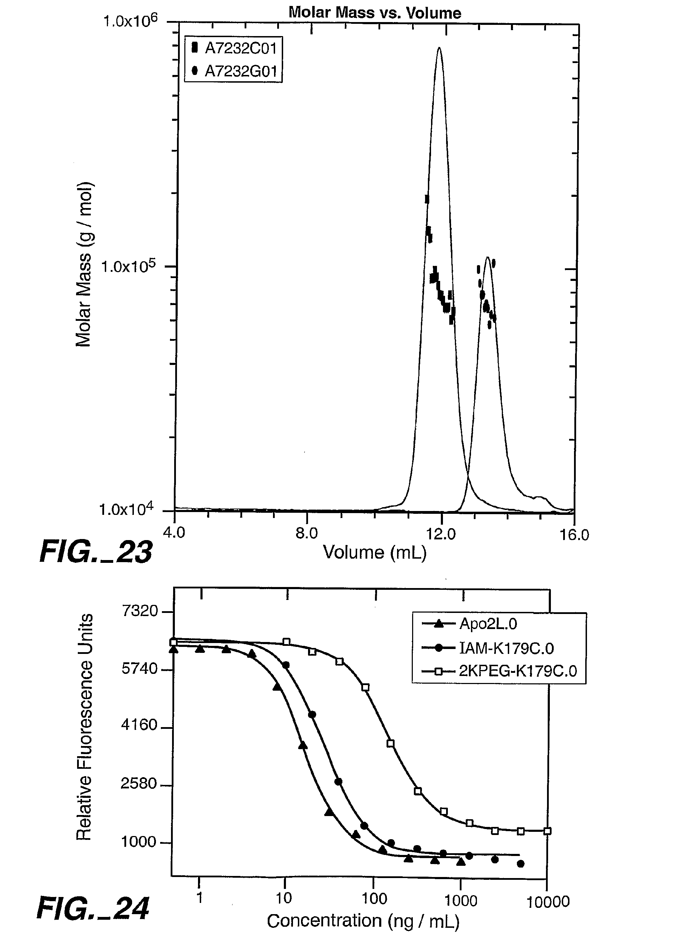

The hydrodynamic properties of 2K PEG-K179C-Apo2L.0 were examined by SEC-MALS on a Superdex 200 column equilibrated and eluted with PBS. 2K PEG-K179C-Apo2L.0 eluted as a single peak of elution volume (11.8 mL) with a calculated molar mass of 85,000 (Figure 23). Earlier eluting peaks were not detected, suggesting an absence of disulfide-linked oligomers in this preparation. PEGylation resulted in an increased apparent molecular weight since the iodoacetamide-modified form of K179C-Apo2L.0 eluted at 13.4 mL with a calculated molar mass of 70,000.

Apoptosis-inducing activity on SK-MES cells was measured for 2K PEG-K179C-Apo2L.0 as described in the Examples above. The PEGylated protein was highly active with only a 9-fold reduction in bioactivity relative to unmodified Apo2L.0 (Figure 24).

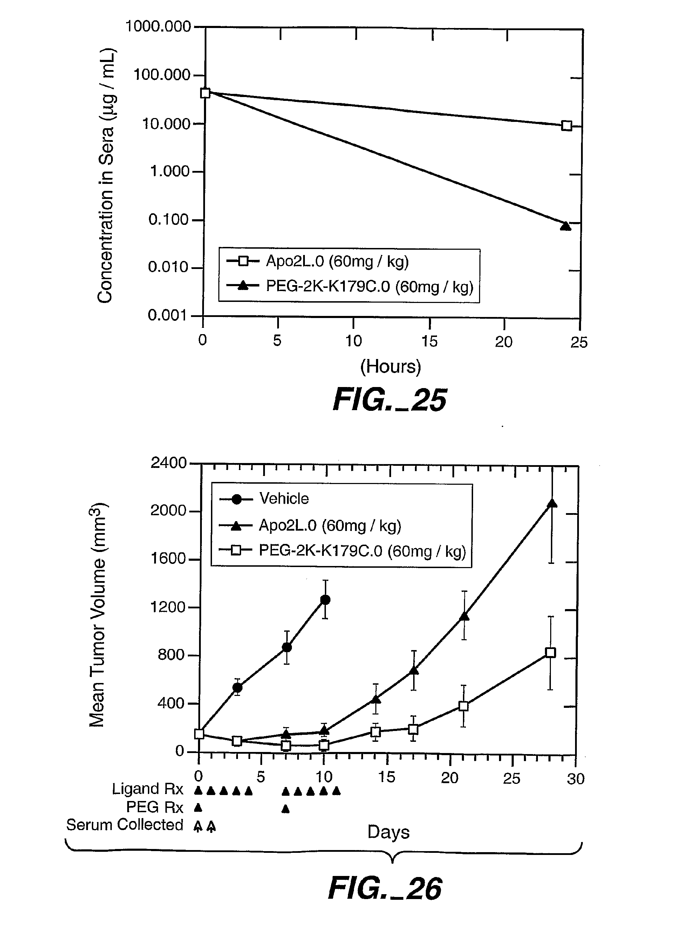

The effect of 2K PEG-K179C-Apo2L.0 on the growth of human COLO205 tumors was determined by using the mouse xenograft model described above (Example 6). Mice (8 per group) were given intraperitoneal injections of vehicle (5x/week), Apo2L.0 (60 mg/kg, 5x/week), or PEG-K179C-Apo2L.0 (60 mg/kg, 1x/week). Plasma samples were taken at 1 minute and 24 hours after the first injection. Apo2L concentrations in these samples were determined by ELISA. As shown in Figure 25, a much higher fraction of the injected dose was retained in the plasma after 24 hours for the PEGylated protein as compared to Apo2L.0. The tumor volume in the mice was measured every third day and treatment was stopped after two weeks. As shown in Figure 26, the 1x/week dosing of PEG-K179C-Apo2L.0 caused a larger reduction in mean tumor volume than 5x/week treatment with Apo2L.0.

### Clauses describing the invention:

1. An isolated Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1) : S96C; S101C ; S111C ; V114C ; R115C; E116C; N134C; N140C; E144C; N152C; S153C; R170C; R170K; R170S; K179C ; D234C ; E249C; R255C; E263C; H264C.
2. An isolated nucleic acid comprising a nucleotide sequence encoding the Apo-2 ligand variant of clause 1.
3. A vector comprising the nucleic acid of clause 2.
4. A host cell comprising the vector of clause 3.
5. The host cell of clause 4 wherein said host cell is E. coli, a yeast cell or CHO cell.
6. A method of making Apo-2 ligand variant polypeptide, comprising the steps of: providing a host cell comprising the vector of clause 4; (b) providing culture media; (c) culturing the host cell in the culture media under conditions sufficient to express the Apo-2 ligand variant polypeptide; (d) recovering the Apo-2 ligand variant polypeptide from the host cell or culture media; and (e) purifying the Apo-2 ligand variant polypeptide.
7. The Apo-2 ligand variant polypeptide of clause 1, wherein the Apo-2 ligand variant polypeptide is conjugated or linked to one or more polyol groups that increase the actual molecular weight of the Apo-2 ligand variant polypeptide.
8. The Apo-2 ligand variant polypeptide of clause 1, wherein the Apo-2 ligand variant polypeptide is conjugated or linked to one or more polyol groups that increase the in vivo half-life of the Apo-2 ligand variant polypeptide.
9. The Apo-2 ligand variant polypeptide of clause 7, wherein the one or more polyol groups is poly (ethylene glycol).
10. The Apo-2 ligand variant polypeptide of clause 9, where the Apo- 2 ligand variant polypeptide is conjugated or linked to one molecule of poly (ethylene glycol) having a molecular weight of about 2000 Daltons.
11. The Apo-2 ligand variant polypeptide of clause 1, wherein the Apo-2 ligand variant polypeptide is a soluble, extracellular domain Apo-2 ligand polypeptide.
12. An isolated Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1): S96C; S101C ; S111C ; V114C; R115C; E116C ; N134C; N140C; E144C; N152C; S153C ; R170C ; R170K; R170S; K179C ; D234C; E249C; R255C; E263C; H264C, wherein the Apo-2 ligand variant polypeptide binds to a polypeptide selected from the group consisting of DR4 receptor and DR5 receptor.
13. An isolated Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1): S96C; S101C ; S112C ; V114C ; R115C; E116C; N134C; N140C; E144C; N152C ; S153C; R170C ; R170K ; R170S ; K179C; D234C; E249C; R255C ; E263C; H264C, wherein the Apo-2 ligand variant polypeptide induces apoptosis in one or more mammalian cells.
14. An isolated nucleic acid comprising a nucleotide sequence encoding the Apo-2 ligand variant of clause 12.
15. A vector comprising the nucleic acid of clause 14.
16. A host cell comprising the vector of clause 15.
17. The host cell of clause 16 wherein said host cell is E. coli, a yeast cell or CHO cell.
18. A method of making Apo-2 ligand variant polypeptide, comprising the steps of: providing a host cell comprising the vector of clause 15; (b) providing culture media; (c) culturing the host cell in the culture media under conditions sufficient to express the Apo-2 ligand variant polypeptide ; (d) recovering the Apo-2 ligand variant polypeptide from the host cell or culture media; and (e) purifying the Apo-2 ligand variant polypeptide.
19. The Apo-2 ligand variant polypeptide of clause 12, wherein the Apo-2 ligand variant polypeptide is conjugated or linked to one or more polyol groups that increase the actual molecular weight of the Apo-2 ligand variant polypeptide.
20. The Apo-2 ligand variant polypeptide of clause 12, wherein the Apo-2 ligand variant polypeptide is conjugated or linked to one or more polyol groups that increase the in vivo half-life of the Apo-2 ligand variant polypeptide.
21. The Apo-2 ligand variant polypeptide of clause 19, wherein the one or more polyol groups is poly (ethylene glycol).
22. The Apo-2 ligand variant polypeptide of clause 21, where the Apo-2 ligand variant polypeptide is conjugated or linked to one molecule of poly (ethylene glycol) having a molecular weight of about 2000 Daltons.
23. The Apo-2 ligand variant polypeptide of clause 12, wherein the Apo-2 ligand variant polypeptide is a soluble, extracellular domain Apo-2 ligand polypeptide.
24. A composition comprising an Apo-2 ligand variant polypeptide conjugated or linked to one or more polyol groups, wherein the Apo-2 ligand variant polypeptide comprises an amino acid sequence which differs from the native sequence Apo-2 ligand polypeptide of Figure 1 (SEQ ID NO : 1) and has one or more of the following amino acid substitutions at the residue position (s) in Figure 1 (SEQ ID NO : 1) : S96C; S101C ; S111C ; V114C ; R115C; E116C ; N134C; N140C; E144C; N152C ; S153C ; R170C ; R170K ; R170S ; K179C; D234C; E249C ; R255C ; E263C; H264C.
25. A composition comprising an Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more polyol groups conjugated or linked to an amino acid substitution at the following residue position (s) in Figure 1 (SEQ ID NO : 1) : S96 ; S101 ; Sill ; V114; R115; E116 ; N134; N140; E144; N152; S153; R170; K179 ; D234 ; E249; R255; E263; H264.
26. A composition comprising an Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more polyol groups conjugated or linked to an amino acid substitution at the following residue position (s) in Figure 1 (SEQ ID NO : 1): S96C; S101C ; S111C ; V114C; R115C; E116C ; N134C; N140C; E144C; N152C; S153C; R170C; R170K; K179C; D234C; E249C ; R255C; E263C; H264C.
27. The composition of clause 24 wherein the one or more polyol groups is polyethylene glycol.
28. The composition of clause 27 wherein the polyethylene glycol has a molecular weight of about 1,000 to about 25,000 Daltons.
29. The composition of clause 28 wherein the polyethylene glycol has a molecular weight of about 2,000 Daltons.
30. A composition comprising an Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1) : S96C; S101C ; S111C ; V114C ; R115C; E116C ; N134C; N140C; E144C; N152C; S153C; R170C ; R170K ; R170S; K179C; D234C ; E249C ; R255C ; E263C ; H264C, wherein the Apo-2 ligand variant polypeptide binds to a polypeptide selected from the group consisting of DR4 receptor and DR5 receptor.
31. A composition comprising an Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more polyol groups conjugated or linked to an amino acid substitution at the residue position (s) in Figure 1 (SEQ ID NO : 1): S96 ; S101 ; S111 ; V114; R115; E116; N134; N140; E144; N152; S153 ; R170; K179 ; D234; E249; R255; E263; H264, wherein the Apo-2 ligand variant polypeptide binds to a polypeptide selected from the group consisting of DR4 receptor and DR5 receptor.
32. A composition comprising an Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more polyol groups conjugated or linked to an amino acid substitution at the residue position (s) in Figure 1 (SEQ ID NO : 1) : S96C; S101C ; S111C ; V114C; R115C; E116C; N134C; N140C; E144C; N152C; S153C; R170C ; R170K ; K179C; D234C; E249C; R255C; E263C; H264C, wherein the Apo-2 ligand variant polypeptide binds to a polypeptide selected from the group consisting of DR4 receptor and DR5 receptor.
33. The composition of clause 30 wherein the one or more polyol groups is polyethylene glycol.
34. The composition of clause 33 wherein the polyethylene glycol has a molecular weight of about 1,000 to about 25,000 Daltons.
35. The composition of clause 34 wherein the polyethylene glycol has a molecular weight of about 2,000 Daltons.
36. An Apo-2 ligand trimer comprising at least one Apo-2 ligand variant polypeptide comprising an amino acid sequence which differs from the native sequence Apo-2 ligand polypeptide sequence of Figure 1 (SEQ ID NO : 1) and has one or more amino acid substitutions at the following residue position (s) in Figure 1 (SEQ ID NO : 1): S96 ; S101 ; Sill ; V114 ; R115 ; E116; N134; N140 ; E144 ; N152; S153 ; R170; K179 ; D234 ; E249; R255; E263; H264.
37. An Apo-2 ligand trimer comprising at least one Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1): S96C; S101C ; S111C ; V114C; R115C ; E116C ; N134C; N140C; E144C ; N152C ; S153C; R170C; R170K; R170S ; K179C; D234C ; E249C ; R255C; E263C; H264C.
38. An Apo-2 ligand trimer comprising at least one Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1): S96C; S101C ; S111C ; V114C; R115C ; E116C; N134C; N140C; E144C; N152C; S153C; R170C; R170K ; R170S; K179C; D234C; E249C; R255C; E263C; H264C, wherein the Apo-2 ligand variant polypeptide binds to a polypeptide selected from the group consisting of DR4 receptor and DR5 receptor.
39. An Apo-2 ligand trimer comprising at least one Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1) : S96C; S101C ; S111C ; V114C; R115C ; E116C; N134C; N140C; E144C; N152C ; S153C; R170C; R170K ; R170S; K179C; D234C; E249C; R255C; E263C; H264C, wherein the Apo-2 ligand variant polypeptide induces apoptosis in one or more mammalian cells.
40. The Apo-2 ligand trimer of clause 36, wherein the trimer comprises at least two of said Apo-2 ligand variant polypeptides.
41. The Apo-2 ligand trimer of clause 36, wherein the Apo-2 ligand trimer comprises three of said Apo-2 ligand variant polypeptides.
42. An Apo-2 ligand trimer comprising at least one Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1) : S96C; S101C ; S111C ; V114C; R115C ; E116C ; N134C ; N140C ; E144C ; N152C ; S153C; R170C; R170K ; R170S; K179C; D234C; E249C; R255C; E263C ; H264C.
43. An Apo-2 ligand trimer comprising at least one Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more polyol groups conjugated or linked to an amino acid substitution at the residue position (s) in Figure 1 (SEQ ID NO : 1): S96C; S101C ; S111C ; V114C; R115C; E116C; N134C; N140C; E144C; N152C; S153C ; R170C; R170K; K179C; D234C; E249C; R255C; E263C; H264C, wherein the Apo-2 ligand variant polypeptide binds to a polypeptide selected from the group consisting of DR4 receptor and DR5 receptor.
44. The Apo-2 ligand trimer of clause 42, wherein the trimer comprises three of said Apo-2 ligand variant polypeptides conjugated or linked to one or more polyol groups.
45. The Apo-2 ligand trimer of clause 42 wherein the one or more polyol groups is polyethylene glycol.
46. The Apo-2 ligand trimer of clause 45 wherein the polyethylene glycol has a molecular weight of about 1,000 to about 25,000 Daltons.
47. The Apo-2 ligand trimer of clause 46 wherein the polyethylene glycol has a molecular weight of about 2,000 Daltons.
48. The isolated Apo-2 ligand variant polypeptide of clause 42, wherein the Apo-2 ligand variant polypeptide induces apoptosis in one or more mammalian cells.
49. An isolated Apo-2 ligand variant polypeptide comprising an amino acid sequence which differs from the native sequence Apo-2 ligand polypeptide sequence of Figure 1 (SEQ ID NO : 1) and has one or more amino acid substitutions at a residue position identified from an x-ray crystal structure of the DR5*Apo2L complex as shown in Figure 3 such that the residue position is: (a) outside of the receptor contact region of the DR5*Apo2L complex as shown in Figure 3; and (b) displays high solvent accessibility in the crystal structure of the DR5*Apo2L complex as shown in Figure 3.
50. The isolated Apo-2 ligand variant polypeptide of clause 49, wherein the residue position is located on one face of the Apo2L monomer from top to bottom as shown in the crystal structure of the DR5^{*}Apo2L complex as shown in Figure 3.
51. The isolated Apo-2 ligand variant polypeptide of clause 49, wherein the Apo-2 ligand variant polypeptide has one or more of the following amino acid substitutions at the residue position (s) in Figure 1 (SEQ ID NO : 1): V114; R115; E116; N134; N140; E144; N152; S153; R170; D234; E249; R255; E263; H264.
52. The isolated Apo-2 ligand variant polypeptide of clause 49, wherein the isolated Apo-2 ligand variant polypeptide is conjugated or linked to one or more polyol groups.
53. The isolated Apo-2 ligand variant polypeptide of clause 52, wherein the one or more polyol groups is polyethylene glycol.
54. The isolated Apo-2 ligand variant polypeptide of clause 53, wherein the polyethylene glycol has a molecular weight of about 1,000 to about 25,000 Daltons.
55. The isolated Apo-2 ligand variant polypeptide of clause 54, wherein the polyethylene glycol has a molecular weight of about 2,000 Daltons.
56. The isolated Apo-2 ligand variant polypeptide of clause 49, wherein the Apo-2 ligand variant polypeptide binds to a polypeptide selected from the group consisting of DR4 receptor and DR5 receptor.
57. The isolated Apo-2 ligand variant polypeptide of clause 49, wherein the Apo-2 ligand variant polypeptide induces apoptosis in one or more mammalian cells.
58. An isolated Apo-2 ligand variant polypeptide comprising an amino acid sequence which differs from the native sequence Apo-2 ligand polypeptide sequence of Figure 1 (SEQ ID NO : 1) by having an amino acid substitution at a residue position in Figure 1 (SEQ ID NO : 1) selected from the group consisting of S96C; S101C ; S111C ; V114C; R115C; E116C ; N134C; N140C; E144C ; N152C ; S153C; R170C; R170K; R170S; K179C ; D234C ; E249C; R255C ; E263C ; and H264C.
59. A composition comprising an Apo-2 ligand variant polypeptide conjugated or linked to a polyethylene glycol group having a molecular weight of about 2,000 Daltons, wherein the Apo-2 ligand variant polypeptide comprises an amino acid sequence which differs from the native sequence Apo-2 ligand polypeptide sequence of Figure 1 (SEQ ID NO : 1) and the polyethylene glycol group is conjugated or linked to a residue position in Figure 1 (SEQ ID NO : 1) selected from the group consisting of S96C; S101C ; S111C ; V114C ; R115C; E116C; N134C; N140C; E144C; N152C ; S153C ; R170C ; R170K ; R170S; K179C; D234C ; E249C; R255C; E263C; and H264C.
60. An Apo-2 ligand trimer comprising three Apo-2 ligand variant polypeptides conjugated or linked to a polyethylene glycol group having a molecular weight of about 2,000 Daltons, wherein the Apo-2 ligand variant polypeptides comprise an amino acid sequence which differs from the native sequence Apo-2 ligand polypeptide sequence of Figure 1 (SEQ ID NO : 1) and the polyethylene glycol group is conjugated or linked to a residue position in Figure 1 (SEQ ID NO : 1) selected from the group consisting of S96C; S101C ; S111C ; V114C; R115C; E116C; N134C; N140C ; E144C ; N152C ; S153C ; R170C; R170K; R170S; K179C; D234C; E249C; R255C; E263C; and H264C.
61. The isolated Apo-2 ligand variant polypeptide of clause 60, wherein the Apo-2 ligand variant polypeptide binds to a polypeptide selected from the group consisting of DR4 receptor and DR5 receptor.
62. The isolated Apo-2 ligand variant polypeptide of clause 60, wherein the Apo-2 ligand variant polypeptide induces apoptosis in one or more mammalian cells.
63. A pharmaceutical composition comprising an effective amount of isolated Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1) : S96C; S101C ; S111C ; V114C; R115C; E116C; N134C; N140C; E144C ; N152C ; S153C ; R170C; R170K; R170S; K179C; D234C; E249C; R255C; E263C; and H264C, in admixture with a pharmaceutically acceptable carrier.
64. A pharmaceutical composition comprising an effective amount of a composition comprising an Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1): S96C; S101C ; S111C ; V114C ; R115C ; E116C; N134C ; N140C ; E144C; N152C; S153C; R170C; R170K; R170S; K179C ; D234C; E249C; R255C; E263C; and H264C, in admixture with a pharmaceutically acceptable carrier, wherein the composition binds to a polypeptide selected from the group consisting of DR4 receptor and DR5 receptor.
65. A pharmaceutical composition comprising an effective amount of Apo-2 ligand trimer comprising at least one Apo-2 ligand variant polypeptide comprising an amino acid sequence which differs from the native sequence Apo-2 ligand polypeptide sequence of Figure 1 (SEQ ID NO : 1) and has one or more amino acid substitutions at the following residue position (s) in Figure 1 (SEQ ID NO : 1) : S96 ; S101 ; Sill ; V114; R115 ; E116; N134; N140; E144; N152; 5153 ; R170; K179 ; D234; E249; R255; E263 ; and H264, in admixture with a pharmaceutically acceptable carrier.
66. The pharmaceutical composition of clause 63, wherein said pharmaceutical composition further comprises one or more divalent metal ions.
67. The pharmaceutical composition of clause 63, wherein the Apo-2 ligand variant polypeptide induces apoptosis in one or more mammalian cells.
68. A method of inducing apoptosis in mammalian cells comprising exposing mammalian cells expressing a receptor selected from the group consisting of DR4 receptor and DR5 receptor to a therapeutically effective amount of isolated Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1): S96C; S101C ; S111C ; V114C ; R115C; E116C; N134C; N140C; E144C; N152C; S153C; R170C; R170K; R170S; K179C ; D234C ; E249C; R255C; E263C; H264C.
69. A method of inducing apoptosis in mammalian cells comprising exposing mammalian cells expressing a receptor selected from the group consisting of DR4 receptor and DR5 receptor to a therapeutically effective amount of a composition comprising Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more polyol groups conjugated or linked to an amino acid substitution at the residue position (s) in Figure 1 (SEQ ID NO : 1): S96 ; S101 ; Sill ; V114; R115; E116; N134; N140; E144; N152; S153 ; R170; K179; D234; E249 ; R255 ; E263; H264.
70. A method of inducing apoptosis in mammalian cells comprising exposing mammalian cells expressing a receptor selected from the group consisting of DR4 receptor and DR5 receptor to a therapeutically effective amount of Apo-2 ligand trimer comprising at least one Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID No : 1) : S96C; S101C ; S111C ; V114C; R115C; E116C; N134C; N140C; E144C; N152C; S153C ; R170C; R170K; R170S; K179C ; D234C; E249C; R255C; E263C; H264C.
71. The method of clause 68 wherein the mammalian cells are colon or colorectal cancer cells.
72. A method of treating cancer in a mammal, comprising administering to said mammal an effective amount of isolated Apo-2 ligand variant polypeptide comprising an amino acid sequence which differs from the native sequence Apo-2 ligand polypeptide sequence of Figure 1 (SEQ iu NO : 1) and has one or more of the following amino acid substitutions at the residue position (s) in Figure 1 (SEQ ID NO : 1): S96C; S101C ; S111C ; V114C; R115C; E116C; N134C; N140C; E144C; N152C; S153C; R170C; R170K ; R170S ; K179C; D234C; E249C; R255C; E263C; H264C.
73. A method of treating cancer in a mammal, comprising administering to said mammal an effective amount of a composition comprising Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID NO : 1) and has one or more polyol groups conjugated or linked to an amino acid substitution at the residue position (s) in Figure 1 (SEQ ID NO : 1): S96 ; S101 ; Sill ; V114 ; R115; E116; N134; N140; E144 ; N152; S153 ; R170; K179 ; D234; E249 ; R255; E263; H264, wherein the composition binds to a polypeptide selected from the group consisting of DR4 receptor and DR5 receptor.
74. A method of treating cancer in a mammal, comprising administering to said mammal an effective amount of Apo-2 ligand trimer comprising at least one Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1) : S96C; S101C ; S111C ; V114C ; R115C ; E116C ; N134C; N140C; E144C; N152C; S153C; R170C; R170K; R170S; K179C; D234C; E249C; R255C; E263C; H264C.
75. The method of clause 72, wherein said cancer is lung cancer, breast cancer, colon cancer or colorectal cancer.
76. A method of treating an immune-related disease in a mammal comprising administering to said mammal an effective amount of isolated Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1) : S96C; S101C ; S111C ; V114C; R115C; E116C; N134C; N140C; E144C ; N152C; S153C ; R170C; R170K; R170S; K179C; D234C; E249C; R255C; E263C; H264C.
77. A method of treating an immune-related disease in a mammal comprising administering to said mammal an effective amount of a composition comprising Apo-2 ligand variant polypeptide conjugated or linked to one or more polyol groups, wherein the Apo-2 ligand variant polypeptide comprises an amino acid sequence which differs from the native sequence Apo-2 ligand polypeptide sequence of Figure 1 (SEQ ID No : 1) and has one or more polyol groups conjugated or linked to an amino acid substitution at the residue position (s) in Figure 1 (SEQ ID N0 : 1) : S96 ; S101 ; Sill ; V114 ; R115; E116; N134 ; N140 ; E144; N152; S153 ; R170; K179; D234; E249; R255; E263 ; H264, wherein the composition binds to a polypeptide selected from the group consisting of DR4 receptor and DR5 receptor.
78. A method of treating an immune-related disease in a mammal comprising administering to said mammal an effective amount of Apo-2 ligand trimer comprising at least one Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1): S96C; S101C ; S111C ; V114C ; R115C; E116C; N134C ; N140C; E144C ; N152C; S153C; R170C; R170K; R170S ; K179C ; D234C; E249C ; R255C ; E263C; H264C.
79. The method of clause 76, wherein said immune-related disease is arthritis or multiple sclerosis.
80. A method of preparing an Apo-2 ligand oligomer, comprising linking at least two Apo-2 ligand trimers, wherein at least one Apo-2 ligand monomer in each Apo-2 ligand trimer comprises an Apo-2 ligand variant polypeptide having a cysteine amino acid substitution at amino acid residue position 170 in Figure 1 (SEQ ID NO : 1), and wherein the Apo-2 ligand trimers are linked by disulfide bonds between the cysteine amino acid residues at position 170 in the Apo-2 ligand variant polypeptides.
81. An Apo-2 ligand oligomer comprising at least two Apo-2 ligand trimers, wherein at least one Apo-2 ligand monomer in each Apo-2 ligand trimer comprises an Apo-2 ligand variant polypeptide having a cysteine amino acid substitution at amino acid residue position 170 in Figure 1 (SEQ ID NO : 1), and wherein the Apo-2 ligand trimers are linked by disulfide bonds between the cysteine amino acid residues at position 170 in the Apo-2 ligand variant polypeptides.
82. A kit, comprising a container and, within the container, an isolated Apo-2 ligand variant polypeptide comprising an amino acid sequence which differs from the native sequence Apo-2 ligand polypeptide sequence of Figure 1 (SEQ ID NO : 1) and has one or more of the following amino acid substitutions at the residue position (s) in Figure 1 (SEQ ID NO : 1) : S96C; S101C ; S111C ; V114C; R115C; E116C; N134C; N140C; E144C; N152C; S153C ; R170C; R170K; R170S; K179C; D234C; E249C; R255C; E263C; H264C.

## Claims

1. A conjugate comprising an Apo-2 ligand variant polypeptide covalently linked to one or more polyol groups, wherein the Apo-2 ligand variant comprises an amino acid sequence which differs from the native sequence Apo-2 ligand polypeptide sequence of Figure 1 (SEQ ID NO: 1) by the substitution of one or more amino acids for a cysteine amino acid residue, wherein the polyol groups are conjugated to the Apo-2 ligand variant via the cysteine residues.

2. The conjugate of claim 1, wherein the Apo-2 ligand polypeptide has at least 80% amino acid sequence identity to an Apo-2 ligand polypeptide having an amino acid sequence from residues 114 to 281 inclusive of Figure 1 (SEQ ID NO: 1).

3. The conjugate of claim 1 or claim 2, wherein the Apo-2 ligand variant polypeptide has one or more of the following amino acid substitutions at the residue position(s) in Figure 1 (SEQ ID NO:1): S96C; S101C; S111C; V114C; R115C; E116C; N134C; N140C; E144C; N152C; S153C; R170C; R170K; R170S; K179C; D234C; E249C; R255C; E263C; H264C.

4. The conjugate of any one of claims 1 to 3, wherein Apo-2 ligand variant polypeptide comprises one or more amino acid substitutions at a residue position identified from an x-ray crystal structure of the DR5•Apo2L complex as shown in Figure 3 such that the residue position is:
(a) outside of the receptor contact region of the DR5·Apo2L complex as shown in Figure 3; and
(b) displays high solvent accessibility in the crystal structure of the DR5•Apo2L complex as shown in Figure 3.

5. The conjugate of any one of the preceding claims, wherein the Apo-2 ligand variant polypeptide binds to a polypeptide selected from the group consisting of DR4 receptor and DR5 receptor.

6. The conjugate of any one of the preceding claims, wherein, the Apo-2 ligand variant polypeptide induces apoptosis in one or more mammalian cells.

7. The Apo-2 ligand variant polypeptide of any one of the preceding claims, wherein the Apo-2 ligand variant polypeptide is conjugated or linked to one or more polyol groups that increase the actual molecular weight of the Apo-2 ligand variant polypeptide and/or that increase the in vivo half-life of the Apo-2 ligand variant polypeptide.

8. The conjugate of any one of the preceding claims, wherein the one or more polyol groups is poly(ethylene glycol).

9. The conjugate of any one of the preceding claims, wherein the Apo-2 ligand variant polypeptide is conjugated or linked to one molecule of poly(ethylene glycol) having a molecular weight of about 2000 Daltons.

10. The conjugate of any one of the preceding claims, wherein the Apo-2 ligand variant polypeptide is a soluble, extracellular domain Apo-2 ligand polypeptide.

11. An Apo-2 ligand trimer comprising at least one Apo-2 ligand conjugate of any one of claims 1 to 10.

12. A composition comprising a conjugate of any one of claims 1 to 10, in combination with a carrier.

13. A conjugate of any one of claims 1 to 10 for use in therapy.

14. A conjugate of any one of claims 1 to 10 for use in a method of treating cancer or an immune related disorder.

15. The conjugate for use in a method of treating cancer of claim 14, wherein the cancer is lung cancer, breast cancer, colon cancer or colorectal cancer or the immune related disorder is arthritis or multiple sclerosis.

16. An Apo-2 ligand variant polypeptide which comprises an amino acid sequence which differs from the native sequence Apo-2 ligand polypeptide sequence of Figure 1 (SEQ ID NO: 1) by the substitution of one or more amino acids for a cysteine amino acid residue at the residue position(s) in Figure 1 (SEQ ID NO:1): S96C; S101C; S111C; V114C; R115C; E116C; N134C; N140C; E144C; N152C; S153C; R170C; R170K; R170S; K179C; D234C; E249C; R255C; E263C; H264C.


Sequence Listing

<110> Genentech, Inc.

<120> APO-2 Ligand Variants and Uses Thereof

<130> SJK/FP6720684

<140> EP TBC
<141> 2002-10-01

<150> EP 02766436.6
<151> 2002-10-01

<150> PCT/US02/31210
<151> 2002-10-01

<150> US 60/326,622
<151> 2001-10-02

<160> 6

<210> 1
<211> 281
<212> PRT
<213> Homo Sapiens

<400> 1

<210> 2
<211> 1042
<212> DNA
<213> Homo Sapiens

<220>
<221> unsure
<222> 447
<223> unknown base

<400> 2
tttcctcact gactataaaa gaatagagaa ggaagggctt cagtgaccgg 50

ctgcctggct gacttacagc agtcagactc tgacaggatc atggctatga 100

tggaggtcca ggggggaccc agcctgggac agacctgcgt gctgatcgtg 150

atcttcacag tgctcctgca gtctctctgt gtggctgtaa cttacgtgta 200

ctttaccaac gagctgaagc agatgcagga caagtactcc aaaagtggca 250

ttgcttgttt cttaaaagaa gatgacagtt attgggaccc caatgacgaa 300

gagagtatga acagcccctg ctggcaagtc aagtggcaac tccgtcagct 350

cgttagaaag atgattttga gaacctctga ggaaaccatt tctacagttc 400

aagaaaagca acaaaatatt tctcccctag tgagagaaag aggtccncag 450

agagtagcag ctcacataac tgggaccaga ggaagaagca acacattgtc 500

ttctccaaac tccaagaatg aaaaggctct gggccgcaaa ataaactcct 550

gggaatcatc aaggagtggg cattcattcc tgagcaactt gcacttgagg 600

aatggtgaac tggtcatcca tgaaaaaggg ttttactaca tctattccca 650

aacatacttt cgatttcagg aggaaataaa agaaaacaca aagaacgaca 700

aacaaatggt ccaatatatt tacaaataca caagttatcc tgaccctata 750

ttgttgatga aaagtgctag aaatagttgt tggtctaaag atgcagaata 800

tggactctat tccatctatc aagggggaat atttgagctt aaggaaaatg 850

acagaatttt tgtttctgta acaaatgagc acttgataga catggaccat 900

gaagccagtt ttttcggggc ctttttagtt ggctaactga cctggaaaga 950

aaaagcaata acctcaaagt gactattcag ttttcaggat gatacactat 1000

gaagatgttt caaaaaatct gaccaaaaca aacaaacaga aa 1042

<210> 3
<211> 468
<212> PRT
<213> Homo Sapiens

<400> 3
Met Ala Pro Pro Pro Ala Arg Val His Leu Gly Ala Phe Leu Ala
1               5                   10                  15

Val Thr Pro Asn Pro Gly Ser Ala Ala Ser Gly Thr Glu Ala Ala
                20                  25                  30

Ala Ala Thr Pro Ser Lys Val Trp Gly Ser Ser Ala Gly Arg Ile
                35                  40                  45

Glu Pro Arg Gly Gly Gly Arg Gly Ala Leu Pro Thr Ser Met Gly
                50                  55                  60

Gln His Gly Pro Ser Ala Arg Ala Arg Ala Gly Arg Ala Pro Gly
                65                  70                  75

Pro Arg Pro Ala Arg Glu Ala Ser Pro Arg Leu Arg Val His Lys
                80                  85                  90

Thr Phe Lys Phe Val Val Val Gly Val Leu Leu Gln Val Val Pro
                95                  100                 105

Ser Ser Ala Ala Thr Ile Lys Leu His Asp Gln Ser Ile Gly Thr
                110                 115                 120

Gln Gln Trp Glu His Ser Pro Leu Gly Glu Leu Cys Pro Pro Gly
                125                 130                 135

Ser His Arg Ser Glu Arg Pro Gly Ala Cys Asn Arg Cys Thr Glu
                140                 145                 150

Gly Val Gly Tyr Thr Asn Ala Ser Asn Asn Leu Phe Ala Cys Leu
                155                 160                 165

Pro Cys Thr Ala Cys Lys Ser Asp Glu Glu Glu Arg Ser Pro Cys
                170                 175                 180

Thr Thr Thr Arg Asn Thr Ala Cys Gln Cys Lys Pro Gly Thr Phe
                185                 190                 195

Arg Asn Asp Asn Ser Ala Glu Met Cys Arg Lys Cys Ser Thr Gly
                200                 205                 210

Cys Pro Arg Gly Met Val Lys Val Lys Asp Cys Thr Pro Trp Ser
                215                 220                 225

Asp Ile Glu Cys Val His Lys Glu Ser Gly Asn Gly His Asn Ile
                230                 235                 240

Trp Val Ile Leu Val Val Thr Leu Val Val Pro Leu Leu Leu Val
                245                 250                 255

Ala Val Leu Ile Val Cys Cys Cys Ile Gly Ser Gly Cys Gly Gly
                260                 265                 270

Asp Pro Lys Cys Met Asp Arg Val Cys Phe Trp Arg Leu Gly Leu
                275                 280                 285

Leu Arg Gly Pro Gly Ala Glu Asp Asn Ala His Asn Glu Ile Leu
                290                 295                 300

Ser Asn Ala Asp Ser Leu Ser Thr Phe Val Ser Glu Gln Gln Met
                305                 310                 315

Glu Ser Gln Glu Pro Ala Asp Leu Thr Gly Val Thr Val Gln Ser
                320                 325                 330

Pro Gly Glu Ala Gln Cys Leu Leu Gly Pro Ala Glu Ala Glu Gly
                335                 340                 345

Ser Gln Arg Arg Arg Leu Leu Val Pro Ala Asn Gly Ala Asp Pro
                350                 355                 360

Thr Glu Thr Leu Met Leu Phe Phe Asp Lys Phe Ala Asn Ile Val
                365                 370                 375

Pro Phe Asp Ser Trp Asp Gln Leu Met Arg Gln Leu Asp Leu Thr
                380                 385                 390

Lys Asn Glu Ile Asp Val Val Arg Ala Gly Thr Ala Gly Pro Gly
                395                 400                 405

Asp Ala Leu Tyr Ala Met Leu Met Lys Trp Val Asn Lys Thr Gly
                410                 415                 420

Arg Asn Ala Ser Ile His Thr Leu Leu Asp Ala Leu Glu Arg Met
                425                 430                 435

Glu Glu Arg His Ala Lys Glu Lys Ile Gln Asp Leu Leu Val Asp
                440                 445                 450

Ser Gly Lys Phe Ile Tyr Leu Glu Asp Gly Thr Gly Ser Ala Val
                455                 460                 465

Ser Leu Glu

<210> 4
<211> 1407
<212> DNA
<213> Homo Sapiens

<400> 4
atggcgccac caccagctag agtacatcta ggtgcgttcc tggcagtgac 50

tccgaatccc gggagcgcag cgagtgggac agaggcagcc gcggccacac 100

ccagcaaagt gtggggctct tccgcgggga ggattgaacc acgaggcggg 150

ggccgaggag cgctccctac ctccatggga cagcacggac ccagtgcccg 200

ggcccgggca gggcgcgccc caggacccag gccggcgcgg gaagccagcc 250

ctcggctccg ggtccacaag accttcaagt ttgtcgtcgt cggggtcctg 300

ctgcaggtcg tacctagctc agctgcaacc atgatcaatc aattggcaca 350

aattggcaca cagcaatggg aacatagccc tttgggagag ttgtgtccac 400

caggatctca tagatcagaa cgtcctggag cctgtaaccg gtgcacagag 450

ggtgtgggtt acaccaatgc ttccaacaat ttgtttgctt gcctcccatg 500

tacagcttgt aaatcagatg aagaagagag aagtccctgc accacgacca 550

ggaacacagc atgtcagtgc aaaccaggaa ctttccggaa tgacaattct 600

gctgagatgt gccggaagtg cagcacaggg tgccccagag ggatggtcaa 650

ggtcaaggat tgtacgccct ggagtgacat cgagtgtgtc cacaaagaat 700

caggcaatgg acataatata tgggtgattt tggttgtgac tttggttgtt 750

ccgttgctgt tggtggctgt gctgattgtc tgttgttgca tcggctcagg 800

ttgtggaggg gaccccaagt gcatggacag ggtgtgtttc tggcgcttgg 850

gtctcctacg agggcctggg gctgaggaca atgctcacaa cgagattctg 900

agcaacgcag actcgctgtc cactttcgtc tctgagcagc aaatggaaag 950

ccaggagccg gcagatttga caggtgtcac tgtacagtcc ccaggggagg 1000

cacagtgtct gctgggaccg gcagaagctg aagggtctca gaggaggagg 1050

ctgctggttc cagcaaatgg tgctgacccc actgagactc tgatgctgtt 1100

ctttgacaag tttgcaaaca tcgtgccctt tgactcctgg gaccagctca 1150

tgaggcagct ggacctcacg aaaaatgaga tcgatgtggt cagagctggt 1200

acagcaggcc caggggatgc cttgtatgca atgctgatga aatgggtcaa 1250

caaaactgga cggaacgcct cgatccacac cctgctggat gccttggaga 1300

ggatggaaga gagacatgca aaagagaaga ttcaggacct cttggtggac 1350

tctggaaagt tcatctactt agaagatggc acaggctctg ccgtgtcctt 1400

ggagtga 1407

<210> 5
<211> 411
<212> PRT
<213> Homo Sapiens

<220>
<221> unsure
<222> 410
<223> unknown amino acid

<400> 5
Met Glu Gln Arg Gly Gln Asn Ala Pro Ala Ala Ser Gly Ala Arg
1               5                   10                  15

Lys Arg His Gly Pro Gly Pro Arg Glu Ala Arg Gly Ala Arg Pro
                20                  25                  30

Gly Leu Arg Val Pro Lys Thr Leu Val Leu Val Val Ala Ala Val
                35                  40                  45

Leu Leu Leu Val Ser Ala Glu Ser Ala Leu Ile Thr Gln Gln Asp
                50                  55                  60

Leu Ala Pro Gln Gln Arg Ala Ala Pro Gln Gln Lys Arg Ser Ser
                65                  70                  75

Pro Ser Glu Gly Leu Cys Pro Pro Gly His His Ile Ser Glu Asp
                80                  85                  90

Gly Arg Asp Cys Ile Ser Cys Lys Tyr Gly Gln Asp Tyr Ser Thr
                95                  100                 105

His Trp Asn Asp Leu Leu Phe Cys Leu Arg Cys Thr Arg Cys Asp
                110                 115                 120

Ser Gly Glu Val Glu Leu Ser Pro Cys Thr Thr Thr Arg Asn Thr
                125                 130                 135

Val Cys Gln Cys Glu Glu Gly Thr Phe Arg Glu Glu Asp Ser Pro
                140                 145                 150

Glu Met Cys Arg Lys Cys Arg Thr Gly Cys Pro Arg Gly Met Val
                155                 160                 165

Lys Val Gly Asp Cys Thr Pro Trp Ser Asp Ile Glu Cys Val His
                170                 175                 180

Lys Glu Ser Gly Ile Ile Ile Gly Val Thr Val Ala Ala Val Val
                185                 190                 195

Leu Ile Val Ala Val Phe Val Cys Lys Ser Leu Leu Trp Lys Lys
                200                 205                 210

Val Leu Pro Tyr Leu Lys Gly Ile Cys Ser Gly Gly Gly Gly Asp
                215                 220                 225

Pro Glu Arg Val Asp Arg Ser Ser Gln Arg Pro Gly Ala Glu Asp
                230                 235                 240

Asn Val Leu Asn Glu Ile Val Ser Ile Leu Gln Pro Thr Gln Val
                245                 250                 255

Pro Glu Gln Glu Met Glu Val Gln Glu Pro Ala Glu Pro Thr Gly
                260                 265                 270

Val Asn Met Leu Ser Pro Gly Glu Ser Glu His Leu Leu Glu Pro
                275                 280                 285

Ala Glu Ala Glu Arg Ser Gln Arg Arg Arg Leu Leu Val Pro Ala
                290                 295                 300

Asn Glu Gly Asp Pro Thr Glu Thr Leu Arg Gln Cys Phe Asp Asp
                305                 310                 315

Phe Ala Asp Leu Val Pro Phe Asp Ser Trp Glu Pro Leu Met Arg
                320                 325                 330

Lys Leu Gly Leu Met Asp Asn Glu Ile Lys Val Ala Lys Ala Glu
                335                 340                 345

Ala Ala Gly His Arg Asp Thr Leu Tyr Thr Met Leu Ile Lys Trp
                350                 355                 360

Val Asn Lys Thr Gly Arg Asp Ala Ser Val His Thr Leu Leu Asp
                365                 370                 375

Ala Leu Glu Thr Leu Gly Glu Arg Leu Ala Lys Gln Lys Ile Glu
                380                 385                 390

Asp His Leu Leu Ser Ser Gly Lys Phe Met Tyr Leu Glu Gly Asn
                395                 400                 405

Ala Asp Ser Ala Xaa Ser
                410

<210> 6
<211> 440
<212> PRT
<213> Homo Sapiens

<400> 6